# EUROPEAN PATENT APPLICATION

(11) **EP 2 336 306 A2**
(43) Date of publication of application: **22.06.2011**
(21) Application number: 10013988.0
(22) Date of filing: 08.06.2007
(51) Int. Cl.: C12N 9/10

(54) **Novel EPSP synthase genes conferring herbicide resistance**

(30) Priority: 09.06.2006 US 812360 P
(62) Divisional of application: 07798271.8
(71) Applicant: Athenix Corporation, Research Triangle Park, NC 27709 (US)
(72) Inventor: Carr, Brian, Raleigh, NC 27613 (US); Vande Berg, Brian, Durham, NC 27713 (US); Tomso, Daniel John, Bahama, NC 27503 (US); Peters, Cheryl, Raleigh, NC 27612 (US)
(74) Representative: Power, David

(57) **Abstract**

Compositions and methods for conferring herbicide resistance or tolerance to bacteria, plants, plant cells, tissues and seeds are provided. Compositions comprising a coding sequence for a polypeptide that confers resistance or tolerance to glyphosate Q herbicides are provided. The coding sequences can be used in DNA constructs or expression cassettes for transformation and expression in plants. Compositions also comprise transformed bacteria, plants, plant cells, tissues, and seeds. In particular, isolated nucleic acid molecules corresponding to glyphosate resistant nucleic acid sequences are provided. Additionally, amino acid sequences corresponding to the polynucleotides are encompassed. In particular, the present invention provides for isolated nucleic acid molecules comprising nucleotide sequences encoding the amino acid sequence shown in SEQ ID NOS: 12, 2, 4, 6, 8, 10, or 14 or the nucleotide sequence set forth in SEQ ID NOS: 11, 1, 3, 5, 7, 9, 13, 28, 29, 30, 31, 32, 33, or 34.

SEQUENCE LISTING

<110> Athenix Corporation
      Brian Vande Berg
      Cheryl Peters
      Brian Carr
      Daniel John Tomso

<120> Novel EPSP Synthase Genes Conferring
  Herbicide Resistance

<130> 45600/329201

<150> 60/812,360
<151> 2006-06-09

<160> 38

<170> FastSEQ for Windows Version 4.0

<210> 1
<211> 1404
<212> DNA
<213> Arthrobacter arilaiti

<220>
<221> CDS
<222> (1)...(1404)

<400> 1
atg ctg ctg cgc acc gtg aaa aca tgc ctc gag ccg aag gat cta cca    48
Met Leu Leu Arg Thr Val Lys Thr Cys Leu Glu Pro Lys Asp Leu Pro
 1               5                  10                  15

atc tgt act gca agc gcc cct gga agc cca acc gat aag ctg cac aac    96
Ile Cys Thr Ala Ser Ala Pro Gly Ser Pro Thr Asp Lys Leu His Asn
            20                  25                  30

gca gaa aag acc tgg tgg gcc gcg cct cat gcc gcc acc ggc ctg gac    144
Ala Glu Lys Thr Trp Trp Ala Ala Pro His Ala Ala Thr Gly Leu Asp
        35                  40                  45

gcc atc gtc tcg gtg ccc gct tcg aag tcc ttg acc aac cgg tat ctg    192
Ala Ile Val Ser Val Pro Ala Ser Lys Ser Leu Thr Asn Arg Tyr Leu
    50                  55                  60

atc ctg gcg gcc ctg gcc tcc tcg cca tcc acc atc cac aac acg ctc    240
Ile Leu Ala Ala Leu Ala Ser Ser Pro Ser Thr Ile His Asn Thr Leu
65                  70                  75                  80

atc agc cgc gac acc gaa ctg atg ctc gat gcg ctg gcc gct ttc ggc    288
Ile Ser Arg Asp Thr Glu Leu Met Leu Asp Ala Leu Ala Ala Phe Gly
                85                  90                  95

atc ggc atc gaa cgc acc acg cag cca gat ggc tcc acc acc gtg gcc    336
Ile Gly Ile Glu Arg Thr Thr Gln Pro Asp Gly Ser Thr Thr Val Ala
            100                 105                 110

atc acc ccg ggc aag ctg gcc acc ggc ccg ctg tcc atc gac tgc ggc    384
Ile Thr Pro Gly Lys Leu Ala Thr Gly Pro Leu Ser Ile Asp Cys Gly

        115                 120                 125

ctg gcc ggc acc gtc atg cgc ttc gtt ccg ccg ctg gcc gcc gtt gcc 432
Leu Ala Gly Thr Val Met Arg Phe Val Pro Pro Leu Ala Ala Val Ala
    130                 135                 140

ggt gcc agc gtg gca ttc gat ggc gat gaa gca gcc cgc gtg cgc ccg 480
Gly Ala Ser Val Ala Phe Asp Gly Asp Glu Ala Ala Arg Val Arg Pro
145                 150                 155                 160

atg gcc ccg gtg ctc gac gcc ttg gaa acc ctg ggt gcc cgg atc gaa 528
Met Ala Pro Val Leu Asp Ala Leu Glu Thr Leu Gly Ala Arg Ile Glu
                165                 170                 175

tat gcc ggc act ccc ggc atg ctg ccc ttc acc atg gac gcc agc gca 576
Tyr Ala Gly Thr Pro Gly Met Leu Pro Phe Thr Met Asp Ala Ser Ala
            180                 185                 190

ttg cag gag cgc cac gag gtg ctc atc gac gcc tcg ggc agc tcc cag 624
Leu Gln Glu Arg His Glu Val Leu Ile Asp Ala Ser Gly Ser Ser Gln
        195                 200                 205

ttc atc tcc gca ctg ctg ctg gtc ggc caa gca ctg ccc ggt ggg ctg 672
Phe Ile Ser Ala Leu Leu Leu Val Gly Gln Ala Leu Pro Gly Gly Leu
    210                 215                 220

aag ctg cgg gcc gcc gcg ggg cat atc gcc tcc cct gac cac att gcc 720
Lys Leu Arg Ala Ala Ala Gly His Ile Ala Ser Pro Asp His Ile Ala
225                 230                 235                 240

atg acc gtg caa acc ctg cgc gaa ctg ggc gtc gag gta gcc gtg ggc 768
Met Thr Val Gln Thr Leu Arg Glu Leu Gly Val Glu Val Ala Val Gly
                245                 250                 255

gag gat gca cgc agc tgg tcc atc gca ccg ggt cag ctt tcc ggg ttc 816
Glu Asp Ala Arg Ser Trp Ser Ile Ala Pro Gly Gln Leu Ser Gly Phe
            260                 265                 270

act atc acc gtg gaa ccg gac ctg tcc aac gcc ggt ccc ttc ctg gcg 864
Thr Ile Thr Val Glu Pro Asp Leu Ser Asn Ala Gly Pro Phe Leu Ala
        275                 280                 285

gcc gcc ttg gcc acc aac ggc acc gtg cgc gtg ccc ttc tgg cct gca 912
Ala Ala Leu Ala Thr Asn Gly Thr Val Arg Val Pro Phe Trp Pro Ala
    290                 295                 300

tcc acc acc cag gtg ggc ggc aaa tgg gtg cag atc cta agc cgg atg 960
Ser Thr Thr Gln Val Gly Gly Lys Trp Val Gln Ile Leu Ser Arg Met
305                 310                 315                 320

ggc gca gaa atc agc cac ggc gag gac gga gtg ctc acc gta cgc ggc 1008
Gly Ala Glu Ile Ser His Gly Glu Asp Gly Val Leu Thr Val Arg Gly
                325                 330                 335

acc gga gtg atc cgc ggc atc gac tac gcc gat gcc tcc gaa ttg gct 1056
Thr Gly Val Ile Arg Gly Ile Asp Tyr Ala Asp Ala Ser Glu Leu Ala
            340                 345                 350

ccc acc ctg gcc gcg ctg tgc acc ctg gcg gac tcc ccc agc aag ctg 1104
Pro Thr Leu Ala Ala Leu Cys Thr Leu Ala Asp Ser Pro Ser Lys Leu
        355                 360                 365

acc ggc atc ggg cac ctg cgc ggg cac gaa acg gac cgg ctg gcg gca     1152
Thr Gly Ile Gly His Leu Arg Gly His Glu Thr Asp Arg Leu Ala Ala
    370                 375                 380

ctg gaa acc gag ctt gcc aag gtc ggt gcc acg gtg acc tcc acc gac     1200
Leu Glu Thr Glu Leu Ala Lys Val Gly Ala Thr Val Thr Ser Thr Asp
385                 390                 395                 400

gac gcg ttg gaa atc att ccc gga acc ctg cag gca gcc gat ctg gac     1248
Asp Ala Leu Glu Ile Ile Pro Gly Thr Leu Gln Ala Ala Asp Leu Asp
                405                 410                 415

tcc tac gag gat cac cgg atg gcc acc gca ggc gca ctg ctg ggc ctg     1296
Ser Tyr Glu Asp His Arg Met Ala Thr Ala Gly Ala Leu Leu Gly Leu
            420                 425                 430

gcc atc gaa ggg gtg cgc gtg gaa aat atc gcc acc acc gcc aag acc     1344
Ala Ile Glu Gly Val Arg Val Glu Asn Ile Ala Thr Thr Ala Lys Thr
        435                 440                 445

atg ccc gac ttc ccg caa ctc tgg acc gac atg gcc gcc acc gca aag     1392
Met Pro Asp Phe Pro Gln Leu Trp Thr Asp Met Ala Ala Thr Ala Lys
    450                 455                 460

ggc cag ggc taa                                                     1404
Gly Gln Gly  *
465

<210> 2
<211> 467
<212> PRT
<213> Arthrobacter arilaiti

<400> 2
Met Leu Leu Arg Thr Val Lys Thr Cys Leu Glu Pro Lys Asp Leu Pro
 1               5                  10                  15
Ile Cys Thr Ala Ser Ala Pro Gly Ser Pro Thr Asp Lys Leu His Asn
            20                  25                  30
Ala Glu Lys Thr Trp Trp Ala Ala Pro His Ala Ala Thr Gly Leu Asp
        35                  40                  45
Ala Ile Val Ser Val Pro Ala Ser Lys Ser Leu Thr Asn Arg Tyr Leu
    50                  55                  60
Ile Leu Ala Ala Leu Ala Ser Ser Pro Ser Thr Ile His Asn Thr Leu
65                  70                  75                  80
Ile Ser Arg Asp Thr Glu Leu Met Leu Asp Ala Leu Ala Ala Phe Gly
                85                  90                  95
Ile Gly Ile Glu Arg Thr Thr Gln Pro Asp Gly Ser Thr Thr Val Ala
            100                 105                 110
Ile Thr Pro Gly Lys Leu Ala Thr Gly Pro Leu Ser Ile Asp Cys Gly
        115                 120                 125
Leu Ala Gly Thr Val Met Arg Phe Val Pro Pro Leu Ala Ala Val Ala
    130                 135                 140
Gly Ala Ser Val Ala Phe Asp Gly Asp Glu Ala Ala Arg Val Arg Pro
145                 150                 155                 160
Met Ala Pro Val Leu Asp Ala Leu Glu Thr Leu Gly Ala Arg Ile Glu
                165                 170                 175
Tyr Ala Gly Thr Pro Gly Met Leu Pro Phe Thr Met Asp Ala Ser Ala
            180                 185                 190
Leu Gln Glu Arg His Glu Val Leu Ile Asp Ala Ser Gly Ser Ser Gln

195                 200                 205
Phe Ile Ser Ala Leu Leu Leu Val Gly Gln Ala Leu Pro Gly Gly Leu
    210                 215                 220
Lys Leu Arg Ala Ala Ala Gly His Ile Ala Ser Pro Asp His Ile Ala
225                 230                 235                 240
Met Thr Val Gln Thr Leu Arg Glu Leu Gly Val Glu Val Ala Val Gly
                245                 250                 255
Glu Asp Ala Arg Ser Trp Ser Ile Ala Pro Gly Gln Leu Ser Gly Phe
            260                 265                 270
Thr Ile Thr Val Glu Pro Asp Leu Ser Asn Ala Gly Pro Phe Leu Ala
        275                 280                 285
Ala Ala Leu Ala Thr Asn Gly Thr Val Arg Val Pro Phe Trp Pro Ala
    290                 295                 300
Ser Thr Thr Gln Val Gly Gly Lys Trp Val Gln Ile Leu Ser Arg Met
305                 310                 315                 320
Gly Ala Glu Ile Ser His Gly Glu Asp Gly Val Leu Thr Val Arg Gly
                325                 330                 335
Thr Gly Val Ile Arg Gly Ile Asp Tyr Ala Asp Ala Ser Glu Leu Ala
            340                 345                 350
Pro Thr Leu Ala Ala Leu Cys Thr Leu Ala Asp Ser Pro Ser Lys Leu
        355                 360                 365
Thr Gly Ile Gly His Leu Arg Gly His Glu Thr Asp Arg Leu Ala Ala
    370                 375                 380
Leu Glu Thr Glu Leu Ala Lys Val Gly Ala Thr Val Thr Ser Thr Asp
385                 390                 395                 400
Asp Ala Leu Glu Ile Ile Pro Gly Thr Leu Gln Ala Ala Asp Leu Asp
                405                 410                 415
Ser Tyr Glu Asp His Arg Met Ala Thr Ala Gly Ala Leu Leu Gly Leu
            420                 425                 430
Ala Ile Glu Gly Val Arg Val Glu Asn Ile Ala Thr Thr Ala Lys Thr
        435                 440                 445
Met Pro Asp Phe Pro Gln Leu Trp Thr Asp Met Ala Ala Thr Ala Lys
    450                 455                 460
Gly Gln Gly
465

<210> 3
<211> 1395
<212> DNA
<213> Arthrobacter ramosus

<220>
<221> CDS
<222> (1)...(1395)

<400> 3
atg cca gga acc ccc gcc acg cag aca gac gat tca ggg acg agc aca    48
Met Pro Gly Thr Pro Ala Thr Gln Thr Asp Asp Ser Gly Thr Ser Thr
 1               5                  10                  15

gct tcc gcg ctt ccc ctg tgg ccc gca ccc ttc gcc agc cac cct gtg    96
Ala Ser Ala Leu Pro Leu Trp Pro Ala Pro Phe Ala Ser His Pro Val
            20                  25                  30

gat gcc acg gtc acg gtt cct ggt tcg aag tcg ctg acc aac cgc tat    144
Asp Ala Thr Val Thr Val Pro Gly Ser Lys Ser Leu Thr Asn Arg Tyr
        35                  40                  45

ctg gtc ctg gcg gca ttg gcc aac ggc ccc agc agg ttg cgt gca ccc    192
Leu Val Leu Ala Ala Leu Ala Asn Gly Pro Ser Arg Leu Arg Ala Pro

    50                  55                  60

ttg cat tcg cgc gat tcg gcg ctg atg gtc gag gcc ctg cgc cgg ctt 240
Leu His Ser Arg Asp Ser Ala Leu Met Val Glu Ala Leu Arg Arg Leu
65                  70                  75                  80

ggc gcc acc att aca gag gtt ccc ggc gat gga cag tac gga ccg gac 288
Gly Ala Thr Ile Thr Glu Val Pro Gly Asp Gly Gln Tyr Gly Pro Asp
                85                  90                  95

ctt gaa gtc acc ccg atc gac cct gcg gcg gcg gca tcg gag acc gcc 336
Leu Glu Val Thr Pro Ile Asp Pro Ala Ala Ala Ala Ser Glu Thr Ala
            100                 105                 110

atc gac tgc ggc ctg gcc ggc acg gtc atg cgc ttc gtg ccg ccg ctt 384
Ile Asp Cys Gly Leu Ala Gly Thr Val Met Arg Phe Val Pro Pro Leu
        115                 120                 125

gcg gca ctt cgc aac ggc gct tcg gtg ttt gac ggc gat ccg cac gca 432
Ala Ala Leu Arg Asn Gly Ala Ser Val Phe Asp Gly Asp Pro His Ala
    130                 135                 140

cgc cag cgt ccc atg ggc acc atc atc gaa gcc ctc cgg acc ctc gga 480
Arg Gln Arg Pro Met Gly Thr Ile Ile Glu Ala Leu Arg Thr Leu Gly
145                 150                 155                 160

gtg gac gtc agc gcc atg gac ggg aag gcg ccg ggc tcc ttg ccg ttc 528
Val Asp Val Ser Ala Met Asp Gly Lys Ala Pro Gly Ser Leu Pro Phe
                165                 170                 175

aag gtc tcc ggc acg ggc tca gtg cgc ggt ggc cac ttg gtg atc gac 576
Lys Val Ser Gly Thr Gly Ser Val Arg Gly Gly His Leu Val Ile Asp
            180                 185                 190

gcg agc gct tcc tcg cag ttc gtc tcc gcc ctg ctg ctc gtc ggt gcc 624
Ala Ser Ala Ser Ser Gln Phe Val Ser Ala Leu Leu Leu Val Gly Ala
        195                 200                 205

cgt ttc gaa gag ggc ctg cac ctc gag cat gtc ggc aag ccc gtg ccg 672
Arg Phe Glu Glu Gly Leu His Leu Glu His Val Gly Lys Pro Val Pro
    210                 215                 220

agc ctc gac cac atc aac atg act gtg gcg gtc ctg cgc ggg gtt gga 720
Ser Leu Asp His Ile Asn Met Thr Val Ala Val Leu Arg Gly Val Gly
225                 230                 235                 240

gtc cag gta gat gac tcc gtg ccg aac cat tgg cga gtc tcc ccc ggc 768
Val Gln Val Asp Asp Ser Val Pro Asn His Trp Arg Val Ser Pro Gly
                245                 250                 255

gcc atc cag gcg ttc gac gag cga atc gaa cag gac ctc tcc aac gcg 816
Ala Ile Gln Ala Phe Asp Glu Arg Ile Glu Gln Asp Leu Ser Asn Ala
            260                 265                 270

ggt ccc ttc ctc gcc gcg gcg ctc gcc acg aag gga acc gtg cgg atc 864
Gly Pro Phe Leu Ala Ala Ala Leu Ala Thr Lys Gly Thr Val Arg Ile
        275                 280                 285

ccc aac tgg cct gcc agc acc acg caa gtc gga gat ctc tgg cgg aac 912
Pro Asn Trp Pro Ala Ser Thr Thr Gln Val Gly Asp Leu Trp Arg Asn
    290                 295                 300

att ctt gcc acc atg ggc gct acc gtc acg ctg gac aac ggg acc ctg     960
Ile Leu Ala Thr Met Gly Ala Thr Val Thr Leu Asp Asn Gly Thr Leu
305                 310                 315                 320

aca gtc acc ggc ggt tcc gag atc ctg ggc gcg gac ttc gac gaa acg     1008
Thr Val Thr Gly Gly Ser Glu Ile Leu Gly Ala Asp Phe Asp Glu Thr
                325                 330                 335

agc gaa ctc gcc ccc acg gta gcg gca ttg tgc gcc ttg gca acc agc     1056
Ser Glu Leu Ala Pro Thr Val Ala Ala Leu Cys Ala Leu Ala Thr Ser
            340                 345                 350

cct tcc cgg ctc acc ggc atc gcg cac ctg cgc gga cac gag acg gac     1104
Pro Ser Arg Leu Thr Gly Ile Ala His Leu Arg Gly His Glu Thr Asp
        355                 360                 365

agg ctg gcc gcg ctc gtc gcc gaa atc aac cgg ctt ggc ggc gac gcc     1152
Arg Leu Ala Ala Leu Val Ala Glu Ile Asn Arg Leu Gly Gly Asp Ala
    370                 375                 380

gag gaa acc agc gac ggc ctg gta atc cgg ccc gca gcg ctg cac tcc     1200
Glu Glu Thr Ser Asp Gly Leu Val Ile Arg Pro Ala Ala Leu His Ser
385                 390                 395                 400

ggc gtc gtg cat agc tac gcc gac cac cgc atg gct acg gcg ggc gcc     1248
Gly Val Val His Ser Tyr Ala Asp His Arg Met Ala Thr Ala Gly Ala
                405                 410                 415

atc ctt ggg ctc gcc gtc gag ggt gtc cgg gtc gaa gac atc gcg acg     1296
Ile Leu Gly Leu Ala Val Glu Gly Val Arg Val Glu Asp Ile Ala Thr
            420                 425                 430

acg tcc aag acc atg ccc gaa ttc ccg cag atc tgg acg tcg atg ctc     1344
Thr Ser Lys Thr Met Pro Glu Phe Pro Gln Ile Trp Thr Ser Met Leu
        435                 440                 445

agt gca ggg cca gcg gcc gcg gcc gcc ggt tca acg gag aca agc aac     1392
Ser Ala Gly Pro Ala Ala Ala Ala Ala Gly Ser Thr Glu Thr Ser Asn
    450                 455                 460

tga                                                                 1395
 *

<210> 4
<211> 464
<212> PRT
<213> Arthrobacter ramosus

<400> 4
Met Pro Gly Thr Pro Ala Thr Gln Thr Asp Asp Ser Gly Thr Ser Thr
 1               5                  10                  15
Ala Ser Ala Leu Pro Leu Trp Pro Ala Pro Phe Ala Ser His Pro Val
            20                  25                  30
Asp Ala Thr Val Thr Val Pro Gly Ser Lys Ser Leu Thr Asn Arg Tyr
        35                  40                  45
Leu Val Leu Ala Ala Leu Ala Asn Gly Pro Ser Arg Leu Arg Ala Pro
    50                  55                  60
Leu His Ser Arg Asp Ser Ala Leu Met Val Glu Ala Leu Arg Arg Leu

65                  70                  75                  80
Gly Ala Thr Ile Thr Glu Val Pro Gly Asp Gly Gln Tyr Gly Pro Asp
                85                  90                  95
Leu Glu Val Thr Pro Ile Asp Pro Ala Ala Ala Ser Glu Thr Ala
            100                 105                 110
Ile Asp Cys Gly Leu Ala Gly Thr Val Met Arg Phe Val Pro Pro Leu
        115                 120                 125
Ala Ala Leu Arg Asn Gly Ala Ser Val Phe Asp Gly Asp Pro His Ala
    130                 135                 140
Arg Gln Arg Pro Met Gly Thr Ile Ile Glu Ala Leu Arg Thr Leu Gly
145                 150                 155                 160
Val Asp Val Ser Ala Met Asp Gly Lys Ala Pro Gly Ser Leu Pro Phe
                165                 170                 175
Lys Val Ser Gly Thr Gly Ser Val Arg Gly Gly His Leu Val Ile Asp
            180                 185                 190
Ala Ser Ala Ser Ser Gln Phe Val Ser Ala Leu Leu Leu Val Gly Ala
        195                 200                 205
Arg Phe Glu Glu Gly Leu His Leu Glu His Val Gly Lys Pro Val Pro
    210                 215                 220
Ser Leu Asp His Ile Asn Met Thr Val Ala Val Leu Arg Gly Val Gly
225                 230                 235                 240
Val Gln Val Asp Asp Ser Val Pro Asn His Trp Arg Val Ser Pro Gly
                245                 250                 255
Ala Ile Gln Ala Phe Asp Glu Arg Ile Glu Gln Asp Leu Ser Asn Ala
            260                 265                 270
Gly Pro Phe Leu Ala Ala Ala Leu Ala Thr Lys Gly Thr Val Arg Ile
        275                 280                 285
Pro Asn Trp Pro Ala Ser Thr Thr Gln Val Gly Asp Leu Trp Arg Asn
    290                 295                 300
Ile Leu Ala Thr Met Gly Ala Thr Val Thr Leu Asp Asn Gly Thr Leu
305                 310                 315                 320
Thr Val Thr Gly Gly Ser Glu Ile Leu Gly Ala Asp Phe Asp Glu Thr
                325                 330                 335
Ser Glu Leu Ala Pro Thr Val Ala Ala Leu Cys Ala Leu Ala Thr Ser
            340                 345                 350
Pro Ser Arg Leu Thr Gly Ile Ala His Leu Arg Gly His Glu Thr Asp
        355                 360                 365
Arg Leu Ala Ala Leu Val Ala Glu Ile Asn Arg Leu Gly Gly Asp Ala
    370                 375                 380
Glu Glu Thr Ser Asp Gly Leu Val Ile Arg Pro Ala Ala Leu His Ser
385                 390                 395                 400
Gly Val Val His Ser Tyr Ala Asp His Arg Met Ala Thr Ala Gly Ala
                405                 410                 415
Ile Leu Gly Leu Ala Val Glu Gly Val Arg Val Glu Asp Ile Ala Thr
            420                 425                 430
Thr Ser Lys Thr Met Pro Glu Phe Pro Gln Ile Trp Thr Ser Met Leu
        435                 440                 445
Ser Ala Gly Pro Ala Ala Ala Ala Ala Gly Ser Thr Glu Thr Ser Asn
    450                 455                 460

<210> 5
<211> 1368
<212> DNA
<213> Gram positive bacteria

<220>
<221> misc_feature
<222> (0)...(0)
<223> Gram+ bacteria - strain unknown

<221> CDS
<222> (1)...(1368)

<400> 5
atg aca ggt acc acc gcc gcc aat acc gaa ccg gac aaa gcc gcc agc    48
Met Thr Gly Thr Thr Ala Ala Asn Thr Glu Pro Asp Lys Ala Ala Ser
 1               5                  10                  15

ctc ccg cta tgg ccg gcc cct tac gcc aat ggg cca gtg gac gcc act    96
Leu Pro Leu Trp Pro Ala Pro Tyr Ala Asn Gly Pro Val Asp Ala Thr
            20                  25                  30

gtg aca gtc ccc ggt tca aaa tcg ctg acc aac cgg ttc ctg gtg ctg    144
Val Thr Val Pro Gly Ser Lys Ser Leu Thr Asn Arg Phe Leu Val Leu
        35                  40                  45

gca gcc ttg gcg gac ggt ccc tcg cgg ctc cgg gcc cca ctg cat tca    192
Ala Ala Leu Ala Asp Gly Pro Ser Arg Leu Arg Ala Pro Leu His Ser
    50                  55                  60

cgc gat tct gcc ctg atg atc caa gcc ctt cgg cag ttg ggt gcc acc    240
Arg Asp Ser Ala Leu Met Ile Gln Ala Leu Arg Gln Leu Gly Ala Thr
 65                  70                  75                  80

gtc acc gag gta ccc ggt gac ggt gac tat ggg ccc gac ctg gag atc    288
Val Thr Glu Val Pro Gly Asp Gly Asp Tyr Gly Pro Asp Leu Glu Ile
                85                  90                  95

acg ccc ctg gac ccc tcc gcc gcg ggc tca agc acc gcc atc gac tgc    336
Thr Pro Leu Asp Pro Ser Ala Ala Gly Ser Ser Thr Ala Ile Asp Cys
            100                 105                 110

ggc ctg gcg ggt acg gtc atg agg ttc gtt ccc ccg ctt gct gcc ctg    384
Gly Leu Ala Gly Thr Val Met Arg Phe Val Pro Pro Leu Ala Ala Leu
        115                 120                 125

cgc agc ggc gca acc gtc ttc gac ggc gat ccc cat gcc cgc aac cgt    432
Arg Ser Gly Ala Thr Val Phe Asp Gly Asp Pro His Ala Arg Asn Arg
    130                 135                 140

ccc atg gga acc atc atc gag gca ctt ctt gcc ttg gga gtc cct gtt    480
Pro Met Gly Thr Ile Ile Glu Ala Leu Leu Ala Leu Gly Val Pro Val
145                 150                 155                 160

gca gcc gaa ggc ggc agg acg ccg tcg gcc ctt ccc ttc acg gtg gaa    528
Ala Ala Glu Gly Gly Arg Thr Pro Ser Ala Leu Pro Phe Thr Val Glu
                165                 170                 175

ggc acc ggc gag gtg cgc ggc ggc cac ttg gtc atc gac gcc agc gct    576
Gly Thr Gly Glu Val Arg Gly Gly His Leu Val Ile Asp Ala Ser Ala
            180                 185                 190

tcc tcc cag ttt gtt tcg gca ttg ctg ttg gtg ggc gcc cgc ttc acc    624
Ser Ser Gln Phe Val Ser Ala Leu Leu Leu Val Gly Ala Arg Phe Thr
        195                 200                 205

gaa ggc ctg cac ttg gag cac gtg ggc aaa ccc gtt ccc agc ctg gat    672
Glu Gly Leu His Leu Glu His Val Gly Lys Pro Val Pro Ser Leu Asp
    210                 215                 220

cac atc acc atg acc gtt gag gtg ctt cgg agc gtg ggt gtc acc gtg    720

His Ile Thr Met Thr Val Glu Val Leu Arg Ser Val Gly Val Thr Val
225                 230                 235                 240

gat gac tcc gta ccc aac cac tgg cgc gta tcg cct ggc aag atc acc     768
Asp Asp Ser Val Pro Asn His Trp Arg Val Ser Pro Gly Lys Ile Thr
                245                 250                 255

gct ttt gac cag cgg atc gag cag gat ctc tcc aat gcc gga ccc ttc     816
Ala Phe Asp Gln Arg Ile Glu Gln Asp Leu Ser Asn Ala Gly Pro Phe
            260                 265                 270

ctg gcc gcc gcc ctg gcc act cat ggc act gtc cgc att ccc aac tgg     864
Leu Ala Ala Ala Leu Ala Thr His Gly Thr Val Arg Ile Pro Asn Trp
        275                 280                 285

ccc att ggc acc acg cag gtg ggc gat ctc tgg cgg acc atc ctc gct     912
Pro Ile Gly Thr Thr Gln Val Gly Asp Leu Trp Arg Thr Ile Leu Ala
    290                 295                 300

gcc atg ggc gcg acc gtc acc ctc gac ggg gga acg ctg aca gtg acc     960
Ala Met Gly Ala Thr Val Thr Leu Asp Gly Gly Thr Leu Thr Val Thr
305                 310                 315                 320

ggc ggg aat gag atc aag ggt gcc gat ttc gac gaa acc agc gaa ctc     1008
Gly Gly Asn Glu Ile Lys Gly Ala Asp Phe Asp Glu Thr Ser Glu Leu
                325                 330                 335

gcg ccg acg gta gct gcc ctt tgc gcc ctg gcc acc ggt cca tcg cga     1056
Ala Pro Thr Val Ala Ala Leu Cys Ala Leu Ala Thr Gly Pro Ser Arg
            340                 345                 350

ctg act ggc atc gcc cac ctt cgc gga cac gag acg gat cgc ctc gcc     1104
Leu Thr Gly Ile Ala His Leu Arg Gly His Glu Thr Asp Arg Leu Ala
        355                 360                 365

gcc ctg gtt gcc gag atc aac agg ctt ggt ggt gat gcg gaa gag acc     1152
Ala Leu Val Ala Glu Ile Asn Arg Leu Gly Gly Asp Ala Glu Glu Thr
    370                 375                 380

gca gat gga ctg ctt att cgc ccc gcc acc ctc cac ggc ggc gtg atc     1200
Ala Asp Gly Leu Leu Ile Arg Pro Ala Thr Leu His Gly Gly Val Ile
385                 390                 395                 400

cac agt tac gca gac cac cgg atg gcc act gcc ggc gcc atc ctg ggg     1248
His Ser Tyr Ala Asp His Arg Met Ala Thr Ala Gly Ala Ile Leu Gly
                405                 410                 415

ctc gcc gtt gac ggc gtc cag gtt gaa gac atc gcc acg aca tcc aag     1296
Leu Ala Val Asp Gly Val Gln Val Glu Asp Ile Ala Thr Thr Ser Lys
            420                 425                 430

acc atg ccc gag ttc ccg gag atg tgg gca aga atg ctt gac tcc gcc     1344
Thr Met Pro Glu Phe Pro Glu Met Trp Ala Arg Met Leu Asp Ser Ala
        435                 440                 445

ggc acg aac gag gcc ggc aac tga                                     1368
Gly Thr Asn Glu Ala Gly Asn  *
    450                 455

<210> 6

<211> 455
<212> PRT
<213> Gram positive bacteria

<400> 6
Met Thr Gly Thr Thr Ala Ala Asn Thr Glu Pro Asp Lys Ala Ala Ser
1               5                   10                  15
Leu Pro Leu Trp Pro Ala Pro Tyr Ala Asn Gly Pro Val Asp Ala Thr
            20                  25                  30
Val Thr Val Pro Gly Ser Lys Ser Leu Thr Asn Arg Phe Leu Val Leu
        35                  40                  45
Ala Ala Leu Ala Asp Gly Pro Ser Arg Leu Arg Ala Pro Leu His Ser
    50                  55                  60
Arg Asp Ser Ala Leu Met Ile Gln Ala Leu Arg Gln Leu Gly Ala Thr
65                  70                  75                  80
Val Thr Glu Val Pro Gly Asp Gly Asp Tyr Gly Pro Asp Leu Glu Ile
                85                  90                  95
Thr Pro Leu Asp Pro Ser Ala Ala Gly Ser Ser Thr Ala Ile Asp Cys
            100                 105                 110
Gly Leu Ala Gly Thr Val Met Arg Phe Val Pro Pro Leu Ala Ala Leu
        115                 120                 125
Arg Ser Gly Ala Thr Val Phe Asp Gly Asp Pro His Ala Arg Asn Arg
    130                 135                 140
Pro Met Gly Thr Ile Ile Glu Ala Leu Leu Ala Leu Gly Val Pro Val
145                 150                 155                 160
Ala Ala Glu Gly Gly Arg Thr Pro Ser Ala Leu Pro Phe Thr Val Glu
                165                 170                 175
Gly Thr Gly Glu Val Arg Gly Gly His Leu Val Ile Asp Ala Ser Ala
            180                 185                 190
Ser Ser Gln Phe Val Ser Ala Leu Leu Leu Val Gly Ala Arg Phe Thr
        195                 200                 205
Glu Gly Leu His Leu Glu His Val Gly Lys Pro Val Pro Ser Leu Asp
    210                 215                 220
His Ile Thr Met Thr Val Glu Val Leu Arg Ser Val Gly Val Thr Val
225                 230                 235                 240
Asp Asp Ser Val Pro Asn His Trp Arg Val Ser Pro Gly Lys Ile Thr
                245                 250                 255
Ala Phe Asp Gln Arg Ile Glu Gln Asp Leu Ser Asn Ala Gly Pro Phe
            260                 265                 270
Leu Ala Ala Ala Leu Ala Thr His Gly Thr Val Arg Ile Pro Asn Trp
        275                 280                 285
Pro Ile Gly Thr Thr Gln Val Gly Asp Leu Trp Arg Thr Ile Leu Ala
    290                 295                 300
Ala Met Gly Ala Thr Val Thr Leu Asp Gly Gly Thr Leu Thr Val Thr
305                 310                 315                 320
Gly Gly Asn Glu Ile Lys Gly Ala Asp Phe Asp Glu Thr Ser Glu Leu
                325                 330                 335
Ala Pro Thr Val Ala Ala Leu Cys Ala Leu Ala Thr Gly Pro Ser Arg
            340                 345                 350
Leu Thr Gly Ile Ala His Leu Arg Gly His Glu Thr Asp Arg Leu Ala
        355                 360                 365
Ala Leu Val Ala Glu Ile Asn Arg Leu Gly Gly Asp Ala Glu Glu Thr
    370                 375                 380
Ala Asp Gly Leu Leu Ile Arg Pro Ala Thr Leu His Gly Gly Val Ile
385                 390                 395                 400
His Ser Tyr Ala Asp His Arg Met Ala Thr Ala Gly Ala Ile Leu Gly
                405                 410                 415
Leu Ala Val Asp Gly Val Gln Val Glu Asp Ile Ala Thr Thr Ser Lys
            420                 425                 430
Thr Met Pro Glu Phe Pro Glu Met Trp Ala Arg Met Leu Asp Ser Ala
        435                 440                 445

Gly Thr Asn Glu Ala Gly Asn
    450                 455

<210> 7
<211> 1386
<212> DNA
<213> Arthrobacter ureafaciens

<220>
<221> CDS
<222> (1)...(1386)

<400> 7
atg aca ggt acc acc gca gca aac acg ggt tca aac tcc gtc gac acc    48
Met Thr Gly Thr Thr Ala Ala Asn Thr Gly Ser Asn Ser Val Asp Thr
 1               5                  10                  15

ctg ccc ctc tgg gca gct ccc tac gcc acc agg cca gtg gat gcc aca    96
Leu Pro Leu Trp Ala Ala Pro Tyr Ala Thr Arg Pro Val Asp Ala Thr
            20                  25                  30

gtg acg gtg ccg ggc tca aaa tcg ctc acc aac cgt ttc ctg gtc ctc   144
Val Thr Val Pro Gly Ser Lys Ser Leu Thr Asn Arg Phe Leu Val Leu
        35                  40                  45

gca gca ttg gcc gac ggc cct tcc cgc ctg cgg gcc ccc ctt cac tcc   192
Ala Ala Leu Ala Asp Gly Pro Ser Arg Leu Arg Ala Pro Leu His Ser
    50                  55                  60

cgc gat tcc gtc ctt atg att cag gcg ctc aga caa ctg ggt gcc acc   240
Arg Asp Ser Val Leu Met Ile Gln Ala Leu Arg Gln Leu Gly Ala Thr
 65                  70                  75                  80

gtc acc gag gta ccc ggc gac ggt gat tat gga ccg gac ctt gag atc   288
Val Thr Glu Val Pro Gly Asp Gly Asp Tyr Gly Pro Asp Leu Glu Ile
                 85                  90                  95

act ccc atg gat cct tcg gct tcg ggt gcg gag act gcc atc gat tgc   336
Thr Pro Met Asp Pro Ser Ala Ser Gly Ala Glu Thr Ala Ile Asp Cys
            100                 105                 110

ggc ctt gcc ggt acg gtt atg cgc ttc gtt cca cct tta gct gcg cta   384
Gly Leu Ala Gly Thr Val Met Arg Phe Val Pro Pro Leu Ala Ala Leu
        115                 120                 125

cgc aat ggt cca acc acg ttc gac ggc gat ccc cat gcc cgt aag cgg   432
Arg Asn Gly Pro Thr Thr Phe Asp Gly Asp Pro His Ala Arg Lys Arg
    130                 135                 140

ccc atg gga acc atc atc gaa gcg ctg ctc gcc ctc gga gtt cct gtc   480
Pro Met Gly Thr Ile Ile Glu Ala Leu Leu Ala Leu Gly Val Pro Val
145                 150                 155                 160

gcc acc gaa ggc gga agg acg ccg tcg gcc ctt ccc ttc tct gtg gat   528
Ala Thr Glu Gly Gly Arg Thr Pro Ser Ala Leu Pro Phe Ser Val Asp
                165                 170                 175

ggc act ggc gag gtt cgg ggc ggc cat ctg gtg atc gat gcc agc gca   576
Gly Thr Gly Glu Val Arg Gly Gly His Leu Val Ile Asp Ala Ser Ala
            180                 185                 190

tcg tcc cag ttc gtt tcg gcc ttg ctg ctt gta ggc gcc aga ttc acc     624
Ser Ser Gln Phe Val Ser Ala Leu Leu Leu Val Gly Ala Arg Phe Thr
        195                 200                 205

gaa ggc ctt cac ctg gaa cac gtt ggc aag cct gtc ccc agc ctg gac     672
Glu Gly Leu His Leu Glu His Val Gly Lys Pro Val Pro Ser Leu Asp
    210                 215                 220

cac atc aat atg acc gtt tcc gtc ctt cgc gga gtg ggc gtc aag gtg     720
His Ile Asn Met Thr Val Ser Val Leu Arg Gly Val Gly Val Lys Val
225                 230                 235                 240

gac gat tcg gta ccc aac cac tgg cgg gtc gca cct ggc ccc atc cac     768
Asp Asp Ser Val Pro Asn His Trp Arg Val Ala Pro Gly Pro Ile His
                245                 250                 255

gcc ttc gac cag cgg atc gaa cag gac ctt tcc aac gcc ggc ccg ttc     816
Ala Phe Asp Gln Arg Ile Glu Gln Asp Leu Ser Asn Ala Gly Pro Phe
            260                 265                 270

ctt gcc gca gcc ctg gca acc cgc gga acc gtc cgg atc ccc aac tgg     864
Leu Ala Ala Ala Leu Ala Thr Arg Gly Thr Val Arg Ile Pro Asn Trp
        275                 280                 285

ccc acc cag acc acc cag gtg ggt gat ctt tgg cgg tcc atc ctg gtt     912
Pro Thr Gln Thr Thr Gln Val Gly Asp Leu Trp Arg Ser Ile Leu Val
    290                 295                 300

gag atg ggt gcc acg gtg aca ctc gaa aac ggc acg ctc acg gtt aag     960
Glu Met Gly Ala Thr Val Thr Leu Glu Asn Gly Thr Leu Thr Val Lys
305                 310                 315                 320

ggc ggc ccg gaa atc aaa ggc gcc gac ttc gac gaa acc agc gag ctc     1008
Gly Gly Pro Glu Ile Lys Gly Ala Asp Phe Asp Glu Thr Ser Glu Leu
                325                 330                 335

gcg ccc acg gtc gct gcg ctg tgc gca ctg gcc aca ggc cct tca cgg     1056
Ala Pro Thr Val Ala Ala Leu Cys Ala Leu Ala Thr Gly Pro Ser Arg
            340                 345                 350

ctg acc ggc atc gcg cac ctc cgt ggc cat gaa acc gat cgg ctc gcc     1104
Leu Thr Gly Ile Ala His Leu Arg Gly His Glu Thr Asp Arg Leu Ala
        355                 360                 365

gcg ctg gcc gct gaa atc aat cgg ctc ggc gga gac gca gaa gaa aca     1152
Ala Leu Ala Ala Glu Ile Asn Arg Leu Gly Gly Asp Ala Glu Glu Thr
    370                 375                 380

gct gac ggc ctg atc atc cgc ccg gcc act ctg cat ccc ggc gtc gta     1200
Ala Asp Gly Leu Ile Ile Arg Pro Ala Thr Leu His Pro Gly Val Val
385                 390                 395                 400

cac agc tac gcc gac cac cgc atg gct acg gcc ggc gct att ttg ggg     1248
His Ser Tyr Ala Asp His Arg Met Ala Thr Ala Gly Ala Ile Leu Gly
                405                 410                 415

ctc gcc gtt gag ggc gtc cag gtg gag gat atc gct acc acc tcc aag     1296
Leu Ala Val Glu Gly Val Gln Val Glu Asp Ile Ala Thr Thr Ser Lys
            420                 425                 430

acg gtg ccc gaa ttt ccg cag atg tgg gag gcc atg ctg cag cag ggc     1344
Thr Val Pro Glu Phe Pro Gln Met Trp Glu Ala Met Leu Gln Gln Gly
        435                 440                 445

acg ggc gag gac gaa acc gtc acc agt gag gcc agc aac tga             1386
Thr Gly Glu Asp Glu Thr Val Thr Ser Glu Ala Ser Asn  *
    450                 455                 460

<210> 8
<211> 461
<212> PRT
<213> Arthrobacter ureafaciens

<400> 8
Met Thr Gly Thr Thr Ala Ala Asn Thr Gly Ser Asn Ser Val Asp Thr
 1               5                  10                  15
Leu Pro Leu Trp Ala Ala Pro Tyr Ala Thr Arg Pro Val Asp Ala Thr
            20                  25                  30
Val Thr Val Pro Gly Ser Lys Ser Leu Thr Asn Arg Phe Leu Val Leu
        35                  40                  45
Ala Ala Leu Ala Asp Gly Pro Ser Arg Leu Arg Ala Pro Leu His Ser
    50                  55                  60
Arg Asp Ser Val Leu Met Ile Gln Ala Leu Arg Gln Leu Gly Ala Thr
65                  70                  75                  80
Val Thr Glu Val Pro Gly Asp Gly Asp Tyr Gly Pro Asp Leu Glu Ile
                85                  90                  95
Thr Pro Met Asp Pro Ser Ala Ser Gly Ala Glu Thr Ala Ile Asp Cys
            100                 105                 110
Gly Leu Ala Gly Thr Val Met Arg Phe Val Pro Pro Leu Ala Ala Leu
        115                 120                 125
Arg Asn Gly Pro Thr Thr Phe Asp Gly Asp Pro His Ala Arg Lys Arg
    130                 135                 140
Pro Met Gly Thr Ile Ile Glu Ala Leu Leu Ala Leu Gly Val Pro Val
145                 150                 155                 160
Ala Thr Glu Gly Gly Arg Thr Pro Ser Ala Leu Pro Phe Ser Val Asp
                165                 170                 175
Gly Thr Gly Glu Val Arg Gly Gly His Leu Val Ile Asp Ala Ser Ala
            180                 185                 190
Ser Ser Gln Phe Val Ser Ala Leu Leu Leu Val Gly Ala Arg Phe Thr
        195                 200                 205
Glu Gly Leu His Leu Glu His Val Gly Lys Pro Val Pro Ser Leu Asp
    210                 215                 220
His Ile Asn Met Thr Val Ser Val Leu Arg Gly Val Gly Val Lys Val
225                 230                 235                 240
Asp Asp Ser Val Pro Asn His Trp Arg Val Ala Pro Gly Pro Ile His
                245                 250                 255
Ala Phe Asp Gln Arg Ile Glu Gln Asp Leu Ser Asn Ala Gly Pro Phe
            260                 265                 270
Leu Ala Ala Ala Leu Ala Thr Arg Gly Thr Val Arg Ile Pro Asn Trp
        275                 280                 285
Pro Thr Gln Thr Thr Gln Val Gly Asp Leu Trp Arg Ser Ile Leu Val
    290                 295                 300
Glu Met Gly Ala Thr Val Thr Leu Glu Asn Gly Thr Leu Thr Val Lys
305                 310                 315                 320
Gly Gly Pro Glu Ile Lys Gly Ala Asp Phe Asp Glu Thr Ser Glu Leu
                325                 330                 335
Ala Pro Thr Val Ala Ala Leu Cys Ala Leu Ala Thr Gly Pro Ser Arg
            340                 345                 350
Leu Thr Gly Ile Ala His Leu Arg Gly His Glu Thr Asp Arg Leu Ala

355                 360                 365
Ala Leu Ala Ala Glu Ile Asn Arg Leu Gly Gly Asp Ala Glu Glu Thr
    370                 375                 380
Ala Asp Gly Leu Ile Ile Arg Pro Ala Thr Leu His Pro Gly Val Val
385                 390                 395                 400
His Ser Tyr Ala Asp His Arg Met Ala Thr Ala Gly Ala Ile Leu Gly
                405                 410                 415
Leu Ala Val Glu Gly Val Gln Val Glu Asp Ile Ala Thr Thr Ser Lys
            420                 425                 430
Thr Val Pro Glu Phe Pro Gln Met Trp Glu Ala Met Leu Gln Gln Gly
        435                 440                 445
Thr Gly Glu Asp Glu Thr Val Thr Ser Glu Ala Ser Asn
    450                 455                 460

<210> 9
<211> 1296
<212> DNA
<213> Paenibacillus pabuli

<220>
<221> CDS
<222> (1)...(1296)

<400> 9
atg gac gtt atc gtt aaa cca acc cca tcc ctg aac ggg gaa att gga    48
Met Asp Val Ile Val Lys Pro Thr Pro Ser Leu Asn Gly Glu Ile Gly
 1               5                  10                  15

gct ttg tct tcc aag aac tac acc aca cgc tac ttg cta gct gct gcg    96
Ala Leu Ser Ser Lys Asn Tyr Thr Thr Arg Tyr Leu Leu Ala Ala Ala
            20                  25                  30

ctg gca gaa ggc aca agt acg att cat tac cct gct cac agt gaa gat    144
Leu Ala Glu Gly Thr Ser Thr Ile His Tyr Pro Ala His Ser Glu Asp
        35                  40                  45

agt gac gct atg cgc aga tgt att agt gat ctt gga gcg gtg ctc gaa    192
Ser Asp Ala Met Arg Arg Cys Ile Ser Asp Leu Gly Ala Val Leu Glu
    50                  55                  60

gaa gat gat agc aaa atc gtt att cag ggc ttc ggc agc cat cca cgt    240
Glu Asp Asp Ser Lys Ile Val Ile Gln Gly Phe Gly Ser His Pro Arg
 65                  70                  75                  80

gat gtg cgt gaa tta aat gta ggc aat gcg ggt gca gtg ctg cgt ttc    288
Asp Val Arg Glu Leu Asn Val Gly Asn Ala Gly Ala Val Leu Arg Phe
                85                  90                  95

ctg atg ggg gta acg gca ctt tgt cct gat gtt acg ttt gta aat acg    336
Leu Met Gly Val Thr Ala Leu Cys Pro Asp Val Thr Phe Val Asn Thr
            100                 105                 110

tac ccg gat tct ctg ggc aaa cgc cca cat gat gac ctg atc gat gcg    384
Tyr Pro Asp Ser Leu Gly Lys Arg Pro His Asp Asp Leu Ile Asp Ala
        115                 120                 125

ctt ggt cag ctt ggt gtt gag gta caa cac gaa caa gga cgc ttg cca    432
Leu Gly Gln Leu Gly Val Glu Val Gln His Glu Gln Gly Arg Leu Pro
    130                 135                 140

atc acg atc aaa ggg ggt cag gcc aag ggt gga cat atc cgt gta tcc     480
Ile Thr Ile Lys Gly Gly Gln Ala Lys Gly Gly His Ile Arg Val Ser
145                 150                 155                 160

ggt tcg gtc agc tct cag tat ttg agc gcg tta ctg ttt gta act cct     528
Gly Ser Val Ser Ser Gln Tyr Leu Ser Ala Leu Leu Phe Val Thr Pro
                165                 170                 175

ctt ctg gcc gaa gac agc acg att gaa gta tta aac gac ttg aaa tcc     576
Leu Leu Ala Glu Asp Ser Thr Ile Glu Val Leu Asn Asp Leu Lys Ser
            180                 185                 190

aaa gtg gtt att ggc cag acg ctg gaa gta ttg gaa cag gcg ggc atc     624
Lys Val Val Ile Gly Gln Thr Leu Glu Val Leu Glu Gln Ala Gly Ile
        195                 200                 205

gtc att cat gcg agt gat gat tac atg tcc ttc cgc gta cct ggc ggt     672
Val Ile His Ala Ser Asp Asp Tyr Met Ser Phe Arg Val Pro Gly Gly
    210                 215                 220

caa gcc tat aaa cca caa aca tat acg gtt caa gga gac tat ccg ggt     720
Gln Ala Tyr Lys Pro Gln Thr Tyr Thr Val Gln Gly Asp Tyr Pro Gly
225                 230                 235                 240

tca gca gct gtc ctc gcg gcc gcg gct gtc acc caa tcg gat gtt aaa     768
Ser Ala Ala Val Leu Ala Ala Ala Ala Val Thr Gln Ser Asp Val Lys
                245                 250                 255

att ttg cga ttg atg gaa cag agc aaa caa ggt gag cgt gcc att gta     816
Ile Leu Arg Leu Met Glu Gln Ser Lys Gln Gly Glu Arg Ala Ile Val
            260                 265                 270

gac gtt ctg cgc atg atg gaa gtg ccg ttg acg cat gaa aac gat gtg     864
Asp Val Leu Arg Met Met Glu Val Pro Leu Thr His Glu Asn Asp Val
        275                 280                 285

gta cac gtg caa ggt aac ggt aca ttg aaa gcc gtg gaa ttc gat gga     912
Val His Val Gln Gly Asn Gly Thr Leu Lys Ala Val Glu Phe Asp Gly
    290                 295                 300

gat gcc gcg aca gac gcg gtt ttg gcc atg gta gcg gcg gca gtg ttt     960
Asp Ala Ala Thr Asp Ala Val Leu Ala Met Val Ala Ala Ala Val Phe
305                 310                 315                 320

gcg gaa ggc acc tca cgg ttc tat aat gta gag aac tta cgt tac aag     1008
Ala Glu Gly Thr Ser Arg Phe Tyr Asn Val Glu Asn Leu Arg Tyr Lys
                325                 330                 335

gaa tgt gac cga att aca gat tat ttg aac gaa ctg cgg aag gca gga     1056
Glu Cys Asp Arg Ile Thr Asp Tyr Leu Asn Glu Leu Arg Lys Ala Gly
            340                 345                 350

gcc aac gta gaa gaa cgt cag gcc gag att atc gta cat ggt cgt ccg     1104
Ala Asn Val Glu Glu Arg Gln Ala Glu Ile Ile Val His Gly Arg Pro
        355                 360                 365

gaa ggc gtc gaa ggc ggc gtt gag att aac gct cac tac gat cat cgc     1152
Glu Gly Val Glu Gly Gly Val Glu Ile Asn Ala His Tyr Asp His Arg
    370                 375                 380

gta att atg gca ctg acc gtt gtc ggt ctg cgt tcc aaa gaa ccg ctt     1200

Val Ile Met Ala Leu Thr Val Val Gly Leu Arg Ser Lys Glu Pro Leu
385                 390                 395                 400

cgt att cgg gat gca cac cat gta gcc aag tct tat ccg caa tat ttc    1248
Arg Ile Arg Asp Ala His His Val Ala Lys Ser Tyr Pro Gln Tyr Phe
                405                 410                 415

gat cat ttg cag gcg ctt ggc gcc tcg gtt caa tgg gta aaa gag taa    1296
Asp His Leu Gln Ala Leu Gly Ala Ser Val Gln Trp Val Lys Glu  *
            420                 425                 430

<210> 10
<211> 431
<212> PRT
<213> Paenibacillus pabuli

<400> 10
Met Asp Val Ile Val Lys Pro Thr Pro Ser Leu Asn Gly Glu Ile Gly
 1               5                  10                  15
Ala Leu Ser Ser Lys Asn Tyr Thr Thr Arg Tyr Leu Leu Ala Ala Ala
            20                  25                  30
Leu Ala Glu Gly Thr Ser Thr Ile His Tyr Pro Ala His Ser Glu Asp
        35                  40                  45
Ser Asp Ala Met Arg Arg Cys Ile Ser Asp Leu Gly Ala Val Leu Glu
    50                  55                  60
Glu Asp Asp Ser Lys Ile Val Ile Gln Gly Phe Gly Ser His Pro Arg
65                  70                  75                  80
Asp Val Arg Glu Leu Asn Val Gly Asn Ala Gly Ala Val Leu Arg Phe
                85                  90                  95
Leu Met Gly Val Thr Ala Leu Cys Pro Asp Val Thr Phe Val Asn Thr
            100                 105                 110
Tyr Pro Asp Ser Leu Gly Lys Arg Pro His Asp Asp Leu Ile Asp Ala
        115                 120                 125
Leu Gly Gln Leu Gly Val Glu Val Gln His Glu Gln Gly Arg Leu Pro
    130                 135                 140
Ile Thr Ile Lys Gly Gly Gln Ala Lys Gly Gly His Ile Arg Val Ser
145                 150                 155                 160
Gly Ser Val Ser Ser Gln Tyr Leu Ser Ala Leu Leu Phe Val Thr Pro
                165                 170                 175
Leu Leu Ala Glu Asp Ser Thr Ile Glu Val Leu Asn Asp Leu Lys Ser
            180                 185                 190
Lys Val Val Ile Gly Gln Thr Leu Glu Val Leu Glu Gln Ala Gly Ile
        195                 200                 205
Val Ile His Ala Ser Asp Asp Tyr Met Ser Phe Arg Val Pro Gly Gly
    210                 215                 220
Gln Ala Tyr Lys Pro Gln Thr Tyr Thr Val Gln Gly Asp Tyr Pro Gly
225                 230                 235                 240
Ser Ala Ala Val Leu Ala Ala Ala Ala Val Thr Gln Ser Asp Val Lys
                245                 250                 255
Ile Leu Arg Leu Met Glu Gln Ser Lys Gln Gly Glu Arg Ala Ile Val
            260                 265                 270
Asp Val Leu Arg Met Met Glu Val Pro Leu Thr His Glu Asn Asp Val
        275                 280                 285
Val His Val Gln Gly Asn Gly Thr Leu Lys Ala Val Glu Phe Asp Gly
    290                 295                 300
Asp Ala Ala Thr Asp Ala Val Leu Ala Met Val Ala Ala Ala Val Phe
305                 310                 315                 320
Ala Glu Gly Thr Ser Arg Phe Tyr Asn Val Glu Asn Leu Arg Tyr Lys
                325                 330                 335

Glu Cys Asp Arg Ile Thr Asp Tyr Leu Asn Glu Leu Arg Lys Ala Gly
                340                 345                 350
Ala Asn Val Glu Glu Arg Gln Ala Glu Ile Ile Val His Gly Arg Pro
        355                 360                 365
Glu Gly Val Glu Gly Gly Val Glu Ile Asn Ala His Tyr Asp His Arg
    370                 375                 380
Val Ile Met Ala Leu Thr Val Val Gly Leu Arg Ser Lys Glu Pro Leu
385                 390                 395                 400
Arg Ile Arg Asp Ala His His Val Ala Lys Ser Tyr Pro Gln Tyr Phe
                405                 410                 415
Asp His Leu Gln Ala Leu Gly Ala Ser Val Gln Trp Val Lys Glu
                420                 425                 430

<210> 11
<211> 1296
<212> DNA
<213> Paenibacillus pabuli

<220>
<221> CDS
<222> (1)...(1296)

<400> 11
atg gac gtt atc gtt aaa ccg acc cca tcc ctg aac gga gaa att gga    48
Met Asp Val Ile Val Lys Pro Thr Pro Ser Leu Asn Gly Glu Ile Gly
 1               5                  10                  15

gct ttg tct tcc aaa aac tac acc aca cgc tac ttg cta gct gct gct    96
Ala Leu Ser Ser Lys Asn Tyr Thr Thr Arg Tyr Leu Leu Ala Ala Ala
            20                  25                  30

ctg gca gaa ggc acg agt acg atc cat tac ccg gct cac agt gaa gat    144
Leu Ala Glu Gly Thr Ser Thr Ile His Tyr Pro Ala His Ser Glu Asp
        35                  40                  45

agt gac gct atg cgc aga tgt att cgc gat ctt gga gca gtg ctt gaa    192
Ser Asp Ala Met Arg Arg Cys Ile Arg Asp Leu Gly Ala Val Leu Glu
    50                  55                  60

gaa gat gac agc aaa att gtt atc cag ggc ttt ggc agc cat cca cat    240
Glu Asp Asp Ser Lys Ile Val Ile Gln Gly Phe Gly Ser His Pro His
65                  70                  75                  80

gat gtg cgt gaa tta aat gta ggc aat gcg ggt gca gtg ctg cgt ttc    288
Asp Val Arg Glu Leu Asn Val Gly Asn Ala Gly Ala Val Leu Arg Phe
                85                  90                  95

cta atg ggg gta aca gca ctt tgt cct gag gtg acg ttt gta aat acg    336
Leu Met Gly Val Thr Ala Leu Cys Pro Glu Val Thr Phe Val Asn Thr
            100                 105                 110

tac ccg gat tct ctt ggc aaa cgc cca cat gat gac ctg atc gat gcg    384
Tyr Pro Asp Ser Leu Gly Lys Arg Pro His Asp Asp Leu Ile Asp Ala
        115                 120                 125

ctt ggt cag ctc ggt gtt gag gta cag cac gaa caa gga cgc ttg cca    432
Leu Gly Gln Leu Gly Val Glu Val Gln His Glu Gln Gly Arg Leu Pro
    130                 135                 140

atc acg atc aaa ggt ggt cag gct aag ggt gga cat atc cgt gta tcc    480

Ile Thr Ile Lys Gly Gly Gln Ala Lys Gly Gly His Ile Arg Val Ser
145                 150                 155                 160

ggt tct gtc agc tcc cag tat ttg agc gcg ttg ctg ttt gta act ccg     528
Gly Ser Val Ser Ser Gln Tyr Leu Ser Ala Leu Leu Phe Val Thr Pro
                165                 170                 175

ctt ctg gcc gaa gac agc aca att gaa gta ttg aat gac ttg aaa tcc     576
Leu Leu Ala Glu Asp Ser Thr Ile Glu Val Leu Asn Asp Leu Lys Ser
            180                 185                 190

aaa gtg gtt att ggt cag acg ctg gaa gta ctg gaa caa gcg ggt atc     624
Lys Val Val Ile Gly Gln Thr Leu Glu Val Leu Glu Gln Ala Gly Ile
        195                 200                 205

gtc att cat gcg agt gat gat tac atg tcc ttc cgc gta cct ggc ggc     672
Val Ile His Ala Ser Asp Asp Tyr Met Ser Phe Arg Val Pro Gly Gly
    210                 215                 220

caa gcc tat aaa ccg caa aca tat acg gtt caa gga gac tat ccg ggt     720
Gln Ala Tyr Lys Pro Gln Thr Tyr Thr Val Gln Gly Asp Tyr Pro Gly
225                 230                 235                 240

tca gca gct gtc ctc gcg gct gcg gcg gtc act caa tcc gat gtt aaa     768
Ser Ala Ala Val Leu Ala Ala Ala Ala Val Thr Gln Ser Asp Val Lys
                245                 250                 255

att ttg cga ttg atg gaa cag agc aaa caa ggt gag cgt gct att gtt     816
Ile Leu Arg Leu Met Glu Gln Ser Lys Gln Gly Glu Arg Ala Ile Val
            260                 265                 270

gac gtt ctg cgc atg atg gaa gtg ccg ttg acg cat gag aac gat gtg     864
Asp Val Leu Arg Met Met Glu Val Pro Leu Thr His Glu Asn Asp Val
        275                 280                 285

gtt cac gtg caa ggt aac ggt act ttg aaa gcc gtg gaa ttc gat gga     912
Val His Val Gln Gly Asn Gly Thr Leu Lys Ala Val Glu Phe Asp Gly
    290                 295                 300

gat gcc gcg aca gac gcg gtt ttg gcc atg gta gcg gca gca gtg ttt     960
Asp Ala Ala Thr Asp Ala Val Leu Ala Met Val Ala Ala Ala Val Phe
305                 310                 315                 320

gcg gaa ggc acc tca cgg ttc tat aat gta gag aac tta cgt tac aag     1008
Ala Glu Gly Thr Ser Arg Phe Tyr Asn Val Glu Asn Leu Arg Tyr Lys
                325                 330                 335

gaa tgt gac cga att acg gat tat ttg aac gaa ctg cgg aag gca gga     1056
Glu Cys Asp Arg Ile Thr Asp Tyr Leu Asn Glu Leu Arg Lys Ala Gly
            340                 345                 350

gcc aac gta gaa gaa cgt cag gcc gag att atc gta cat ggt cgt ccg     1104
Ala Asn Val Glu Glu Arg Gln Ala Glu Ile Ile Val His Gly Arg Pro
        355                 360                 365

gaa ggc gtc gaa ggc ggc gtt gag att aac gct cac tac gat cat cgc     1152
Glu Gly Val Glu Gly Gly Val Glu Ile Asn Ala His Tyr Asp His Arg
    370                 375                 380

gta att atg gca ctg acc gtt gtc ggt ctg cgt tcc aaa gaa ccg ctt     1200
Val Ile Met Ala Leu Thr Val Val Gly Leu Arg Ser Lys Glu Pro Leu

385                 390                 395                 400

cgt atc cgg gat gca cac cat gta gcc aag tct tat ccg caa tat ttc    1248
Arg Ile Arg Asp Ala His His Val Ala Lys Ser Tyr Pro Gln Tyr Phe
                405                 410                 415

gat cat ttg cag gcg ctt ggc gcc tcg gtt caa tgg gta aaa gag taa    1296
Asp His Leu Gln Ala Leu Gly Ala Ser Val Gln Trp Val Lys Glu  *
            420                 425                 430

<210> 12
<211> 431
<212> PRT
<213> Paenibacillus pabuli

<400> 12
Met Asp Val Ile Val Lys Pro Thr Pro Ser Leu Asn Gly Glu Ile Gly
 1               5                  10                  15
Ala Leu Ser Ser Lys Asn Tyr Thr Thr Arg Tyr Leu Leu Ala Ala Ala
            20                  25                  30
Leu Ala Glu Gly Thr Ser Thr Ile His Tyr Pro Ala His Ser Glu Asp
        35                  40                  45
Ser Asp Ala Met Arg Arg Cys Ile Arg Asp Leu Gly Ala Val Leu Glu
    50                  55                  60
Glu Asp Asp Ser Lys Ile Val Ile Gln Gly Phe Gly Ser His Pro His
65                  70                  75                  80
Asp Val Arg Glu Leu Asn Val Gly Asn Ala Gly Ala Val Leu Arg Phe
                85                  90                  95
Leu Met Gly Val Thr Ala Leu Cys Pro Glu Val Thr Phe Val Asn Thr
            100                 105                 110
Tyr Pro Asp Ser Leu Gly Lys Arg Pro His Asp Asp Leu Ile Asp Ala
        115                 120                 125
Leu Gly Gln Leu Gly Val Glu Val Gln His Glu Gln Gly Arg Leu Pro
    130                 135                 140
Ile Thr Ile Lys Gly Gly Gln Ala Lys Gly Gly His Ile Arg Val Ser
145                 150                 155                 160
Gly Ser Val Ser Ser Gln Tyr Leu Ser Ala Leu Leu Phe Val Thr Pro
                165                 170                 175
Leu Leu Ala Glu Asp Ser Thr Ile Glu Val Leu Asn Asp Leu Lys Ser
            180                 185                 190
Lys Val Val Ile Gly Gln Thr Leu Glu Val Leu Glu Gln Ala Gly Ile
        195                 200                 205
Val Ile His Ala Ser Asp Asp Tyr Met Ser Phe Arg Val Pro Gly Gly
    210                 215                 220
Gln Ala Tyr Lys Pro Gln Thr Tyr Thr Val Gln Gly Asp Tyr Pro Gly
225                 230                 235                 240
Ser Ala Ala Val Leu Ala Ala Ala Ala Val Thr Gln Ser Asp Val Lys
                245                 250                 255
Ile Leu Arg Leu Met Glu Gln Ser Lys Gln Gly Glu Arg Ala Ile Val
            260                 265                 270
Asp Val Leu Arg Met Met Glu Val Pro Leu Thr His Glu Asn Asp Val
        275                 280                 285
Val His Val Gln Gly Asn Gly Thr Leu Lys Ala Val Glu Phe Asp Gly
    290                 295                 300
Asp Ala Ala Thr Asp Ala Val Leu Ala Met Val Ala Ala Ala Val Phe
305                 310                 315                 320
Ala Glu Gly Thr Ser Arg Phe Tyr Asn Val Glu Asn Leu Arg Tyr Lys
                325                 330                 335
Glu Cys Asp Arg Ile Thr Asp Tyr Leu Asn Glu Leu Arg Lys Ala Gly

340                 345                 350
Ala Asn Val Glu Glu Arg Gln Ala Glu Ile Ile Val His Gly Arg Pro
        355                 360                 365
Glu Gly Val Glu Gly Gly Val Glu Ile Asn Ala His Tyr Asp His Arg
    370                 375                 380
Val Ile Met Ala Leu Thr Val Val Gly Leu Arg Ser Lys Glu Pro Leu
385                 390                 395                 400
Arg Ile Arg Asp Ala His His Val Ala Lys Ser Tyr Pro Gln Tyr Phe
                405                 410                 415
Asp His Leu Gln Ala Leu Gly Ala Ser Val Gln Trp Val Lys Glu
            420                 425                 430

<210> 13
<211> 1251
<212> DNA
<213> Sphingobacterium multivorum/faecium

<220>
<221> CDS
<222> (1)...(1251)

<400> 13
atg aat caa caa gtc atc acg ctg acg cat cct tca aaa aaa ata cag     48
Met Asn Gln Gln Val Ile Thr Leu Thr His Pro Ser Lys Lys Ile Gln
 1               5                  10                  15

ggt acg gtt caa ctc aca ggt tca aaa tct gag agc aac cgt gct ctt     96
Gly Thr Val Gln Leu Thr Gly Ser Lys Ser Glu Ser Asn Arg Ala Leu
            20                  25                  30

att atc cag tca ttg agc aaa gga caa gtt gaa ata gcc aac ctt tct    144
Ile Ile Gln Ser Leu Ser Lys Gly Gln Val Glu Ile Ala Asn Leu Ser
        35                  40                  45

gaa gct gca gat acg gta acg tta aac cgt gtg cta caa att gct tca    192
Glu Ala Ala Asp Thr Val Thr Leu Asn Arg Val Leu Gln Ile Ala Ser
    50                  55                  60

gat ccg aaa cca gga ttc aac aca att gac atc ggt cca gcg gga acg    240
Asp Pro Lys Pro Gly Phe Asn Thr Ile Asp Ile Gly Pro Ala Gly Thr
65                  70                  75                  80

gcc atg cga ttc tta acg gct tac ctc aac ctt gtc aaa gga aat ttt    288
Ala Met Arg Phe Leu Thr Ala Tyr Leu Asn Leu Val Lys Gly Asn Phe
                85                  90                  95

atc ctt aca ggt act gaa cgc atg caa cag cgt cct ata ggt ata tta    336
Ile Leu Thr Gly Thr Glu Arg Met Gln Gln Arg Pro Ile Gly Ile Leu
            100                 105                 110

gtt gat gcc atg aaa gaa att ggt gca gac atc cac tat gac aag aaa    384
Val Asp Ala Met Lys Glu Ile Gly Ala Asp Ile His Tyr Asp Lys Lys
        115                 120                 125

gtc gga tac cct cct ttg aaa att gag ggc ggg ctg ttt caa gaa aaa    432
Val Gly Tyr Pro Pro Leu Lys Ile Glu Gly Gly Leu Phe Gln Glu Lys
    130                 135                 140

gac cgt gtc aag att aaa ggt aat atc agc agc caa tat ata tca gcc    480
Asp Arg Val Lys Ile Lys Gly Asn Ile Ser Ser Gln Tyr Ile Ser Ala

145                 150                 155                 160

ctc tta tta att gca cct gca tta aaa aaa ggg ctt act tta gag att     528
Leu Leu Leu Ile Ala Pro Ala Leu Lys Lys Gly Leu Thr Leu Glu Ile
                165                 170                 175

gag ggt gaa tta acc tcc aga cct tat gta tca atg acc ttg gat atg     576
Glu Gly Glu Leu Thr Ser Arg Pro Tyr Val Ser Met Thr Leu Asp Met
            180                 185                 190

ctg aaa agt gtc ggg att cag cat gaa tgg aaa aac aat gcg att aaa     624
Leu Lys Ser Val Gly Ile Gln His Glu Trp Lys Asn Asn Ala Ile Lys
        195                 200                 205

att gcg ccg cag gca ttt gag aaa caa aca ata tat gtc gag cca gat     672
Ile Ala Pro Gln Ala Phe Glu Lys Gln Thr Ile Tyr Val Glu Pro Asp
    210                 215                 220

tgg agc gct gct tcc tat tgg tac gct atc gcc gca cta gca gat gca     720
Trp Ser Ala Ala Ser Tyr Trp Tyr Ala Ile Ala Ala Leu Ala Asp Ala
225                 230                 235                 240

aac gca tcg atc gta ttg ccc gga tta aga aaa aac agc tta cag ggt     768
Asn Ala Ser Ile Val Leu Pro Gly Leu Arg Lys Asn Ser Leu Gln Gly
                245                 250                 255

gat att gct att ata agc att atg gag cat ttt ggt gta caa tcg agc     816
Asp Ile Ala Ile Ile Ser Ile Met Glu His Phe Gly Val Gln Ser Ser
            260                 265                 270

ttt gaa tcg gac gga tta cac tta aat aaa aag gta atc ggt tcg gat     864
Phe Glu Ser Asp Gly Leu His Leu Asn Lys Lys Val Ile Gly Ser Asp
        275                 280                 285

gta agc tta ttt aac ttt aaa gaa tgt ccc gat ctc gca caa act gta     912
Val Ser Leu Phe Asn Phe Lys Glu Cys Pro Asp Leu Ala Gln Thr Val
    290                 295                 300

gtt gtt gtc gcg gct gcg tta aaa cga gat gta tct ttt acg ggc ttg     960
Val Val Val Ala Ala Ala Leu Lys Arg Asp Val Ser Phe Thr Gly Leu
305                 310                 315                 320

gag acc tta aaa att aag gag act gac cgt atc gcg gca cta caa aag     1008
Glu Thr Leu Lys Ile Lys Glu Thr Asp Arg Ile Ala Ala Leu Gln Lys
                325                 330                 335

gaa att gcg aaa ttt gga gcc gag cta att gaa gat ggc gat acc tac     1056
Glu Ile Ala Lys Phe Gly Ala Glu Leu Ile Glu Asp Gly Asp Thr Tyr
            340                 345                 350

cat ctg aaa aca gcg cag gta tat cag cct gaa gag gtt act ttc gat     1104
His Leu Lys Thr Ala Gln Val Tyr Gln Pro Glu Glu Val Thr Phe Asp
        355                 360                 365

act tac gaa gat cat cgc atg gcg atg gcg ttc gca cca ctg gca tta     1152
Thr Tyr Glu Asp His Arg Met Ala Met Ala Phe Ala Pro Leu Ala Leu
    370                 375                 380

gtt ttc gac cag att aag att gct gaa cct caa gtt gta gaa aaa tca     1200
Val Phe Asp Gln Ile Lys Ile Ala Glu Pro Gln Val Val Glu Lys Ser
385                 390                 395                 400

tat cct gat ttt tgg aat cat tta cag gcg caa gct ttt gtc att gaa    1248
Tyr Pro Asp Phe Trp Asn His Leu Gln Ala Gln Ala Phe Val Ile Glu
                405                 410                 415

tag                                                                1251
 *

<210> 14
<211> 416
<212> PRT
<213> Sphingobacterium multivorum/faecium

<400> 14
Met Asn Gln Gln Val Ile Thr Leu Thr His Pro Ser Lys Lys Ile Gln
 1               5                  10                  15
Gly Thr Val Gln Leu Thr Gly Ser Lys Ser Glu Ser Asn Arg Ala Leu
            20                  25                  30
Ile Ile Gln Ser Leu Ser Lys Gly Gln Val Glu Ile Ala Asn Leu Ser
        35                  40                  45
Glu Ala Ala Asp Thr Val Thr Leu Asn Arg Val Leu Gln Ile Ala Ser
    50                  55                  60
Asp Pro Lys Pro Gly Phe Asn Thr Ile Asp Ile Gly Pro Ala Gly Thr
65                  70                  75                  80
Ala Met Arg Phe Leu Thr Ala Tyr Leu Asn Leu Val Lys Gly Asn Phe
                85                  90                  95
Ile Leu Thr Gly Thr Glu Arg Met Gln Gln Arg Pro Ile Gly Ile Leu
            100                 105                 110
Val Asp Ala Met Lys Glu Ile Gly Ala Asp Ile His Tyr Asp Lys Lys
        115                 120                 125
Val Gly Tyr Pro Pro Leu Lys Ile Glu Gly Gly Leu Phe Gln Glu Lys
    130                 135                 140
Asp Arg Val Lys Ile Lys Gly Asn Ile Ser Ser Gln Tyr Ile Ser Ala
145                 150                 155                 160
Leu Leu Leu Ile Ala Pro Ala Leu Lys Lys Gly Leu Thr Leu Glu Ile
                165                 170                 175
Glu Gly Glu Leu Thr Ser Arg Pro Tyr Val Ser Met Thr Leu Asp Met
            180                 185                 190
Leu Lys Ser Val Gly Ile Gln His Glu Trp Lys Asn Asn Ala Ile Lys
        195                 200                 205
Ile Ala Pro Gln Ala Phe Glu Lys Gln Thr Ile Tyr Val Glu Pro Asp
    210                 215                 220
Trp Ser Ala Ala Ser Tyr Trp Tyr Ala Ile Ala Ala Leu Ala Asp Ala
225                 230                 235                 240
Asn Ala Ser Ile Val Leu Pro Gly Leu Arg Lys Asn Ser Leu Gln Gly
                245                 250                 255
Asp Ile Ala Ile Ile Ser Ile Met Glu His Phe Gly Val Gln Ser Ser
            260                 265                 270
Phe Glu Ser Asp Gly Leu His Leu Asn Lys Lys Val Ile Gly Ser Asp
        275                 280                 285
Val Ser Leu Phe Asn Phe Lys Glu Cys Pro Asp Leu Ala Gln Thr Val
    290                 295                 300
Val Val Val Ala Ala Ala Leu Lys Arg Asp Val Ser Phe Thr Gly Leu
305                 310                 315                 320
Glu Thr Leu Lys Ile Lys Glu Thr Asp Arg Ile Ala Ala Leu Gln Lys
                325                 330                 335
Glu Ile Ala Lys Phe Gly Ala Glu Leu Ile Glu Asp Gly Asp Thr Tyr
            340                 345                 350
His Leu Lys Thr Ala Gln Val Tyr Gln Pro Glu Glu Val Thr Phe Asp

Thr Tyr Glu Asp His Arg Met Ala Met Ala Phe Ala Pro Leu Ala Leu
    370                 375                 380
Val Phe Asp Gln Ile Lys Ile Ala Glu Pro Gln Val Val Glu Lys Ser
385                 390                 395                 400
Tyr Pro Asp Phe Trp Asn His Leu Gln Ala Gln Ala Phe Val Ile Glu
                405                 410                 415

<210> 15
<211> 431
<212> PRT
<213> Enterobacteriaceae sp.

<400> 15
Met Lys Val Thr Ile Gln Pro Gly Asp Leu Thr Gly Ile Leu Gln Ser
 1               5                  10                  15
Pro Ala Ser Lys Ser Ser Met Gln Arg Ala Cys Ala Ala Ala Leu Val
            20                  25                  30
Ala Lys Gly Ile Ser Glu Ile Ile Asn Pro Gly His Ser Asn Asp Asp
        35                  40                  45
Lys Ala Ala Arg Asp Ile Val Ser Arg Leu Gly Ala Arg Leu Glu Asp
    50                  55                  60
Gln Pro Asp Gly Ser Leu Gln Ile Thr Ser Glu Gly Val Lys Pro Val
65                  70                  75                  80
Ala Pro Phe Ile Asp Cys Gly Glu Ser Gly Leu Ser Ile Arg Met Phe
                85                  90                  95
Thr Pro Ile Val Ala Leu Ser Lys Glu Glu Val Thr Ile Lys Gly Ser
            100                 105                 110
Gly Ser Leu Val Thr Arg Pro Met Asp Phe Phe Asp Glu Ile Leu Pro
        115                 120                 125
His Leu Gly Val Lys Val Lys Ser Asn Gln Gly Lys Leu Pro Leu Val
    130                 135                 140
Ile Gln Gly Pro Leu Lys Pro Ala Asp Val Thr Val Asp Gly Ser Leu
145                 150                 155                 160
Ser Ser Gln Phe Leu Thr Gly Leu Leu Leu Ala Tyr Ala Ala Ala Asp
                165                 170                 175
Ala Ser Asp Val Ala Ile Lys Val Thr Asn Leu Lys Ser Arg Pro Tyr
            180                 185                 190
Ile Asp Leu Thr Leu Asp Val Met Lys Arg Phe Gly Leu Lys Thr Pro
        195                 200                 205
Glu Asn Arg Asn Tyr Glu Glu Phe Tyr Phe Lys Ala Gly Asn Val Tyr
    210                 215                 220
Asp Glu Thr Lys Met Gln Arg Tyr Thr Val Glu Gly Asp Trp Ser Gly
225                 230                 235                 240
Gly Ala Phe Leu Leu Val Ala Gly Ala Ile Ala Gly Pro Ile Thr Val
                245                 250                 255
Arg Gly Leu Asp Ile Ala Ser Thr Gln Ala Asp Lys Ala Ile Val Gln
            260                 265                 270
Ala Leu Met Ser Ala Asn Ala Gly Ile Ala Ile Asp Ala Lys Glu Ile
        275                 280                 285
Lys Leu His Pro Ala Asp Leu Asn Ala Phe Glu Phe Asp Ala Thr Asp
    290                 295                 300
Cys Pro Asp Leu Phe Pro Pro Leu Val Ala Leu Ala Ser Tyr Cys Lys
305                 310                 315                 320
Gly Glu Thr Lys Ile Lys Gly Val Ser Arg Leu Ala His Lys Glu Ser
                325                 330                 335
Asp Arg Gly Leu Thr Leu Gln Asp Glu Phe Gly Lys Met Gly Val Glu
            340                 345                 350
Ile His Leu Glu Gly Asp Leu Met Arg Val Ile Gly Gly Lys Gly Val
        355                 360                 365

Lys Gly Ala Glu Val Ser Ser Arg His Asp His Arg Ile Ala Met Ala
    370                 375                 380
Cys Ala Val Ala Ala Leu Lys Ala Val Gly Glu Thr Thr Ile Glu His
385                 390                 395                 400
Ala Glu Ala Val Asn Lys Ser Tyr Pro Asp Phe Tyr Ser Asp Leu Lys
                405                 410                 415
Gln Leu Gly Gly Val Val Ser Leu Asn His Gln Phe Asn Phe Ser
            420                 425                 430

<210> 16
<211> 418
<212> PRT
<213> Pseudomonas syringae strain 1448a

<400> 16
Met Arg Pro Gln Ala Thr Leu Thr Val Leu Pro Val Glu Arg Pro Leu
 1               5                  10                  15
Val Gly Arg Val Ser Pro Pro Gly Ser Lys Ser Ile Thr Asn Arg Ala
            20                  25                  30
Leu Leu Leu Ala Gly Leu Ala Lys Gly Thr Ser Arg Leu Thr Gly Ala
        35                  40                  45
Leu Lys Ser Asp Asp Thr Arg Val Met Ser Glu Ala Leu Arg Leu Met
    50                  55                  60
Gly Val Gln Val Asp Glu Pro Asp Asp Ser Thr Phe Val Val Thr Ser
65                  70                  75                  80
Ser Gly His Trp Gln Ala Pro Gln Gln Ala Leu Phe Leu Gly Asn Ala
                85                  90                  95
Gly Thr Ala Thr Arg Phe Leu Thr Ala Ala Leu Ala Asn Phe Glu Gly
            100                 105                 110
Asp Phe Val Val Asp Gly Asp Glu Tyr Met Arg Lys Arg Pro Ile Gly
        115                 120                 125
Pro Leu Val Asp Ala Leu Gln Arg Met Gly Val Glu Val Ser Ala Pro
    130                 135                 140
Ser Gly Cys Pro Pro Val Ala Ile Lys Gly Lys Gly Gly Leu Glu Ala
145                 150                 155                 160
Gly Arg Ile Glu Ile Asp Gly Asn Leu Ser Ser Gln Tyr Val Ser Ala
                165                 170                 175
Leu Leu Met Ala Gly Ala Cys Gly Lys Gly Pro Val Glu Val Ala Leu
            180                 185                 190
Thr Gly Ser Glu Ile Gly Ala Arg Gly Tyr Val Asp Leu Thr Leu Ala
        195                 200                 205
Ala Met Gln Ala Phe Gly Ala Glu Val Gln Ala Ile Gly Glu Thr Ala
    210                 215                 220
Trp Lys Val Ser Ala Thr Gly Tyr Arg Ala Thr Asp Phe His Ile Glu
225                 230                 235                 240
Pro Asp Ala Ser Ala Ala Thr Tyr Leu Trp Ala Ala Gln Ala Leu Thr
                245                 250                 255
Glu Gly Asp Ile Asp Leu Gly Val Ala Ser Asp Ala Phe Thr Gln Pro
            260                 265                 270
Asp Ala Leu Ala Ser Gln Ile Ile Ala Ser Phe Pro Asn Met Pro Ala
        275                 280                 285
Val Ile Asp Gly Ser Gln Met Gln Asp Ala Ile Pro Thr Leu Ala Val
    290                 295                 300
Leu Ala Ala Phe Asn Arg Gln Pro Val Arg Phe Val Gly Ile Ala Asn
305                 310                 315                 320
Leu Arg Val Lys Glu Cys Asp Arg Ile Ser Ala Leu Ser His Gly Leu
                325                 330                 335
Cys Ala Ile Ala Pro Gly Leu Ala Val Glu Glu Gly Asp Asp Leu Leu
            340                 345                 350
Val His Ala Asn Pro Ala Leu Ala Gly Thr Thr Val Asp Ala Leu Ile

355                 360                 365
Asp Thr His Ser Asp His Arg Ile Ala Met Cys Phe Ala Leu Ala Gly
    370                 375                 380
Leu Lys Ile Ala Gly Ile Arg Ile Leu Asp Pro Asp Cys Val Gly Lys
385                 390                 395                 400
Thr Tyr Pro Gly Tyr Trp Asp Ala Leu Ala Ser Leu Gly Val Arg Val
                405                 410                 415
Gln Arg

<210> 17
<211> 418
<212> PRT
<213> Pseudomonas syringae strain DC3000

<400> 17
Met Arg Pro Gln Ala Thr Leu Thr Val Met Pro Val Glu Arg Pro Leu
 1               5                  10                  15
Val Gly Arg Val Ser Pro Pro Gly Ser Lys Ser Ile Thr Asn Arg Ala
            20                  25                  30
Leu Leu Leu Ala Gly Leu Ala Lys Gly Thr Ser Arg Leu Thr Gly Ala
        35                  40                  45
Leu Lys Ser Asp Asp Thr Arg Val Met Ser Glu Ala Leu Arg Leu Met
    50                  55                  60
Gly Val Gln Val Asp Glu Pro Asp Asp Ser Thr Phe Val Val Thr Ser
65                  70                  75                  80
Ser Gly His Trp Gln Ala Pro Gln Gln Ala Leu Phe Leu Gly Asn Ala
                85                  90                  95
Gly Thr Ala Thr Arg Phe Leu Thr Ala Ala Leu Ala Asn Phe Glu Gly
            100                 105                 110
Asp Phe Val Val Asp Gly Asp Glu Tyr Met Arg Lys Arg Pro Ile Gly
        115                 120                 125
Pro Leu Val Asp Ala Leu Gln Arg Met Gly Val Glu Ile Ser Ala Pro
    130                 135                 140
Ser Gly Cys Pro Pro Val Ala Ile Lys Gly Lys Gly Gly Leu Gln Ala
145                 150                 155                 160
Gly Arg Ile Glu Ile Asp Gly Asn Leu Ser Ser Gln Tyr Val Ser Ala
                165                 170                 175
Leu Leu Met Ala Gly Ala Cys Gly Lys Gly Ser Leu Glu Val Ala Leu
            180                 185                 190
Thr Gly Ser Glu Ile Gly Ala Arg Gly Tyr Val Asp Leu Thr Leu Ala
        195                 200                 205
Ala Met Gln Ala Phe Gly Ala Glu Val Gln Ala Ile Gly Asp Ala Ala
    210                 215                 220
Trp Lys Val Ser Ala Thr Gly Tyr His Ala Thr Asp Phe His Ile Glu
225                 230                 235                 240
Pro Asp Ala Ser Ala Ala Thr Tyr Leu Trp Ala Ala Gln Ala Leu Thr
                245                 250                 255
Glu Gly Asn Ile Asp Leu Gly Val Ala Ser Asp Ala Phe Thr Gln Pro
            260                 265                 270
Asp Ala Leu Ala Ser Gln Ile Ile Asp Ser Phe Pro Asn Met Pro Ala
        275                 280                 285
Val Ile Asp Gly Ser Gln Met Gln Asp Ala Ile Pro Thr Leu Ala Val
    290                 295                 300
Leu Ala Ala Phe Asn Arg Gln Pro Val Arg Phe Val Gly Ile Ala Asn
305                 310                 315                 320
Leu Arg Val Lys Glu Cys Asp Arg Ile Ser Ala Leu Cys Asp Gly Leu
                325                 330                 335
Cys Ala Ile Ala Pro Gly Leu Ala Val Glu Glu Gly Asp Asp Leu Ile
            340                 345                 350

Val His Ala Asn Pro Ala Leu Ala Gly Thr Thr Val Asn Ala Leu Ile
        355                 360                 365
Asp Thr His Ser Asp His Arg Ile Ala Met Cys Phe Ala Leu Ala Gly
    370                 375                 380
Leu Lys Ile Lys Gly Ile His Ile Gln Asp Pro Asp Cys Val Ala Lys
385                 390                 395                 400
Thr Tyr Pro Gly Tyr Trp Asp Ala Leu Ala Ser Leu Gly Val Ser Val
                405                 410                 415
Gln Arg

<210> 18
<211> 418
<212> PRT
<213> Pseudomonas syringae strain B728

<400> 18
Met Arg Pro Gln Ala Thr Leu Thr Val Leu Pro Val Glu Arg Pro Leu
 1               5                  10                  15
Val Gly Arg Val Ser Pro Pro Gly Ser Lys Ser Ile Thr Asn Arg Ala
            20                  25                  30
Leu Leu Leu Ala Gly Leu Ala Lys Gly Thr Ser Arg Leu Thr Gly Ala
        35                  40                  45
Leu Lys Ser Asp Asp Thr Arg Val Met Ser Glu Ala Leu Arg Leu Met
    50                  55                  60
Gly Val Gln Val Asp Glu Pro Asp Asp Ser Thr Phe Val Val Thr Ser
65                  70                  75                  80
Ser Gly His Trp Gln Ala Pro Gln Gln Ala Leu Phe Leu Gly Asn Ala
                85                  90                  95
Gly Thr Ala Thr Arg Phe Leu Thr Ala Ala Leu Ala Asn Phe Glu Gly
            100                 105                 110
Asp Phe Val Val Asp Gly Asp Glu Tyr Met Arg Lys Arg Pro Ile Gly
        115                 120                 125
Pro Leu Val Asp Ala Leu Gln Arg Met Gly Val Glu Val Ser Ala Pro
    130                 135                 140
Ser Gly Cys Pro Pro Val Ala Ile Lys Gly Lys Gly Gly Leu Glu Ala
145                 150                 155                 160
Gly Arg Ile Glu Ile Asp Gly Asn Leu Ser Ser Gln Tyr Val Ser Ala
                165                 170                 175
Leu Leu Met Ala Gly Ala Cys Gly Lys Gly Pro Val Glu Val Ala Leu
            180                 185                 190
Thr Gly Ser Glu Ile Gly Ala Arg Gly Tyr Leu Asp Leu Thr Leu Ala
        195                 200                 205
Ala Met Arg Ala Phe Gly Ala Glu Val Gln Ala Ile Gly Asp Ala Ala
    210                 215                 220
Trp Lys Val Ser Ala Thr Gly Tyr Arg Ala Thr Asp Phe His Ile Glu
225                 230                 235                 240
Pro Asp Ala Ser Ala Ala Thr Tyr Leu Trp Ala Ala Gln Ala Leu Thr
                245                 250                 255
Glu Gly Ala Ile Asp Leu Gly Val Ala Ser Asn Ala Phe Thr Gln Pro
            260                 265                 270
Asp Ala Leu Ala Ser Gln Ile Ile Ala Ser Phe Pro Asn Met Pro Ala
        275                 280                 285
Val Ile Asp Gly Ser Gln Met Gln Asp Ala Ile Pro Thr Leu Ala Val
    290                 295                 300
Leu Ala Ala Phe Asn Arg Gln Pro Val Arg Phe Val Gly Ile Ala Asn
305                 310                 315                 320
Leu Arg Val Lys Glu Cys Asp Arg Ile Ser Ala Leu Ser Asn Gly Leu
                325                 330                 335
Cys Ala Ile Ala Pro Gly Leu Ala Val Glu Glu Gly Asp Asp Leu Ile

                340                 345                 350
Val Thr Ala Asn Pro Thr Leu Ala Gly Thr Thr Val Asp Ala Leu Ile
        355                 360                 365
Asp Thr His Ser Asp His Arg Ile Ala Met Cys Phe Ala Leu Ala Gly
    370                 375                 380
Leu Lys Ile Ala Gly Ile Arg Ile Leu Asp Pro Asp Cys Val Ala Lys
385                 390                 395                 400
Thr Tyr Pro Gly Tyr Trp Asp Ala Leu Ala Ser Leu Gly Val Ser Val
                405                 410                 415
Gln Arg

<210> 19
<211> 419
<212> PRT
<213> Brevundomonas vesicularis

<400> 19
Met Met Met Gly Arg Ala Lys Leu Thr Ile Ile Pro Pro Gly Lys Pro
 1               5                  10                  15
Leu Thr Gly Arg Ala Met Pro Pro Gly Ser Lys Ser Ile Thr Asn Arg
            20                  25                  30
Ala Leu Leu Leu Ala Gly Leu Ala Lys Gly Thr Ser Arg Leu Thr Gly
        35                  40                  45
Ala Leu Lys Ser Asp Asp Thr Arg Tyr Met Ala Glu Ala Leu Arg Ala
    50                  55                  60
Met Gly Val Thr Ile Asp Glu Pro Asp Asp Thr Thr Phe Ile Val Lys
65                  70                  75                  80
Gly Ser Gly Lys Leu Gln Pro Pro Ala Ala Pro Leu Phe Leu Gly Asn
                85                  90                  95
Ala Gly Thr Ala Thr Arg Phe Leu Thr Ala Ala Ala Ala Leu Val Asp
            100                 105                 110
Gly Lys Val Ile Val Asp Gly Asp Ala His Met Arg Lys Arg Pro Ile
        115                 120                 125
Gly Pro Leu Val Asp Ala Leu Arg Ser Leu Gly Ile Asp Ala Ser Ala
    130                 135                 140
Glu Thr Gly Cys Pro Pro Val Thr Ile Asn Gly Thr Gly Arg Phe Glu
145                 150                 155                 160
Ala Ser Arg Val Gln Ile Asp Gly Gly Leu Ser Ser Gln Tyr Val Ser
                165                 170                 175
Ala Leu Leu Met Met Ala Ala Gly Gly Asp Arg Ala Val Asp Val Glu
            180                 185                 190
Leu Leu Gly Glu His Ile Gly Ala Leu Gly Tyr Ile Asp Leu Thr Val
        195                 200                 205
Ala Ala Met Arg Ala Phe Gly Ala Lys Val Glu Arg Val Ser Pro Val
    210                 215                 220
Ala Trp Arg Val Glu Pro Thr Gly Tyr His Ala Ala Asp Phe Val Ile
225                 230                 235                 240
Glu Pro Asp Ala Ser Ala Ala Thr Tyr Leu Trp Ala Ala Glu Val Leu
                245                 250                 255
Ser Gly Gly Lys Ile Asp Leu Gly Thr Pro Ala Glu Gln Phe Ser Gln
            260                 265                 270
Pro Asp Ala Lys Ala Tyr Asp Leu Ile Ser Lys Phe Pro His Leu Pro
        275                 280                 285
Ala Val Ile Asp Gly Ser Gln Met Gln Asp Ala Ile Pro Thr Leu Ala
    290                 295                 300
Val Leu Ala Ala Phe Asn Glu Met Pro Val Arg Phe Val Gly Ile Glu
305                 310                 315                 320
Asn Leu Arg Val Lys Glu Cys Asp Arg Ile Arg Ala Leu Ser Ser Gly
                325                 330                 335

Leu Ser Arg Ile Val Pro Asn Leu Gly Thr Glu Glu Gly Asp Asp Leu
            340                 345                 350
Ile Ile Ala Ser Asp Pro Ser Leu Ala Gly Lys Ile Leu Thr Ala Glu
        355                 360                 365
Ile Asp Ser Phe Ala Asp His Arg Ile Ala Met Ser Phe Ala Leu Ala
    370                 375                 380
Gly Leu Lys Ile Gly Gly Ile Thr Ile Leu Asp Pro Asp Cys Val Ala
385                 390                 395                 400
Lys Thr Phe Pro Ser Tyr Trp Asn Val Leu Ser Ser Leu Gly Val Ala
                405                 410                 415
Tyr Glu Asp

<210> 20
<211> 425
<212> PRT
<213> Agrobacterium tumefaciens strain C58

<400> 20
Met Ile Glu Leu Thr Ile Thr Pro Pro Gly His Pro Leu Ser Gly Lys
 1               5                  10                  15
Val Glu Pro Pro Gly Ser Lys Ser Ile Thr Asn Arg Ala Leu Leu Leu
            20                  25                  30
Ala Gly Leu Ala Lys Gly Lys Ser His Leu Ser Gly Ala Leu Lys Ser
        35                  40                  45
Asp Asp Thr Leu Tyr Met Ala Glu Ala Leu Arg Glu Met Gly Val Lys
    50                  55                  60
Val Thr Glu Pro Asp Ala Thr Thr Phe Val Val Glu Gly Thr Gly Val
65                  70                  75                  80
Leu Gln Gln Pro Glu Lys Pro Leu Phe Leu Gly Asn Ala Gly Thr Ala
                85                  90                  95
Thr Arg Phe Leu Thr Ala Ala Gly Ala Leu Val Asp Gly Ala Val Ile
            100                 105                 110
Ile Asp Gly Asp Glu His Met Arg Lys Arg Pro Ile Leu Pro Leu Val
        115                 120                 125
Gln Ala Leu Arg Ala Leu Gly Val Glu Ala Asp Ala Pro Thr Gly Cys
    130                 135                 140
Pro Pro Val Thr Val Arg Gly Lys Gly Met Gly Phe Pro Lys Gly Ser
145                 150                 155                 160
Val Thr Ile Asp Ala Asn Leu Ser Ser Gln Tyr Val Ser Ala Leu Leu
                165                 170                 175
Met Ala Ala Ala Cys Gly Asp Lys Pro Val Asp Ile Ile Leu Lys Gly
            180                 185                 190
Glu Glu Ile Gly Ala Lys Gly Tyr Ile Asp Leu Thr Thr Ser Ala Met
        195                 200                 205
Glu Ala Phe Gly Ala Lys Val Glu Arg Val Ser Asn Ala Ile Trp Arg
    210                 215                 220
Val His Pro Thr Gly Tyr Thr Ala Thr Asp Phe His Ile Glu Pro Asp
225                 230                 235                 240
Ala Ser Ala Ala Thr Tyr Leu Trp Gly Ala Glu Leu Leu Thr Gly Gly
                245                 250                 255
Ala Ile Asp Ile Gly Thr Pro Ala Asp Lys Phe Thr Gln Pro Asp Ala
            260                 265                 270
Lys Ala Tyr Glu Val Met Ala Gln Phe Pro His Leu Pro Ala Glu Ile
        275                 280                 285
Asp Gly Ser Gln Met Gln Asp Ala Ile Pro Thr Ile Ala Val Ile Ala
    290                 295                 300
Ala Phe Asn Glu Thr Pro Val Arg Phe Val Gly Ile Ala Asn Leu Arg
305                 310                 315                 320
Val Lys Glu Cys Asp Arg Ile Arg Ala Val Ser Leu Gly Leu Asn Glu

                    325                     330                     335
Ile Arg Glu Gly Leu Ala His Glu Glu Gly Asp Asp Leu Ile Val His
                340                     345                     350
Ala Asp Pro Ser Leu Ala Gly Gln Thr Val Asp Ala Ser Ile Asp Thr
            355                     360                     365
Phe Ala Asp His Arg Ile Ala Met Ser Phe Ala Leu Ala Ala Leu Lys
    370                     375                     380
Ile Gly Gly Ile Ala Ile Gln Asn Pro Ala Cys Val Ala Lys Thr Tyr
385                     390                     395                     400
Pro Gly Tyr Trp Lys Ala Leu Ala Ser Leu Gly Val Asp Tyr Thr Glu
                405                     410                     415
Lys Glu Ser Ala Ala Glu Pro Gln His
                420                     425

<210> 21
<211> 428
<212> PRT
<213> Clostridium acetobutylicum

<400> 21
Met Asn Cys Val Lys Ile Asn Pro Cys Cys Leu Lys Gly Asp Ile Lys
 1               5                  10                  15
Ile Pro Pro Ser Lys Ser Leu Gly His Arg Ala Ile Ile Cys Ala Ala
                20                  25                  30
Leu Ser Glu Glu Glu Ser Thr Ile Glu Asn Ile Ser Tyr Ser Lys Asp
            35                  40                  45
Ile Lys Ala Thr Cys Ile Gly Met Ser Lys Leu Gly Ala Leu Ile Ile
    50                  55                  60
Glu Asp Ala Lys Asp Asn Ser Thr Leu Lys Ile Lys Lys Gln Lys Leu
65                  70                  75                  80
Val Ser Lys Glu Lys Val Tyr Ile Asp Cys Ser Glu Ser Gly Ser Thr
                85                  90                  95
Val Arg Phe Leu Ile Pro Ile Ser Leu Ile Glu Glu Arg Asn Val Val
                100                 105                 110
Phe Asp Gly Gln Gly Lys Leu Ser Tyr Arg Pro Leu Asp Ser Tyr Phe
            115                 120                 125
Asn Ile Phe Asp Glu Lys Glu Ile Ala Tyr Ser His Pro Glu Gly Lys
    130                 135                 140
Val Leu Pro Leu Gln Ile Lys Gly Arg Leu Lys Ala Gly Met Phe Asn
145                 150                 155                 160
Leu Pro Gly Asn Ile Ser Ser Gln Phe Ile Ser Gly Leu Met Phe Ser
                165                 170                 175
Leu Pro Phe Leu Glu Gly Asp Ser Ile Ile Asn Ile Thr Thr Asn Leu
                180                 185                 190
Glu Ser Val Gly Tyr Val Asp Met Thr Ile Asp Met Leu Lys Lys Phe
            195                 200                 205
Gly Ile Glu Ile Glu Asn Lys Ala Tyr Lys Ser Phe Phe Ile Lys Gly
    210                 215                 220
Asn Gln Lys Cys Lys Gly Thr Lys Tyr Lys Val Glu Gly Asp Phe Ser
225                 230                 235                 240
Gln Ala Ala Phe Trp Leu Ser Ala Gly Ile Leu Asn Gly Asn Ile Asn
                245                 250                 255
Cys Lys Asp Leu Asn Ile Ser Ser Leu Gln Gly Asp Lys Val Ile Leu
                260                 265                 270
Asp Ile Leu Lys Lys Met Gly Gly Ala Ile Asp Glu Lys Ser Phe Ser
            275                 280                 285
Ser Lys Lys Ser His Thr His Gly Ile Val Ile Asp Ala Ser Gln Cys
    290                 295                 300
Pro Asp Leu Val Pro Ile Leu Ser Val Val Ala Ala Leu Ser Glu Gly
305                 310                 315                 320

Thr Thr Lys Ile Val Asn Ala Ala Arg Leu Arg Ile Lys Glu Ser Asp
                325                 330                 335
Arg Leu Lys Ala Met Ala Thr Glu Leu Asn Lys Leu Gly Ala Glu Val
            340                 345                 350
Val Glu Leu Glu Asp Gly Leu Leu Ile Glu Gly Lys Glu Lys Leu Lys
        355                 360                 365
Gly Gly Glu Val Glu Ser Trp Asn Asp His Arg Ile Ala Met Ala Leu
    370                 375                 380
Gly Ile Ala Ala Leu Arg Cys Glu Glu Ser Val Thr Ile Asn Gly Ser
385                 390                 395                 400
Glu Cys Val Ser Lys Ser Tyr Pro Gln Phe Trp Ser Asp Leu Lys Gln
                405                 410                 415
Leu Gly Gly Asp Val His Glu Trp Ser Leu Gly Glu
            420                 425

<210> 22
<211> 444
<212> PRT
<213> Ochrobactrum/Brucella

<400> 22
Met Ala Cys Leu Pro Asp Asp Ser Gly Pro His Val Gly His Ser Thr
1               5                   10                  15
Pro Pro Cys Leu Asp Gln Glu Pro Cys Thr Leu Ser Ser Gln Lys Thr
            20                  25                  30
Val Thr Val Thr Pro Pro Asn Phe Pro Leu Thr Gly Lys Val Ala Pro
        35                  40                  45
Pro Gly Ser Lys Ser Ile Thr Asn Arg Ala Leu Leu Leu Ala Ala Leu
    50                  55                  60
Ala Lys Gly Thr Ser Arg Leu Ser Gly Ala Leu Lys Ser Asp Asp Thr
65                  70                  75                  80
Arg His Met Ser Val Ala Leu Arg Gln Met Gly Val Thr Ile Asp Glu
                85                  90                  95
Pro Asp Asp Thr Thr Phe Val Val Thr Ser Gln Gly Ser Leu Gln Leu
            100                 105                 110
Pro Ala Gln Pro Leu Phe Leu Gly Asn Ala Gly Thr Ala Met Arg Phe
        115                 120                 125
Leu Thr Ala Ala Val Ala Thr Val Gln Gly Thr Val Val Leu Asp Gly
    130                 135                 140
Asp Glu Tyr Met Gln Lys Arg Pro Ile Gly Pro Leu Leu Ala Thr Leu
145                 150                 155                 160
Gly Gln Asn Gly Ile Gln Val Asp Ser Pro Thr Gly Cys Pro Pro Val
                165                 170                 175
Thr Val His Gly Ala Gly Lys Val Gln Ala Arg Arg Phe Glu Ile Asp
            180                 185                 190
Gly Gly Leu Ser Ser Gln Tyr Val Ser Ala Leu Leu Met Leu Ala Ala
        195                 200                 205
Cys Gly Glu Ala Pro Ile Glu Val Ala Leu Thr Gly Lys Asp Ile Gly
    210                 215                 220
Ala Arg Gly Tyr Val Asp Leu Thr Leu Asp Cys Met Arg Ala Phe Gly
225                 230                 235                 240
Ala Gln Val Asp Ile Val Asp Asp Thr Thr Trp Arg Val Ala Pro Thr
                245                 250                 255
Gly Tyr Thr Ala His Asp Tyr Leu Ile Glu Pro Asp Ala Ser Ala Ala
            260                 265                 270
Thr Tyr Leu Trp Ala Ala Glu Val Leu Thr Gly Gly Arg Ile Asp Ile
        275                 280                 285
Gly Val Ala Ala Gln Asp Phe Thr Gln Pro Asp Ala Lys Ala Gln Ala
    290                 295                 300
Val Ile Ala Gln Phe Pro Asn Met Gln Ala Thr Val Val Gly Ser Gln

305                 310                 315                 320
Met Gln Asp Ala Ile Pro Thr Leu Ala Val Leu Ala Ala Phe Asn Asn
                325                 330                 335
Thr Pro Val Arg Phe Thr Glu Leu Ala Asn Leu Arg Val Lys Glu Cys
            340                 345                 350
Asp Arg Val Gln Ala Leu His Asp Gly Leu Asn Glu Ile Arg Pro Gly
        355                 360                 365
Leu Ala Thr Ile Glu Gly Asp Asp Leu Leu Val Ala Ser Asp Pro Ala
    370                 375                 380
Leu Ala Gly Thr Ala Cys Thr Ala Leu Ile Asp Thr His Ala Asp His
385                 390                 395                 400
Arg Ile Ala Met Cys Phe Ala Leu Ala Gly Leu Lys Val Ser Gly Ile
                405                 410                 415
Arg Ile Gln Asp Pro Asp Cys Val Ala Lys Thr Tyr Pro Asp Tyr Trp
            420                 425                 430
Lys Ala Leu Ala Ser Leu Gly Val His Leu Ser Tyr
        435                 440

<210> 23
<211> 425
<212> PRT
<213> Agrobacterium tumefaciens strain C58

<400> 23
Met Ile Glu Leu Thr Ile Thr Pro Pro Gly His Pro Leu Ser Gly Lys
 1               5                  10                  15
Val Glu Pro Pro Gly Ser Lys Ser Ile Thr Asn Arg Ala Leu Leu Leu
            20                  25                  30
Ala Gly Leu Ala Lys Gly Lys Ser Arg Leu Thr Gly Ala Leu Lys Ser
        35                  40                  45
Asp Asp Thr Leu Tyr Met Ala Glu Ala Leu Arg Glu Met Gly Val Lys
    50                  55                  60
Val Thr Glu Pro Asp Ala Thr Thr Phe Val Val Glu Ser Ser Gly Gly
65                  70                  75                  80
Leu His Gln Pro Glu Lys Pro Leu Phe Leu Gly Asn Ala Gly Thr Ala
                85                  90                  95
Thr Arg Phe Leu Thr Ala Ala Ala Ala Leu Val Asp Gly Ala Val Ile
            100                 105                 110
Ile Asp Gly Asp Glu His Met Arg Lys Arg Pro Ile Met Pro Leu Val
        115                 120                 125
Glu Ala Leu Arg Ser Leu Gly Val Glu Ala Glu Ala Pro Thr Gly Cys
    130                 135                 140
Pro Pro Val Thr Val Cys Gly Lys Gly Thr Gly Phe Pro Lys Gly Ser
145                 150                 155                 160
Val Thr Ile Asp Ala Asn Leu Ser Ser Gln Tyr Val Ser Ala Leu Leu
                165                 170                 175
Met Ala Ala Ala Cys Gly Asp Lys Pro Val Asp Ile Ile Leu Lys Gly
            180                 185                 190
Glu Glu Ile Gly Ala Lys Gly Tyr Ile Asp Leu Thr Thr Ser Ala Met
        195                 200                 205
Glu Ala Phe Gly Ala Lys Val Glu Arg Val Ser Asn Ala Ile Trp Arg
    210                 215                 220
Val His Pro Thr Gly Tyr Thr Ala Thr Asp Phe His Ile Glu Pro Asp
225                 230                 235                 240
Ala Ser Ala Ala Thr Tyr Leu Trp Gly Ala Glu Leu Leu Thr Gly Gly
                245                 250                 255
Ala Ile Asp Ile Gly Thr Pro Ala Asp Lys Phe Thr Gln Pro Asp Ala
            260                 265                 270
Lys Ala His Glu Val Met Ala Gln Phe Pro His Leu Pro Ala Glu Ile
        275                 280                 285

Asp Gly Ser Gln Met Gln Asp Ala Ile Pro Thr Ile Ala Val Leu Ala
    290                 295                 300
Ala Phe Asn Glu Thr Pro Val Arg Phe Val Gly Ile Ala Asn Leu Arg
305                 310                 315                 320
Val Lys Glu Cys Asp Arg Ile Arg Ala Val Ser Leu Gly Leu Asn Glu
                325                 330                 335
Ile Arg Asp Gly Leu Ala His Glu Glu Gly Asp Asp Leu Ile Val His
            340                 345                 350
Ser Asp Pro Ser Leu Ala Gly Gln Thr Val Asn Ala Ser Ile Asp Thr
        355                 360                 365
Phe Ala Asp His Arg Ile Ala Met Ser Phe Ala Leu Ala Ala Leu Lys
    370                 375                 380
Ile Gly Gly Ile Ala Ile Gln Asn Pro Ala Cys Val Gly Lys Thr Tyr
385                 390                 395                 400
Pro Gly Tyr Trp Lys Ala Leu Ala Ser Leu Gly Val Glu Tyr Ser Glu
                405                 410                 415
Lys Glu Thr Ala Ala Glu Pro Gln His
            420                 425

<210> 24
<211> 414
<212> PRT
<213> Sulfolobus solfataricus

<400> 24
Met Ile Val Lys Ile Tyr Pro Ser Lys Ile Ser Gly Ile Ile Lys Ala
 1               5                  10                  15
Pro Gln Ser Lys Ser Leu Ala Ile Arg Leu Ile Phe Leu Ser Leu Phe
            20                  25                  30
Thr Arg Val Tyr Leu His Asn Leu Val Leu Ser Glu Asp Val Ile Asp
        35                  40                  45
Ala Ile Lys Ser Val Arg Ala Leu Gly Val Lys Val Lys Asn Asn Ser
    50                  55                  60
Glu Phe Ile Pro Pro Glu Lys Leu Glu Ile Lys Glu Arg Phe Ile Lys
65                  70                  75                  80
Leu Lys Gly Ser Ala Thr Thr Leu Arg Met Leu Ile Pro Ile Leu Ala
                85                  90                  95
Ala Ile Gly Gly Glu Val Thr Ile Asp Ala Asp Glu Ser Leu Arg Arg
            100                 105                 110
Arg Pro Leu Asn Arg Ile Val Gln Ala Leu Ser Asn Tyr Gly Ile Ser
        115                 120                 125
Phe Ser Ser Tyr Ser Leu Pro Leu Thr Ile Thr Gly Lys Leu Ser Ser
    130                 135                 140
Asn Glu Ile Lys Ile Ser Gly Asp Glu Ser Ser Gln Tyr Ile Ser Gly
145                 150                 155                 160
Leu Ile Tyr Ala Leu His Ile Leu Asn Gly Gly Ser Ile Glu Ile Leu
                165                 170                 175
Pro Pro Ile Ser Ser Lys Ser Tyr Ile Leu Leu Thr Ile Asp Leu Phe
            180                 185                 190
Lys Arg Phe Gly Ser Asp Val Lys Phe Tyr Gly Ser Lys Ile His Val
        195                 200                 205
Asn Pro Asn Asn Leu Val Glu Phe Gln Gly Glu Val Ala Gly Asp Tyr
    210                 215                 220
Gly Leu Ala Ser Phe Tyr Ala Leu Ser Ala Leu Val Ser Gly Gly Gly
225                 230                 235                 240
Ile Thr Ile Thr Asn Leu Trp Glu Pro Lys Glu Tyr Phe Gly Asp His
                245                 250                 255
Ser Ile Val Lys Ile Phe Ser Glu Met Gly Ala Ser Ser Glu Tyr Lys
            260                 265                 270
Asp Gly Arg Trp Phe Val Lys Ala Lys Asp Lys Tyr Ser Pro Ile Lys

            275                 280                 285
Ile Asp Ile Asp Asp Ala Pro Asp Leu Ala Met Thr Ile Ala Gly Leu
        290                 295                 300
Ser Ala Ile Ala Glu Gly Thr Ser Glu Ile Ile Gly Ile Glu Arg Leu
305                 310                 315                 320
Arg Ile Lys Glu Ser Asp Arg Ile Glu Ser Ile Arg Lys Ile Leu Gly
                325                 330                 335
Leu Tyr Gly Val Gly Ser Glu Val Lys Tyr Asn Ser Ile Leu Ile Phe
            340                 345                 350
Gly Ile Asn Lys Gly Met Leu Asn Ser Pro Val Thr Asp Cys Leu Asn
        355                 360                 365
Asp His Arg Val Ala Met Met Ser Ser Ala Leu Ala Leu Val Asn Gly
    370                 375                 380
Gly Val Ile Thr Ser Ala Glu Cys Val Gly Lys Ser Asn Pro Asn Tyr
385                 390                 395                 400
Trp Gln Asp Leu Leu Ser Leu Asn Ala Lys Ile Ser Ile Glu
                405                 410

<210> 25
<211> 424
<212> PRT
<213> Fusobacterium nucleatum

<400> 25
Met Arg Asn Met Asn Lys Lys Ile Ile Lys Ala Asp Lys Leu Val Gly
1               5                   10                  15
Glu Val Thr Pro Pro Pro Ser Lys Ser Val Leu His Arg Tyr Ile Ile
            20                  25                  30
Ala Ser Ser Leu Ala Lys Gly Ile Ser Lys Ile Glu Asn Ile Ser Tyr
        35                  40                  45
Ser Asp Asp Ile Ile Ala Thr Ile Glu Ala Met Lys Lys Leu Gly Ala
    50                  55                  60
Asn Ile Glu Lys Lys Asp Asn Tyr Leu Leu Ile Asp Gly Ser Lys Thr
65                  70                  75                  80
Phe Asp Lys Glu Tyr Leu Asn Asn Asp Ser Glu Ile Asp Cys Asn Glu
                85                  90                  95
Ser Gly Ser Thr Leu Arg Phe Leu Phe Pro Leu Ser Ile Val Lys Glu
            100                 105                 110
Asn Lys Ile Leu Phe Lys Gly Lys Gly Lys Leu Phe Lys Arg Pro Leu
        115                 120                 125
Ser Pro Tyr Phe Glu Asn Phe Asp Lys Tyr Gln Ile Lys Cys Ser Ser
    130                 135                 140
Ile Asn Glu Asn Lys Ile Leu Leu Asp Gly Glu Leu Lys Ser Gly Val
145                 150                 155                 160
Tyr Glu Ile Asp Gly Asn Ile Ser Ser Gln Phe Ile Thr Gly Leu Leu
                165                 170                 175
Phe Ser Leu Pro Leu Leu Asn Gly Asn Ser Lys Ile Ile Ile Lys Gly
            180                 185                 190
Lys Leu Glu Ser Ser Ser Tyr Ile Asp Ile Thr Leu Asp Cys Leu Asn
        195                 200                 205
Lys Phe Gly Ile Asn Ile Ile Asn Asn Ser Tyr Lys Glu Phe Ile Ile
    210                 215                 220
Glu Gly Asn Gln Thr Tyr Lys Ser Gly Asn Tyr Gln Val Glu Ala Asp
225                 230                 235                 240
Tyr Ser Gln Val Ala Phe Phe Leu Val Ala Asn Ser Ile Gly Ser Asn
                245                 250                 255
Ile Lys Ile Asn Gly Leu Asn Val Asn Ser Leu Gln Gly Asp Lys Lys
            260                 265                 270
Ile Ile Asp Phe Ile Ser Glu Ile Asp Asn Trp Thr Lys Asn Glu Lys
        275                 280                 285

Leu Ile Leu Asp Gly Ser Glu Thr Pro Asp Ile Ile Pro Ile Leu Ser
    290                 295                 300
Leu Lys Ala Cys Ile Ser Lys Lys Glu Ile Glu Ile Val Asn Ile Ala
305                 310                 315                 320
Arg Leu Arg Ile Lys Glu Ser Asp Arg Leu Ser Ala Thr Val Gln Glu
                325                 330                 335
Leu Ser Lys Leu Gly Phe Asp Leu Ile Glu Lys Glu Asp Ser Ile Leu
            340                 345                 350
Ile Asn Ser Arg Lys Asn Phe Asn Glu Ile Ser Asn Asn Ser Pro Ile
        355                 360                 365
Ser Leu Ser Ser His Ser Asp His Arg Ile Ala Met Thr Val Ala Ile
    370                 375                 380
Ala Ser Thr Cys Tyr Glu Gly Glu Ile Ile Leu Asp Asn Leu Asp Cys
385                 390                 395                 400
Val Lys Lys Ser Tyr Pro Asn Phe Trp Glu Val Phe Leu Ser Leu Gly
                405                 410                 415
Gly Lys Ile Tyr Glu Tyr Leu Gly
            420

<210> 26
<211> 428
<212> PRT
<213> Methanopyrus kandleri

<400> 26
Met Lys Arg Val Glu Leu Glu Gly Ile Pro Glu Val Arg Gly Thr Val
 1               5                  10                  15
Cys Pro Pro Pro Ser Lys Ser Gly Ser His Arg Ala Leu Ile Ala Ala
            20                  25                  30
Ser Leu Cys Asp Gly Ser Thr Glu Leu Trp Asn Val Leu Asp Ala Glu
        35                  40                  45
Asp Val Arg Ala Thr Leu Arg Leu Cys Arg Met Leu Gly Ala Glu Val
    50                  55                  60
Asp Val Asp Gly Glu Glu Arg Leu Glu Ala Thr Val Ser Gly Phe Gly
65                  70                  75                  80
Asp Ser Pro Arg Ala Pro Glu Asp Val Val Asp Cys Gly Asn Ser Gly
                85                  90                  95
Thr Thr Leu Arg Leu Gly Cys Gly Leu Ala Ala Leu Val Glu Gly Thr
            100                 105                 110
Thr Ile Leu Thr Gly Asp Asp Ser Leu Arg Ser Arg Pro Val Gly Asp
        115                 120                 125
Leu Leu Ala Ala Leu Arg Ser Leu Gly Val Asp Ala Arg Gly Arg Val
    130                 135                 140
Val Arg Gly Glu Glu Tyr Pro Pro Val Val Ile Ser Gly Arg Pro Leu
145                 150                 155                 160
Arg Glu Arg Val Ala Val Tyr Gly Asp Val Ser Ser Gln Phe Val Ser
                165                 170                 175
Ala Leu Leu Phe Leu Gly Ala Gly Leu Gly Ala Leu Arg Val Asp Val
            180                 185                 190
Val Gly Asp Leu Arg Ser Arg Pro Tyr Val Asp Met Thr Val Glu Thr
        195                 200                 205
Leu Glu Arg Phe Gly Val Ser Val Val Arg Glu Gly Ser Ser Phe Glu
    210                 215                 220
Val Glu Gly Arg Pro Arg Ser Pro Gly Lys Leu Arg Val Glu Asn Asp
225                 230                 235                 240
Trp Ser Ser Ala Gly Tyr Phe Val Ala Leu Gly Ala Ile Gly Gly Glu
                245                 250                 255
Met Arg Ile Glu Gly Val Asp Leu Asp Ser Ser His Pro Asp Arg Arg
            260                 265                 270
Ile Val Glu Ile Thr Arg Glu Met Gly Ala Glu Val Arg Arg Ile Asp

275                 280                 285
Gly Gly Ile Val Val Arg Ser Thr Gly Arg Leu Glu Gly Val Glu Val
    290                 295                 300
Asp Leu Ser Asp Ser Pro Asp Leu Val Pro Thr Val Ala Ala Met Ala
305                 310                 315                 320
Cys Phe Ala Glu Gly Val Thr Arg Ile Glu Asn Val Gly His Leu Arg
                325                 330                 335
Tyr Lys Glu Val Asp Arg Leu Arg Ala Leu Ala Ala Glu Leu Pro Lys
            340                 345                 350
Phe Gly Val Glu Val Arg Glu Gly Lys Asp Trp Leu Glu Ile Val Gly
        355                 360                 365
Gly Glu Pro Val Gly Ala Arg Val Asp Ser Arg Gly Asp His Arg Met
    370                 375                 380
Ala Met Ala Leu Ala Val Val Gly Ala Phe Ala Arg Gly Lys Thr Val
385                 390                 395                 400
Val Glu Arg Ala Asp Ala Val Ser Ile Ser Tyr Pro Arg Phe Trp Glu
                405                 410                 415
Asp Leu Ala Ser Val Gly Val Pro Val His Ser Val
            420                 425

<210> 27
<211> 436
<212> PRT
<213> Arthrobacter globiformis

<400> 27
Met Ala Leu Glu Arg Gly Gln His Gly Arg Ser Arg Arg Leu Phe Gly
 1               5                  10                  15
Ala Ser Leu Glu Arg Ile Thr Met Glu Thr Asp Arg Leu Val Ile Pro
            20                  25                  30
Gly Ser Lys Ser Ile Thr Asn Arg Ala Leu Leu Leu Ala Ala Ala Ala
        35                  40                  45
Lys Gly Thr Ser Val Leu Val Arg Pro Leu Val Ser Ala Asp Thr Ser
    50                  55                  60
Ala Phe Lys Thr Ala Ile Gln Ala Leu Gly Ala Asn Val Ser Ala Asp
65                  70                  75                  80
Gly Asp Asn Trp Val Val Glu Gly Leu Gly Gln Ala Pro His Leu Asp
                85                  90                  95
Ala Asp Ile Trp Cys Glu Asp Ala Gly Thr Val Ala Arg Phe Leu Pro
            100                 105                 110
Pro Phe Val Ala Ala Gly Gln Gly Lys Phe Thr Val Asp Gly Ser Glu
        115                 120                 125
Gln Leu Arg Arg Arg Pro Leu Arg Pro Leu Val Asp Gly Ile Arg His
    130                 135                 140
Leu Gly Ala Arg Val Ser Ser Glu Gln Leu Pro Leu Thr Ile Glu Ala
145                 150                 155                 160
Ser Gly Leu Ala Gly Gly Glu Tyr Glu Ile Glu Ala His Gln Ser Ser
                165                 170                 175
Gln Phe Ala Ser Gly Leu Ile Met Ala Ala Pro Tyr Ala Arg Gln Gly
            180                 185                 190
Leu Arg Val Arg Ile Pro Asn Pro Val Ser Gln Pro Tyr Leu Thr Met
        195                 200                 205
Thr Leu Arg Met Met Arg Asp Phe Gly Leu Glu Thr Ser Thr Asp Gly
    210                 215                 220
Ala Thr Val Ser Val Pro Pro Gly Arg Tyr Thr Ala Arg Arg Tyr Glu
225                 230                 235                 240
Ile Glu Pro Asp Ala Ser Thr Ala Ser Tyr Phe Ala Ala Ala Ser Ala
                245                 250                 255
Val Ser Gly Arg Ser Phe Glu Phe Gln Gly Leu Gly Thr Asp Ser Ile
            260                 265                 270

Gln Gly Asp Thr Ser Phe Phe Asn Val Leu Gly Arg Leu Gly Ala Glu
        275                 280                 285
Val His Trp Ala Pro Asn Ser Val Thr Ile Ser Gly Pro Glu Arg Leu
    290                 295                 300
Asn Gly Asp Ile Glu Val Asp Met Gly Glu Ile Ser Asp Thr Phe Met
305                 310                 315                 320
Thr Leu Ala Ala Ile Ala Pro Leu Ala Asp Gly Pro Ile Thr Ile Thr
                325                 330                 335
Asn Ile Gly His Ala Arg Leu Lys Glu Ser Asp Arg Ile Ser Ala Met
            340                 345                 350
Glu Thr Asn Leu Arg Thr Leu Gly Val Gln Thr Asp Val Gly His Asp
        355                 360                 365
Trp Met Arg Ile Tyr Pro Ser Thr Pro His Gly Gly Arg Val Asn Cys
    370                 375                 380
His Arg Asp His Arg Ile Ala Met Ala Phe Ser Ile Leu Gly Leu Arg
385                 390                 395                 400
Val Asp Gly Ile Thr Leu Asp Asp Pro Gln Cys Val Gly Lys Thr Phe
                405                 410                 415
Pro Gly Phe Phe Asp Tyr Leu Gly Arg Leu Phe Pro Glu Lys Ala Leu
            420                 425                 430
Thr Leu Pro Gly
        435

<210> 28
<211> 1251
<212> DNA
<213> Artificial Sequence

<220>
<223> synthetic sequence encoding GRG31 (syngrg31)

<400> 28
atgaaccagc aggtgatcac cttgacacat ccaagcaaga agatccaagg caccgtgcag 60
ctcaccggca gcaagagcga gagcaacagg gcgctcatca ttcagagctt gagcaagggc 120
caagtggaga tcgccaacct ctcagaagct gctgacaccg tcaccctcaa cagggtgctg 180
caaattgctt cagatccaaa gcctggcttc aacaccatcg acattgggcc ggccggcacc 240
gccatgaggt tcttgacggc ctacctcaac ctggtgaagg gcaacttcat cctcaccggc 300
actgaaagga tgcagcagcg gcccatcggc atcctggtgg acgccatgaa ggagatcggc 360
gccgacatcc actacgacaa gaaggtgggc tacccgccgc taaagattga aggaggcctc 420
ttccaagaga aggaccgcgt caagatcaag ggcaacatca gcagccagta catctcggcg 480
ctgctgctca tcgcgccggc gctgaagaag gggctgacgc tggagattga aggagagctg 540
acatcaaggc cttatgtgag catgacgctg gacatgctga agagcgtcgg catccagcat 600
gaatggaaga acaacgccat caagattgct cctcaagcat ttgagaagca gaccatctat 660
gtggagccag attggagcgc cgcctcatat tggtacgcca tcgccgcgct ggctgatgca 720
aatgcaagca tcgtgctgcc agggctgagg aagaacagcc tccaaggaga catcgccatc 780
atcagcatca tggagcattt tggagttcaa agttcatttg agagcgacgg cctccacctc 840
aacaagaagg tgattggaag tgatgtgagc ctcttcaact tcaaggagtg cccagatctt 900
gctcaaacag tggtggtggt ggcggcggcg ctgaagagag atgtgagctt caccggcctg 960
gagacgctga agatcaagga gaccgacagg atcgccgcgc tacagaagga gatcgccaag 1020
ttcggcgccg agctgattga agatggagac acctaccacc tcaagacggc gcaggtgtac 1080
cagccagaag aagtcacctt cgacacctat gaagatcaca ggatggcaat ggccttcgcg 1140
ccgctagctc tggtgttcga ccagatcaag attgctgaac ctcaagtggt ggagaagagc 1200
tacccggact tctggaacca cctccaagct caagccttcg tcatcgagta g          1251

<210> 29
<211> 1401
<212> DNA
<213> Artificial Sequence

<220>

<223> synthetic sequence encoding GRG25 (syngrg25)

<400> 29
atgctgctga ggacggtgaa gacctgcttg gagcccaagg acctgcccat ctgcaccgcc 60
tcggcgccgg gctcgccgac ggacaaactt cacaatgctg agaagacatg gtgggcggcg 120
ccgcacgccg ccaccggcct ggacgccatc gtctccgtgc cggcatcaaa gagcctcacc 180
aacagatatt tgatcctggc ggcgctggca agctcgccaa gcaccatcca caacaccttg 240
atctcaaggg acaccgagct gatgctggac gcgctggcgg ccttcggcat cggcatcgag 300
aggacaactc aacctgatgg aagcaccacc gtcgccatca cgccgggcaa gctggccacc 360
gggccgctga gcatcgactg cggcctcgcc ggcaccgtga tgaggttcgt gccgccgctc 420
gccgccgtcg ccggcgcctc agtggcattt gatggagatg aagcagcaag ggtgcggcca 480
atggcgccgg tgctggacgc gctggagacg ctgggagcaa ggattgagta cgccggcacg 540
ccgggcatgc tgcccttcac aatggatgct tctgctcttc aagaaaggca tgaggtgctg 600
attgatgctt ctggaagctc tcagttcatc tcggcgctgc tgctggtggg ccaagctcta 660
cctggaggcc tcaagctccg cgccgccgcc ggccacatcg cctcgcctga tcacatcgcc 720
atgacagttc aaacattgag ggagctcggc gtggaggtgg cggtgggaga agatgcaaga 780
agctggagca tcgcgccggg gcagctctca ggcttcacca tcaccgtgga gcctgatttg 840
agcaacgccg gccccttcct ggcggcggcg ctggcaacaa atggcaccgt ccgcgtgccc 900
ttctggccgg caagcaccac tcaagttgga ggaaaatggg tgcaaatcct ctcaaggatg 960
ggcgccgaga tctctcatgg agaagatggc gtgctgaccg tccgcggcac cggcgtcatc 1020
cgcggcatcg actatgctga tgcttcagag ctggcgccga cgctggcggc gctctgcacc 1080
ctcgccgact cgccaagcaa gctcaccggc atcggccacc tccgcggcca tgaaacagat 1140
cggctggcgg cgctggagac agagctggcc aaggtgggcg ccaccgtgac aagcaccgac 1200
gacgcgctgg agatcatccc tggaactctt caagctgctg atctggattc atatgaagat 1260
cacaggatgg ccaccgccgg cgcgctgctg ggctggccca ttgaaggagt gagggtggag 1320
aacatcgcca ccaccgccaa gaccatgccg gacttcccgc agctgtggac ggacatggcg 1380
gcgacggcca agggccaagg a 1401

<210> 30
<211> 1392
<212> DNA
<213> Artificial Sequence

<220>
<223> synthetic sequence encoding GRG26 (syngrg26)

<400> 30
   atgcctggga cgccggcgac gcagacagat gattctggaa caagcaccgc ctcggcgctg 60
   ccgctgtggc cggcgccctt cgccagccat cctgttgatg ccaccgtcac cgtgcctgga 120
   agcaagagcc tcaccaacag atatttggtg ctggcggcgc tggcaaatgg gccatcaagg 180
   ctccgcgcgc cgcttcattc aagagattcg gcgctgatgg tggaggcgct ccgccgcctc 240
   ggcgccacca tcactgaagt tcctggagat ggtcaatatg gaccagatct ggaggtgacg 300
   cccatcgacc cggcggcggc ggcgtcggag acggccatcg actgcggcct cgccggcacc 360
   gtgatgaggt tcgtgccgcc gctggcggcg ctgaggaatg gagcttcagt ttttgatgga 420
   gatcctcatg caaggcagcg gccaatgggc accatcatcg aggcgctgag gacgctcggc 480
   gtggatgttt cagcaatgga tggcaaggcg ccgggcagct tgcccttcaa ggtgagcggc 540
   accggcagcg tccgcggcgg ccacctggtg attgatgctt cagcaagctc tcaatttgtg 600
   tcggcgctgc tgctggtggg agcaagattt gaagaaggcc tccacctgga gcatgttggc 660
   aagccggtgc caagcctgga ccacatcaac atgacggtgg cggtgctgcg cggcgtcggc 720
   gtgcaagttg atgattcagt tccaaatcat tggagggtga gccccggcgc catccaagca 780
   tttgatgaaa gaattgagca agatctttca aatgctggac ccttcctggc ggcggcgctg 840
   gccaccaagg gcaccgtcag gattccaaat tggccggcaa gcaccactca agttggagat 900
   ctttggagga acatcctggc aacaatgggc gccaccgtga cgctggacaa tggcaccctc 960
   accgtcaccg gcggcagcga gatcctcggc gccgactttg atgaaacttc agagctggcg 1020
   ccgacggtgg cggcgctctg cgcgctagca acctcgccat caaggctcac cggcattgct 1080
   cacctccgcg gccatgaaac agatcggctg gcggcgctgg tggcggagat caacaggctc 1140
   ggcggcgacg ccgaggagac aagtgatggg ctggtgatca ggccggcggc gctgcactca 1200
   ggagtggtgc acagctatgc tgatcacagg atggccaccg ccggcgccat cctcggcctc 1260
   gccgtggaag gagtgagggt ggaggacatc gccaccacca gcaagaccat gccggagttc 1320
   ccgcagatct ggacaagcat gctctccgcc gggccggcgg cggcggcggc cggcagcacc 1380
   gagacaagca ac 1392

<210> 31
<211> 1365
<212> DNA
<213> Artificial Sequence

<220>
<223> synthetic sequence encoding GRG27 (syngrg27)

<400> 31
atgaccggca ccaccgccgc caacaccgag ccggacaagg cggcgtcgct gccgctgtgg 60
ccggcgccat atgcaaatgg gccggtggac gccaccgtca ccgtgcctgg aagcaagagc 120
ctcaccaaca ggttcctggt gctggcggcg ctggctgatg ggccatcaag gctccgcgcg 180
cctcttcatt caagagattc ggcgctgatg atccaagctc ttcggcagct cggcgccacc 240
gtcaccgagg tgccaggaga tggagattat ggacctgatc tggagatcac gccgctggac 300
ccaagcgccg ccggcagcag caccgccatc gactgcggcc tcgccggcac cgtgatgagg 360
ttcgtgccgc cgctggcggc gctgagatca ggcgccaccg tctttgatgg agatcctcat 420
gcaaggaaca ggccaatggg caccatcatc gaggcgctgc tggcgctggg agttcctgtt 480
gctgctgaag gaggaaggac gccgtcggcg ctgcccttca ccgtggaagg caccggcgag 540
gtgcgcggcg gccacctggt gattgatgct tcagcaagct ctcaatttgt gtcggcgctg 600
ctgctggtgg gcgcgcgctt cacagaaggc ctccacctgg agcatgttgg caagccggtg 660
ccaagcctgg atcacatcac catgacggtg gaggtgctga ggagcgtcgg cgtcaccgtg 720
gatgattcag ttcccaacca ctggagggtg tcgccgggca agatcaccgc cttcgaccag 780
aggatcgagc aagatctttc aaatgctgga cccttcctgg cggcggcgct ggcaactcat 840
ggcaccgtga ggattccaaa ttggcccatc ggcaccactc aagttggtga tctttggagg 900
accatcctgg cggcaatggg cgccaccgtc accttggacg gcggcaccct caccgtcacc 960
ggcggcaatg agatcaaagg agctgatttt gatgaaactt cagagctggc gccgacggtg 1020
gcggcgctct gcgcgctggc caccgggcca tcaaggctga ccggcattgc tcacctccgc 1080
ggccatgaaa cagatcggct ggcggcgctg gtggcggaga tcaacaggct gggaggagat 1140
gctgaagaaa ctgctgatgg gctgctgatc aggccggcga cgctacatgg cggcgtcatc 1200
cacagctacg ccgaccacag gatggccacc gccggcgcca tcctcggcct cgccgtcgac 1260
ggcgtccagg tggaggacat cgccaccacc agcaagacca tgccggagtt cccggagatg 1320
tgggcaagga tgctggacag cgccggcacc aacgaggccg gcaac 1365

<210> 32
<211> 1383
<212> DNA
<213> Artificial Sequence

<220>
<223> synthetic sequence encoding GRG28 (syngrg28)

<400> 32
atgaccggca ccaccgccgc caacaccggc agcaacagcg tggacacctt gccgctctgg 60
gcggcgccat atgcaacaag gccggtggac gccaccgtca ccgtgcctgg aagcaagagc 120
ctcaccaaca ggttcctggt gctggcggcg ctggctgatg ggccaagccg cctccgcgcg 180
cctcttcatt caagagattc tgtgctgatg atccaggcgc tgcggcagct cggcgccacc 240
gtcaccgagg tgcccggcga cggcgactat ggacctgatc tggagatcac gccgatggac 300
ccttcagcaa gcggcgccga gacggccatc gactgcggcc tcgccggcac cgtgatgagg 360
ttcgtgccgc cgctggcggc gctgaggaat gggccaacaa catttgatgg agatcctcat 420
gcaaggaaga ggccaatggg caccatcatc gaggcgctgc tggcgctagg agttcctgtg 480
gcaacagaag gaggaaggac gccgtcggcg ctgcccttct ccgtggatgg caccggcgag 540
gtgcgcggcg gccacctggt gattgatgct tcagcaagct ctcaatttgt gtcggcgctg 600
ctgctggtgg gagcaaggtt cacagaaggc ctccacctgg agcatgttgg caagccggtg 660
ccaagcctgg atcacatcaa catgaccgtc tccgtgctgc gcggcgtcgg cgtcaaggtg 720
gatgattcag ttcccaacca ctggagggtg gcgccgggcc ccatccatgc cttcgaccag 780
aggatcgagc aagatctttc aaatgctgga cccttcctgg cggcggcgct ggcaacaaga 840
ggaacagtga ggattccaaa ttggccgacg cagacaactc aagttggtga tctatggagg 900
agcatcctgg tggagatggg cgccaccgtc accttggaga atggcaccct caccgtcaaa 960
ggaggaccag agatcaaagg agctgatttt gatgagacct ccgagctggc gccgacggtg 1020
gcggcgctct gcgcgctggc caccgggcca tcaaggctga ccggcattgc tcacctccgc 1080

ggccatgaaa cagatcggct ggcggcgctg gcggcggaga tcaacaggct gggaggagat 1140
gctgaagaaa ctgctgatgg cctcatcatc aggccggcga cgctgcatcc tggagtagtt 1200
cattcatatg ctgatcacag gatggccacc gccggcgcca tcctcggcct cgccgtggaa 1260
ggagttcaag tggaggacat cgccaccacc agcaagacgg tgccagaatt tcctcaaatg 1320
tgggaggcca tgctgcaaca aggaactgga gaagatgaga ccgtcacctc agaagcaagc 1380
aac 1383

<210> 33
<211> 1293
<212> DNA
<213> Artificial Sequence

<220>
<223> synthetic sequence encoding GRG29 (syngrg29)

<400> 33
atggatgtca tcgtcaagcc gacgccaagc ctcaatggag agatcggcgc gctgagcagc 60
aagaactaca caacaagata tttgctggcg gcggcgctgg cagaaggaac aagcaccatc 120
cactaccctg ctcattcaga agattctgat gccatgagaa gatgcatctc cgacctcggc 180
gccgtgctgg aagaagatga cagcaagatc gtcatccaag gcttcggctc acatccaaga 240
gatgtgaggg agctcaatgt tggaaatgct ggcgccgtgc tgcgcttctt gatgggcgtg 300
acggcgctct gccctgatgt caccttcgtc aacacctacc ccgacagcct cggcaagagg 360
cctcatgatg acctcatcga cgcgctgggg cagctaggag tggaggtgca gcatgagcaa 420
ggaaggctgc ccatcaccat caaaggagga caagccaagg gcggccacat cagagtttct 480
ggaagcgtca gctctcagta cctctcggcg ctgctcttcg tgacgccgct gctggcggag 540
gacagcacca tcgaggtgct aaatgacctc aagagcaagg tggtgatcgg ccagacgctg 600
gaggtgctgg agcaagctgg catcgtcatc catgcttcag atgactacat gagcttccgc 660
gtgcccggcg gccaagccta caagccgcag acctacaccg tccaaggaga ttatcctgga 720
agcgccgccg tgctggccgc cgccgccgtc acccaaagtg atgtgaagat cctccggctg 780
atggagcaat caaagcaagg agaaagagca attgttgatg tgctgaggat gatggaggtg 840
ccgctcaccc atgaaaatga tgtggtgcat gttcaaggaa atggcacctt gaaggcggtg 900
gagtttgatg gagatgctgc aacagatgct gtgctggcaa tggtggcggc ggcggtgttt 960
gctgaaggaa catcaagatt ctacaatgtg gagaacttga gatacaagga atgtgacagg 1020
atcaccgact acctcaacga gctgaggaag gccggcgcca atgtggagga gaggcaagct 1080
gagatcattg ttcatggaag gccagaagga gtggagggcg gcgtggagat caatgctcac 1140
tacgaccacc gcgtcatcat ggcgctaaca gtggtggggc tgaggagcaa ggagccgctg 1200
aggatcagag atgctcatca tgtggccaag agctaccctc aatattttga tcatcttcaa 1260
gctctcggcg cctccgtgca gtgggtgaag gag 1293

<210> 34
<211> 1293
<212> DNA
<213> Artificial Sequence

<220>
<223> synthetic sequence encoding GRG30 (syngrg30)

<400> 34
atggatgtca tcgtcaagcc gacgccaagc ctcaatggag agatcggcgc gctgagcagc 60
aagaactaca caacaagata tttgctggcg gcggcgctgg cagaaggaac aagcaccatc 120
cactaccctg ctcattcaga agattctgat gccatgagaa gatgcatcag ggacctcggc 180
gccgtgctgg aagaagatga cagcaagatc gtcatccaag gcttcggctc acatcctcat 240
gatgtgaggg agctcaatgt tggaaatgct ggcgccgtgc tgcgcttctt gatgggcgtg 300
acggcgctct gcccggaggt gaccttcgtc aacacctacc ccgacagcct cggcaagagg 360
cctcatgatg acctcatcga cgcgctgggg cagctaggag tggaggtgca gcatgagcaa 420
ggaaggctgc ccatcaccat caaaggagga caagcaaaag gaggccacat cagagtttct 480
ggaagcgtca gctctcagta cctctcggcg ctgctcttcg tgacgccgct gctggcggag 540
gacagcacca tcgaggtgct aaatgacctc aagagcaagg tggtgatcgg ccagacgctg 600
gaggtgctgg agcaagctgg catcgtcatc catgcttcag atgactacat gagcttccgc 660
gtgcccggcg gccaagccta caagccgcag acctacaccg tccaaggaga ttatcctgga 720
agcgccgccg tgctggcggc ggcggcggtg acacaaagtg atgtgaagat cctccggctg 780

atggagcaat caaagcaagg agaaagagca attgttgatg tgctgaggat gatggaggtg 840
ccgctcaccc atgaaaatga tgtggtgcat gttcaaggaa atggcacctt gaaggcggtg 900
gagtttgatg gagatgctgc caccgacgcc gtgctggcaa tggtggcggc ggcggtgttt 960
gctgaaggaa catcaagatt ctacaatgtg gagaacttga gatacaagga atgtgacagg 1020
atcaccgact acctcaacga gctgaggaag gccggcgcca atgtggagga gaggcaagct 1080
gagatcattg ttcatggaag gccagaagga gtggagggcg gcgtggagat caatgctcac 1140
tacgaccacc gcgtcatcat ggcgctaaca gtggtggggc tgaggagcaa ggagccgctg 1200
aggatcagag atgctcatca tgtggccaag agctaccctc aatattttga tcatcttcaa 1260
gctctcggcg cctccgtgca gtgggtgaag gag 1293

<210> 35
<211> 431
<212> PRT
<213> Enterobacteriaciae

<400> 35
Met Lys Val Thr Ile Gln Pro Gly Asp Leu Thr Gly Ile Ile Gln Ser
1               5                   10                  15
Pro Ala Ser Lys Ser Ser Met Gln Arg Ala Cys Ala Ala Ala Leu Val
            20                  25                  30
Ala Lys Gly Ile Ser Glu Ile Ile Asn Pro Gly His Ser Asn Asp Asp
        35                  40                  45
Lys Ala Ala Arg Asp Ile Val Ser Arg Leu Gly Ala Arg Leu Glu Asp
    50                  55                  60
Gln Pro Asp Gly Ser Leu Gln Ile Thr Ser Glu Gly Val Lys Pro Val
65                  70                  75                  80
Ala Pro Phe Ile Asp Cys Gly Glu Ser Gly Leu Ser Ile Arg Met Phe
                85                  90                  95
Thr Pro Ile Val Ala Leu Ser Lys Glu Glu Val Thr Ile Lys Gly Ser
            100                 105                 110
Gly Ser Leu Val Thr Arg Pro Met Asp Phe Phe Asp Glu Ile Leu Pro
        115                 120                 125
His Leu Gly Val Lys Val Lys Ser Asn Gln Gly Lys Leu Pro Leu Val
    130                 135                 140
Ile Gln Gly Pro Leu Lys Pro Ala Asp Val Thr Val Asp Gly Ser Leu
145                 150                 155                 160
Ser Ser Gln Phe Leu Thr Gly Leu Leu Leu Ala Tyr Ala Ala Ala Asp
                165                 170                 175
Ala Ser Asp Val Ala Ile Lys Val Thr Asn Leu Lys Ser Arg Pro Tyr
            180                 185                 190
Ile Asp Leu Thr Leu Asp Val Met Lys Arg Phe Gly Leu Lys Thr Pro
        195                 200                 205
Glu Asn Arg Asn Tyr Glu Glu Phe Tyr Phe Lys Ala Gly Asn Val Tyr
    210                 215                 220
Asp Glu Thr Lys Met Gln Arg Tyr Thr Val Glu Gly Asp Trp Ser Gly
225                 230                 235                 240
Gly Ala Phe Leu Leu Val Ala Gly Ala Ile Ala Gly Pro Ile Thr Val
                245                 250                 255
Arg Gly Leu Asp Ile Ala Ser Thr Gln Ala Asp Lys Ala Ile Val Gln
            260                 265                 270
Ala Leu Met Ser Ala Asn Ala Gly Ile Ala Ile Asp Ala Lys Glu Ile
        275                 280                 285
Lys Leu His Pro Ala Asp Leu Asn Ala Phe Glu Phe Asp Ala Thr Asp
    290                 295                 300
Cys Pro Asp Leu Phe Pro Pro Leu Val Ala Leu Ala Ser Tyr Cys Lys
305                 310                 315                 320
Gly Glu Thr Lys Ile Lys Gly Val Ser Arg Leu Ala His Lys Glu Ser
                325                 330                 335
Asp Arg Gly Leu Thr Leu Gln Asp Glu Phe Gly Lys Met Gly Val Glu
            340                 345                 350
Ile His Leu Glu Gly Asp Leu Met Arg Val Ile Gly Gly Lys Gly Val

        355                 360                 365
Lys Gly Ala Glu Val Ser Ser Arg His Asp His Arg Ile Ala Met Ala
    370                 375                 380
Cys Ala Val Ala Ala Leu Lys Ala Val Gly Glu Thr Thr Ile Glu His
385                 390                 395                 400
Ala Glu Ala Val Asn Lys Ser Tyr Pro Asp Phe Tyr Ser Asp Leu Lys
                405                 410                 415
Gln Leu Gly Gly Val Val Ser Leu Asn His Gln Phe Asn Phe Ser
            420                 425                 430


<210> 36
<211> 414
<212> PRT
<213> Sulfolobus solfataricus

<400> 36
Met Ile Val Lys Ile Tyr Pro Ser Lys Ile Ser Gly Ile Ile Lys Ala
 1               5                  10                  15
Pro Gln Ser Lys Ser Leu Ala Ile Arg Leu Ile Phe Leu Ser Leu Phe
            20                  25                  30
Thr Arg Val Tyr Leu His Asn Leu Val Leu Ser Glu Asp Val Ile Asp
        35                  40                  45
Ala Ile Lys Ser Val Arg Ala Leu Gly Val Lys Val Lys Asn Asn Ser
    50                  55                  60
Glu Phe Ile Pro Pro Glu Lys Leu Glu Ile Lys Glu Arg Phe Ile Lys
65                  70                  75                  80
Leu Lys Gly Ser Ala Thr Thr Leu Arg Met Leu Ile Pro Ile Leu Ala
                85                  90                  95
Ala Ile Gly Gly Glu Val Thr Ile Asp Ala Asp Glu Ser Leu Arg Arg
            100                 105                 110
Arg Pro Leu Asn Arg Ile Val Gln Ala Leu Ser Asn Tyr Gly Ile Ser
        115                 120                 125
Phe Ser Ser Tyr Ser Leu Pro Leu Thr Ile Thr Gly Lys Leu Ser Ser
    130                 135                 140
Asn Glu Ile Lys Ile Ser Gly Asp Glu Ser Ser Gln Tyr Ile Ser Gly
145                 150                 155                 160
Leu Ile Tyr Ala Leu His Ile Leu Asn Gly Gly Ser Ile Glu Ile Leu
                165                 170                 175
Pro Pro Ile Ser Ser Lys Ser Tyr Ile Leu Leu Thr Ile Asp Leu Phe
            180                 185                 190
Lys Arg Phe Gly Ser Asp Val Lys Phe Tyr Gly Ser Lys Ile His Val
        195                 200                 205
Asn Pro Asn Asn Leu Val Glu Phe Gln Gly Glu Val Ala Gly Asp Tyr
    210                 215                 220
Gly Leu Ala Ser Phe Tyr Ala Leu Ser Ala Leu Val Ser Gly Gly Gly
225                 230                 235                 240
Ile Thr Ile Thr Asn Leu Trp Glu Pro Lys Glu Tyr Phe Gly Asp His
                245                 250                 255
Ser Ile Val Lys Ile Phe Ser Glu Met Gly Ala Ser Ser Glu Tyr Lys
            260                 265                 270
Asp Gly Arg Trp Phe Val Lys Ala Lys Asp Lys Tyr Ser Pro Ile Lys
        275                 280                 285
Ile Asp Ile Asp Asp Ala Pro Asp Leu Ala Met Thr Ile Ala Gly Leu
    290                 295                 300
Ser Ala Ile Ala Glu Gly Thr Ser Glu Ile Ile Gly Ile Glu Arg Leu
305                 310                 315                 320
Arg Ile Lys Glu Ser Asp Arg Ile Glu Ser Ile Arg Lys Ile Leu Gly
                325                 330                 335
Leu Tyr Gly Val Gly Ser Glu Val Lys Tyr Asn Ser Ile Leu Ile Phe
            340                 345                 350

Gly Ile Asn Lys Gly Met Leu Asn Ser Pro Val Thr Asp Cys Leu Asn
        355                 360                 365
Asp His Arg Val Ala Met Met Ser Ser Ala Leu Ala Leu Val Asn Gly
    370                 375                 380
Gly Val Ile Thr Ser Ala Glu Cys Val Gly Lys Ser Asn Pro Asn Tyr
385                 390                 395                 400
Trp Gln Asp Leu Leu Ser Leu Asn Ala Lys Ile Ser Ile Glu
                405                 410

<210> 37
<211> 409
<212> PRT
<213> Pseudomonas putida

<400> 37
Met Gln Arg Ala Cys Ala Ala Ala Leu Val Ala Lys Gly Ile Ser Glu
 1               5                  10                  15
Ile Ile Asn Pro Gly His Ser Asn Asp Asp Lys Ala Ala Arg Asp Ile
            20                  25                  30
Val Ser Arg Leu Gly Ala Arg Leu Glu Asp Gln Pro Asp Gly Ser Leu
        35                  40                  45
Gln Ile Thr Ser Glu Gly Val Lys Pro Val Ala Pro Phe Ile Asp Cys
    50                  55                  60
Gly Glu Ser Gly Leu Ser Ile Arg Met Phe Thr Pro Ile Val Ala Leu
65                  70                  75                  80
Ser Lys Glu Glu Val Thr Ile Lys Gly Ser Gly Ser Leu Val Thr Arg
                85                  90                  95
Pro Met Asp Phe Phe Asp Glu Ile Leu Pro His Leu Gly Val Lys Val
            100                 105                 110
Lys Ser Asn Gln Gly Lys Leu Pro Leu Val Ile Gln Gly Pro Leu Lys
        115                 120                 125
Pro Ala Asp Val Thr Val Asp Gly Ser Leu Ser Ser Gln Phe Leu Thr
    130                 135                 140
Gly Leu Leu Leu Ala Tyr Ala Ala Ala Asp Ala Ser Asp Val Ala Ile
145                 150                 155                 160
Lys Val Thr Asn Leu Lys Ser Arg Pro Tyr Ile Asp Leu Thr Leu Asp
                165                 170                 175
Val Met Lys Arg Phe Gly Leu Lys Thr Pro Glu Asn Arg Asn Tyr Glu
            180                 185                 190
Glu Phe Tyr Phe Lys Ala Gly Asn Val Tyr Asp Glu Thr Lys Met Gln
        195                 200                 205
Arg Tyr Thr Val Glu Gly Asp Trp Ser Gly Gly Ala Phe Leu Leu Val
    210                 215                 220
Ala Gly Ala Ile Ala Gly Pro Ile Thr Val Arg Gly Leu Asp Ile Ala
225                 230                 235                 240
Ser Thr Gln Ala Asp Lys Ala Ile Val Gln Ala Leu Met Ser Ala Asn
                245                 250                 255
Ala Gly Ile Ala Ile Asp Ala Lys Glu Ile Lys Leu His Pro Ala Asp
            260                 265                 270
Leu Asn Ala Phe Glu Phe Asp Ala Thr Asp Cys Pro Asp Leu Phe Pro
        275                 280                 285
Pro Leu Val Ala Leu Ala Ser Tyr Cys Lys Gly Glu Thr Lys Ile Lys
    290                 295                 300
Gly Val Ser Arg Leu Ala His Lys Glu Ser Asp Arg Gly Leu Thr Leu
305                 310                 315                 320
Gln Asp Glu Phe Gly Lys Met Gly Val Glu Ile His Leu Glu Gly Asp
                325                 330                 335
Leu Met Arg Val Ile Gly Gly Lys Gly Val Lys Gly Ala Glu Val Ser
            340                 345                 350
Ser Arg His Asp His Arg Ile Ala Met Ala Cys Ala Val Ala Ala Leu

355                 360                 365
Lys Ala Val Gly Glu Thr Thr Ile Glu His Ala Glu Ala Val Asn Lys
    370                 375                 380
Ser Tyr Pro Asp Phe Tyr Ser Asp Leu Lys Gln Leu Gly Gly Val Val
385                 390                 395                 400
Ser Leu Asn His Gln Phe Asn Phe Ser
                405



<210> 38
<211> 419
<212> PRT
<213> Brevundomonas vesicularis

<400> 38
   Met Met Met Gly Arg Ala Lys Leu Thr Ile Ile Pro Pro Gly Lys Pro
    1               5                  10                  15
   Leu Thr Gly Arg Ala Met Pro Pro Gly Ser Lys Ser Ile Thr Asn Arg
               20                  25                  30
   Ala Leu Leu Leu Ala Gly Leu Ala Lys Gly Thr Ser Arg Leu Thr Gly
           35                  40                  45
   Ala Leu Lys Ser Asp Asp Thr Arg Tyr Met Ala Glu Ala Leu Arg Ala
       50                  55                  60
   Met Gly Val Thr Ile Asp Glu Pro Asp Asp Thr Thr Phe Ile Val Lys
   65                  70                  75                  80
   Gly Ser Gly Lys Leu Gln Pro Pro Ala Ala Pro Leu Phe Leu Gly Asn
                   85                  90                  95
   Ala Gly Thr Ala Thr Arg Phe Leu Thr Ala Ala Ala Ala Leu Val Asp
               100                 105                 110
   Gly Lys Val Ile Val Asp Gly Asp Ala His Met Arg Lys Arg Pro Ile
           115                 120                 125
   Gly Pro Leu Val Asp Ala Leu Arg Ser Leu Gly Ile Asp Ala Ser Ala
       130                 135                 140
   Glu Thr Gly Cys Pro Pro Val Thr Ile Asn Gly Thr Gly Arg Phe Glu
   145                 150                 155                 160
   Ala Ser Arg Val Gln Ile Asp Gly Gly Leu Ser Ser Gln Tyr Val Ser
                   165                 170                 175
   Ala Leu Leu Met Met Ala Ala Gly Gly Asp Arg Ala Val Asp Val Glu
               180                 185                 190
   Leu Leu Gly Glu His Ile Gly Ala Leu Gly Tyr Ile Asp Leu Thr Val
           195                 200                 205
   Ala Ala Met Arg Ala Phe Gly Ala Lys Val Glu Arg Val Ser Pro Val
       210                 215                 220
   Ala Trp Arg Val Glu Pro Thr Gly Tyr His Ala Ala Asp Phe Val Ile
   225                 230                 235                 240
   Glu Pro Asp Ala Ser Ala Ala Thr Tyr Leu Trp Ala Ala Glu Val Leu
                   245                 250                 255
   Ser Gly Gly Lys Ile Asp Leu Gly Thr Pro Ala Glu Gln Phe Ser Gln
               260                 265                 270
   Pro Asp Ala Lys Ala Tyr Asp Leu Ile Ser Lys Phe Pro His Leu Pro
           275                 280                 285
   Ala Val Ile Asp Gly Ser Gln Met Gln Asp Ala Ile Pro Thr Leu Ala
       290                 295                 300
   Val Leu Ala Ala Phe Asn Glu Met Pro Val Arg Phe Val Gly Ile Glu
   305                 310                 315                 320
   Asn Leu Arg Val Lys Glu Cys Asp Arg Ile Arg Ala Leu Ser Ser Gly
                   325                 330                 335
   Leu Ser Arg Ile Val Pro Asn Leu Gly Thr Glu Glu Gly Asp Asp Leu
               340                 345                 350
   Ile Ile Ala Ser Asp Pro Ser Leu Ala Gly Lys Ile Leu Thr Ala Glu
           355                 360                 365
   Ile Asp Ser Phe Ala Asp His Arg Ile Ala Met Ser Phe Ala Leu Ala
       370                 375                 380
   Gly Leu Lys Ile Gly Gly Ile Thr Ile Leu Asp Pro Asp Cys Val Ala
   385                 390                 395                 400
   Lys Thr Phe Pro Ser Tyr Trp Asn Val Leu Ser Ser Leu Gly Val Ala
                   405                 410                 415
   Tyr Glu Asp

## Description

### FIELD OF THE INVENTION

This invention provides novel genes encoding 5-enolpyruvylshikimate-3-phosphate (EPSP) synthase that provide herbicide resistance. These genes are useful in plant biology, crop breeding, and plant cell culture.

### BACKGROUND OF THE INVENTION

N-phosphonomethylglycine, commonly referred to as glyphosate, is an important agronomic chemical. Glyphosate inhibits the enzyme that converts phosphoenolpyruvic acid (PEP) and 3-phosphoshikimic acid to 5-enolpyruvyl-3-phosphoshikimic acid. Inhibition of this enzyme (5-enolpyruvylshikimate-3-phosphate synthase; referred to herein as "EPSP synthase") kills plant cells by shutting down the shikimate pathway, thereby inhibiting aromatic acid biosynthesis.

Since glyphosate-class herbicides inhibit aromatic amino acid biosynthesis, they not only kill plant cells, but are also toxic to bacterial cells. Glyphosate inhibits many bacterial EPSP synthases, and thus is toxic to these bacteria. However, certain bacterial EPSP synthases have a high tolerance to glyphosate.

Plant cells resistant to glyphosate toxicity can be produced by transforming plant cells to express glyphosate-resistant bacterial EPSP synthases. Notably, the bacterial gene from *Agrobacterium tumefaciens* strain CP4 has been used to confer herbicide resistance on plant cells following expression in plants. A mutated EPSP synthase from *Salmonella typhimurium* strain CT7 confers glyphosate resistance in bacterial cells, and confers glyphosate resistance on plant cells (U.S. Patent Nos. 4,535,060; 4,769,061; and 5,094,945).

U.S. patent 6,040,497 reports mutant maize EPSP synthase enzymes having substitutions of threonine to isoleucine at position 102 and proline to serine at position 106 (the "TIPS" mutation). Such alterations confer glyphosate resistance upon the maize enzyme. A mutated EPSP synthase from *Salmonella typhimurium* strain CT7 confers glyphosate resistance in bacterial cells, and is reported to confer glyphosate resistance upon plant cells (U.S. Patent Nos. 4,535,060; 4,769,061; and 5,094,945). He et al. ((2001) Biochim et Biophysica Acta 1568:1-6) have developed EPSP synthases with increased glyphosate tolerance by mutagenesis and recombination between the *E. coli* and *Salmonella typhimurium* EPSP synthase genes, and suggest that mutations at position 42 (T42M) and position 230 (Q230K) are likely responsible for the observed resistance.

Subsequent work *(*He et al. (2003) Biosci. Biotech. Biochem. 67:1405-1409) shows that the T42M mutation (threonine to methionine) is sufficient to improve tolerance of both the *E. coli* and *Salmonella typhimurium* enzymes. These enzymes contain amino acid substitutions in their active sites that prevent the binding of glyphosate without affecting binding by PEP or S3P. Mutations that occur in the hinge region between the two globular domains of EPSP synthase have been shown to alter the binding affinity of glyphosate but not PEP *(*He *et al.,* 2003, *supra).* Therefore, such enzymes have high catalytic activity, even in the presence of glyphosate.

Due to the many advantages herbicide resistance plants provide, methods for identifying herbicide resistance genes with glyphosate resistance activity are desirable.

### SUMMARY OF INVENTION

Compositions and methods for conferring herbicide resistance or tolerance to bacteria, plants, plant cells, tissues and seeds are provided. Compositions include nucleic acid molecules encoding herbicide resistance or tolerance polypeptides, vectors comprising those nucleic acid molecules, and host cells comprising the vectors. Compositions also include antibodies to the herbicide resistance or tolerance polypeptides. As noted the nucleotide sequences of the invention can be used in DNA constructs or expression cassettes for transformation and expression in organisms, including microorganisms and plants. Compositions also comprise transformed bacteria, plants, plant cells, tissues, and seeds. In addition, methods are provided for producing the polypeptides encoded by the synthetic nucleotides of the invention.

In particular, isolated nucleic acid molecules and variants thereof encoding herbicide resistance- or tolerance polypeptides are provided. Additionally, amino acid sequences and variants thereof encoded by the polynucleotides that confer herbicide resistance or tolerance are encompassed. In particular, the present invention provides for isolated nucleic acid molecules comprising the nucleotide sequence set forth in SEQ ID NO: 1, 3, 5, 7, 9, 11, 13, 28, 29, 30, 31, 32, 33, or 34, a nucleotide sequence encoding the amino acid sequence shown in SEQ ID NO:2, 4, 6, 8, 10, 12, or 14, the herbicide resistance nucleotide sequence deposited in a bacterial host as Accession Nos. NRRL B-30911, NRRL B-30912, NRRL B-30913, NRRL B-30914 N, NRRL B-30915, NRRL B-30916 N, or NRRL B-30917 N, as well as variants and fragments thereof. Nucleotide sequences that are complementary to a nucleotide sequence of the invention, or that hybridize to a sequence of the invention are also encompassed.

### DESCRIPTION OF FIGURES

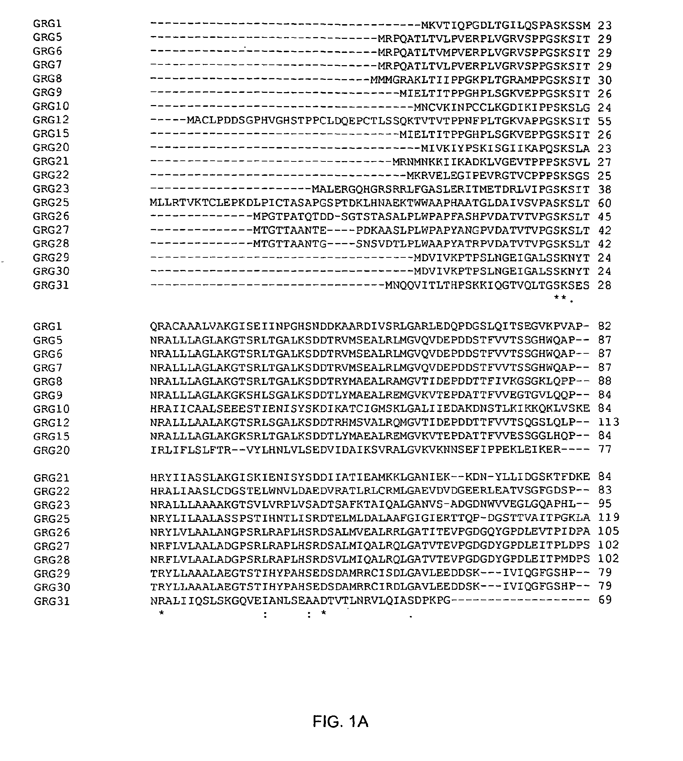

Figures 1A-1E show an alignment of the amino acid sequence of GRG25 (SEQ ID NO:2), GRG26 (SEQ ID NO:4), GRG27 (SEQ ID NO:6), GRG28 (SEQ ID NO:8), GRG29 (SEQ ID NO:10), GRG30 (SEQ ID NO:12), and GRG31 (SEQ ID NO:14) with other glyphosate tolerant EPSP synthase genes, including GRG1 (SEQ ID NO:15), GRG5 (SEQ ID NO:16), GRG6 (SEQ ID NO:17), GRG7 (SEQ ID NO:18), GRG8 (SEQ ID NO:19), GRG9 (SEQ ID NO:20), GRG10 (SEQ ID NO:21), GRG12 (SEQ ID NO:22), GRG 15 (SEQ ID NO:23), GRG20 (SEQ ID NO:24), GRG21 (SEQ ID NO:25), GRG22 (SEQ ID NO:26), and GRG23 (SEQ ID NO:27). The symbol [*] indicates that the residues are identical in all sequences in the alignment; the [:] symbol indicates conservative substitutions; the [.] symbol indicates semi-conservative substitutions.

### DETAILED DESCRIPTION

The present invention is drawn to compositions and methods for regulating herbicide resistance in organisms, particularly in plants or plant cells. The methods involve transforming organisms with a nucleotide sequence encoding a glyphosate resistance gene of the invention. In particular, a nucleotide sequence of the invention is useful for preparing plants that show increased tolerance to the herbicide glyphosate. Thus, transformed bacteria, plants, plant cells, plant tissues and seeds are provided. Compositions include nucleic acids and proteins relating to herbicide tolerance in microorganisms and plants as well as transformed bacteria, plants, plant tissues and seeds. More particularly, nucleotide sequences of the glyphosate resistance genes (*grg25, grg26, grg27, grg28, grg29, grg30, grg3l, syngrg25, syngrg26, syngrg27, syngrg28, syngrg29, syngrg30,* and *syngrg31*) and the amino acid sequences of the proteins encoded thereby are disclosed. The sequences find use in the construction of expression vectors for subsequent transformation into plants of interest, as probes for the isolation of other glyphosate resistance genes, as selectable markers, and the like. Thus, by "glyphosate resistance" or "glyphosate tolerance" gene of the invention is intended the nucleotide sequence set forth in SED ID NO: 1, 3, 5, 7, 9, 11, or 13 and fragments and variants thereof (e.g. SEQ ID NO:28-34) that encode a glyphosate resistance or tolerance polypeptide. Likewise, a "glyphosate resistance" or "glyphosate tolerance" polypeptide of the invention is a polypeptide having the amino acid sequence set forth in SEQ ID NO:2, 4, 6, 8, 10, 12, or 14, and fragments and variants thereof, that confer glyphosate resistance or tolerance to a host cell.

Plasmids containing the herbicide resistance nucleotide sequences of the invention were deposited in the permanent collection of the Agricultural Research Service Culture Collection, Northern Regional Research Laboratory (NRRL), 1815 North University Street, Peoria, Illinois 61604, United States of America, on April 19, 2006, and assigned Accession Nos. N NRRL B-30911 (for *grg25*), NRRL B-30912 (for *grg26*), NRRL B-30913 (for *grg27*), NRRL B-30914 N (for *grg28*), NRRL B-30915 (for *grg29*), NRRL B-30916 N (for *grg30*), and NRRL B-30917 (for *grg31*). This deposit will be maintained under the terms of the Budapest Treaty on the International Recognition of the Deposit of Microorganisms for the Purposes of Patent Procedure. This deposit was made merely as a convenience for those of skilled in the art and is not an admission that a deposit is required under 35 U.S.C. § 112.

By "glyphosate" is intended any herbicidal form of N-phosphonomethylglycine (including any salt thereof) and other forms that result in the production of the glyphosate anion in *planta.* An "herbicide resistance protein" or a protein resulting from expression of an "herbicide resistance-encoding nucleic acid molecule" includes proteins that confer upon a cell the ability to tolerate a higher concentration of an herbicide than cells that do not express the protein, or to tolerate a certain concentration of an herbicide for a longer period of time than cells that do not express the protein. A "glyphosate resistance protein" includes a protein that confers upon a cell the ability to tolerate a higher concentration of glyphosate than cells that do not express the protein, or to tolerate a certain concentration of glyphosate for a longer period of time than cells that do not express the protein. By "tolerate" or "tolerance" is intended either to survive, or to carry out essential cellular functions such as protein synthesis and respiration in a manner that is not readily discernable from untreated cells.

### Isolated Nucleic Acid Molecules, and Variants and Fragments Thereof

One aspect of the invention pertains to isolated or recombinant nucleic acid molecules comprising nucleotide sequences encoding herbicide resistance proteins and polypeptides or biologically active portions thereof, as well as nucleic acid molecules sufficient for use as hybridization probes to identify herbicide resistance-encoding nucleic acids. As used herein, the term "nucleic acid molecule" is intended to include DNA molecules (e.g., recombinant DNA, cDNA or genomic DNA) and RNA molecules (e.g., mRNA) and analogs of the DNA or RNA generated using nucleotide analogs. The nucleic acid molecules can be single-stranded or double-stranded, but preferably are double-stranded DNA.

Nucleotide sequences encoding the proteins of the present invention include the sequences set forth in SEQ ID NOS:1, 3, 5, 7, 9, 11, 13, 28, 29, 30, 31, 32, 33, and 34, the herbicide resistance nucleotide sequence deposited in a bacterial host as Accession Nos. NRRL B-30911, NRRL B-30912, NRRL B-30913, NRRL B-30914 N, NRRL B-30915, NRRL B-30916 N, and NRRL B-30917 N, and variants, fragments, and complements thereof. By "complement" is intended a nucleotide sequence that is sufficiently complementary to a given nucleotide sequence such that it can hybridize to the given nucleotide sequence to thereby form a stable duplex. The corresponding amino acid sequences for the herbicide resistance proteins encoded by these nucleotide sequences are set forth in SEQ ID NOS:2, 4, 6, 8, 10, 12, and 14. The invention also encompasses nucleic acid molecules comprising nucleotide sequences encoding partial-length herbicide resistance proteins, and complements thereof.

An "isolated" or "purified" nucleic acid molecule or protein, or biologically active portion thereof, is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For purposes of the invention, "isolated" when used to refer to nucleic acid molecules excludes isolated chromosomes. For example, in various embodiments, the isolated glyphosate resistance-encoding nucleic acid molecule can contain less than about 5 kb, 4 kb, 3 kb, 2 kb, 1 kb, 0.5 kb, or 0.1 kb of nucleotide sequence that naturally flanks the nucleic acid molecule in genomic DNA of the cell from which the nucleic acid is derived. An herbicide resistance protein that is substantially free of cellular material includes preparations of protein having less than about 30%, 20%, 10%, or 5% (by dry weight) of non-herbicide resistance protein (also referred to herein as a "contaminating protein").

Nucleic acid molecules that are fragments of these herbicide resistance-encoding nucleotide sequences are also encompassed by the present invention. By "fragment" is intended a portion of a nucleotide sequence encoding an herbicide resistance protein. A fragment of a nucleotide sequence may encode a biologically active portion of an herbicide resistance protein, or it may be a fragment that can be used as a hybridization probe or PCR primer using methods disclosed below. Nucleic acid molecules that are fragments of an herbicide resistance nucleotide sequence comprise at least about 15, 20, 50, 75, 100, 200, 300, 350, 400, 450, 500, 550, 600, 650, 700, 750, 800, 850, 900, 950, 1000, 1050, 1100, 1150, 1200, 1250, 1300, 1350, 1400 contiguous nucleotides, or up to the number of nucleotides present in a full-length herbicide resistance-encoding nucleotide sequence disclosed herein (for example, 1404 nucleotides for SEQ ID NO:1; 1395 nucleotides for SEQ ID NO:3, 1368 nucleotides for SEQ ID NO:5, etc) depending upon the intended use. By "contiguous" nucleotides is intended nucleotide residues that are immediately adjacent to one another.

Fragments of the nucleotide sequences of the present invention generally will encode protein fragments that retain the biological activity of the full-length glyphosate resistance protein; i.e., herbicide-resistance activity. By "retains herbicide resistance activity" is intended that the fragment will have at least about 30%, at least about 50%, at least about 70%, or at least about 80% of the herbicide resistance activity of the full-length glyphosate resistance protein disclosed herein as SEQ ID NOS:2, 4, 6, 8, 10, 12, or 14. Methods for measuring herbicide resistance activity are well known in the art. See, for example, U.S. Patent Nos. 4,535,060, and 5,188,642, each of which are herein incorporated by reference in their entirety.

A fragment of an herbicide resistance-encoding nucleotide sequence that encodes a biologically active portion of a protein of the invention will encode at least about 15, 25, 30, 50, 75, 100, 125, 150, 175, 200, 250, 300, 350, 400, 450 contiguous amino acids, or up to the total number of amino acids present in a full-length herbicide resistance protein of the invention (for example, 467 amino acids for SEQ ID NO:2; 464 for SEQ ID NO:4, 455 amino acids for SEQ ID NO:6, etc).

Preferred herbicide resistance proteins of the present invention are encoded by a nucleotide sequence sufficiently identical to the nucleotide sequence of SEQ ID NOS:1, 3, 5, 7, 9, 11, 13, 28, 29, 30, 31, 32, 33, or 34. The term "sufficiently identical" is intended an amino acid or nucleotide sequence that has at least about 60% or 65% sequence identity, about 70% or 75% sequence identity, about 80% or 85% sequence identity, or about 90%, 91%, 92%, 93%, 94%, 95%, 96%, 97%, 98% or 99% sequence identity compared to a reference sequence using one of the alignment programs described herein using standard parameters. One of skill in the art will recognize that these values can be appropriately adjusted to determine corresponding identity of proteins encoded by two nucleotide sequences by taking into account codon degeneracy, amino acid similarity, reading frame positioning, and the like.

To determine the percent identity of two amino acid sequences or of two nucleic acids, the sequences are aligned for optimal comparison purposes. The percent identity between the two sequences is a function of the number of identical positions shared by the sequences (i.e., percent identity = number of identical positions/total number of positions (e.g., overlapping positions) x 100). In one embodiment, the two sequences are the same length. The percent identity between two sequences can be determined using techniques similar to those described below, with or without allowing gaps. In calculating percent identity, typically exact matches are counted.

The determination of percent identity between two sequences can be accomplished using a mathematical algorithm. A nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the BLASTN and BLASTX programs of Altschul et al. (1990) J. Mol. Biol. 215:403. BLAST nucleotide searches can be performed with the BLASTN program, score = 100, wordlength = 12, to obtain nucleotide sequences homologous to glyphosate-resistant nucleic acid molecules of the invention. BLAST protein searches can be performed with the BLASTX program, score = 50, wordlength = 3, to obtain amino acid sequences homologous to herbicide resistance protein molecules of the invention. To obtain gapped alignments for comparison purposes, Gapped BLAST can be utilized as described in Altschul et al. (1997) Nucleic Acids Res. 25:3389. Alternatively, PSI-Blast can be used to perform an iterated search that detects distant relationships between molecules. See Altschul *et al.* (1997) *supra.* When utilizing BLAST, Gapped BLAST, and PSI-Blast programs, the default parameters of the respective programs (e.g., BLASTX and BLASTN) can be used. See www.ncbi.nlm.nih.gov. Another non-limiting example of a mathematical algorithm utilized for the comparison of sequences is the ClustalW algorithm *(*Higgins et al. (1994) Nucleic Acids Res. 22:4673-4680). ClustalW compares sequences and aligns the entirety of the amino acid or DNA sequence, and thus can provide data about the sequence conservation of the entire amino acid sequence. The ClustalW algorithm is used in several commercially available DNA/amino acid analysis software packages, such as the ALIGNX module of the Vector NTI Program Suite (Invitrogen Corporation, Carlsbad, CA). After alignment of amino acid sequences with ClustalW, the percent amino acid identity can be assessed. A non-limiting example of a software program useful for analysis of ClustalW alignments is GENEDOC™. GENEDOC™ (Karl Nicholas) allows assessment of amino acid (or DNA) similarity and identity between multiple proteins. Another non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG sequence alignment software package (available from Accelrys, Inc., 9865 Scranton Rd., San Diego, California, USA). When utilizing the ALIGN program for comparing amino acid sequences, a PAM120 weight residue table, a gap length penalty of 12, and a gap penalty of 4 can be used.

Unless otherwise stated, GAP Version 10, which uses the algorithm of Needleman and Wunsch (1970) *supra,* will be used to determine sequence identity or similarity using the following parameters: % identity and % similarity for a nucleotide sequence using GAP Weight of 50 and Length Weight of 3, and the nwsgapdna.cmp scoring matrix; % identity or % similarity for an amino acid sequence using GAP weight of 8 and length weight of 2, and the BLOSUM62 scoring program. Equivalent programs may also be used. By "equivalent program" is intended any sequence comparison program that, for any two sequences in question, generates an alignment having identical nucleotide residue matches and an identical percent sequence identity when compared to the corresponding alignment generated by GAP Version 10.

The invention also encompasses variant nucleic acid molecules. "Variants" of the herbicide resistance-encoding nucleotide sequences include those sequences that encode an herbicide resistance protein disclosed herein but that differ conservatively because of the degeneracy of the genetic code, as well as those that are sufficiently identical as discussed above. Naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the herbicide resistance proteins disclosed in the present invention as discussed below. Variant proteins encompassed by the present invention are biologically active, that is they retain the desired biological activity of the native protein, that is, herbicide resistance activity. By "retains herbicide resistance activity" is intended that the variant will have at least about 30%, at least about 50%, at least about 70%, or at least about 80% of the herbicide resistance activity of the native protein. Methods for measuring herbicide resistance activity are well known in the art. See, for example, U.S. Patent Nos. 4,535,060, and 5,188,642, each of which are herein incorporated by reference in their entirety.

The skilled artisan will further appreciate that changes can be introduced by mutation into the nucleotide sequences of the invention thereby leading to changes in the amino acid sequence of the encoded herbicide resistance protein, without altering the biological activity of the protein. Thus, variant isolated nucleic acid molecules can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed by the present invention.

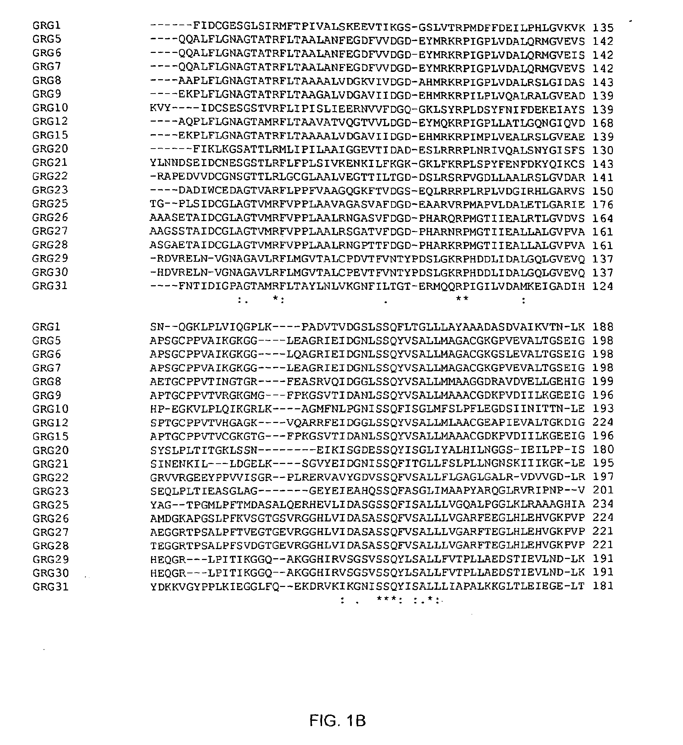

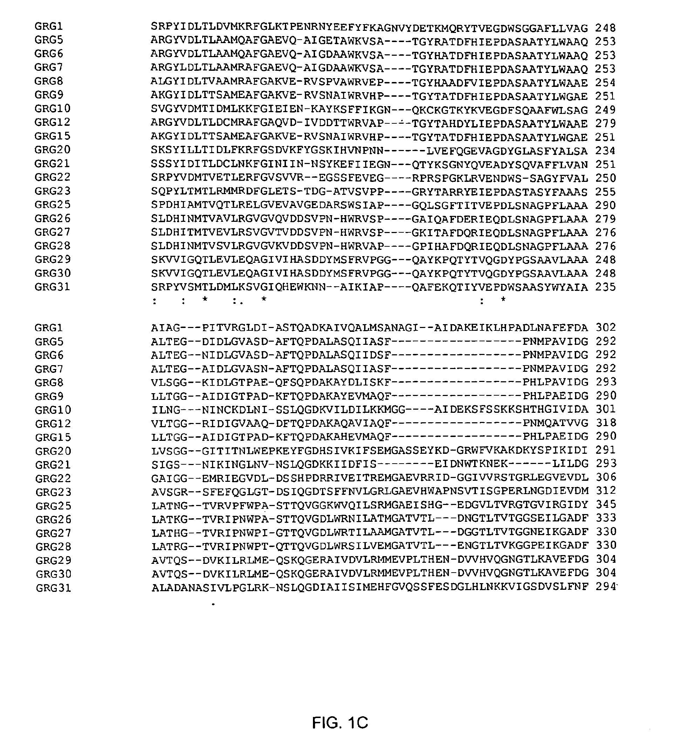

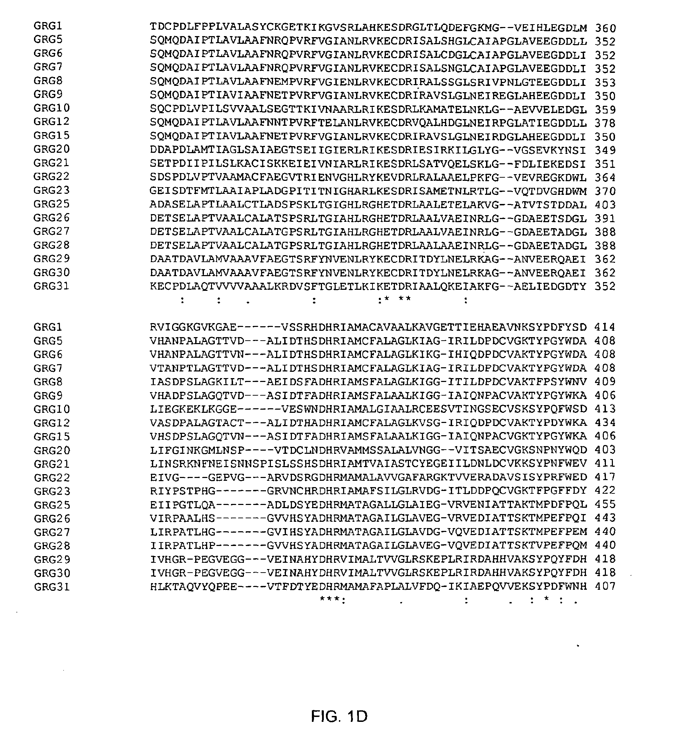

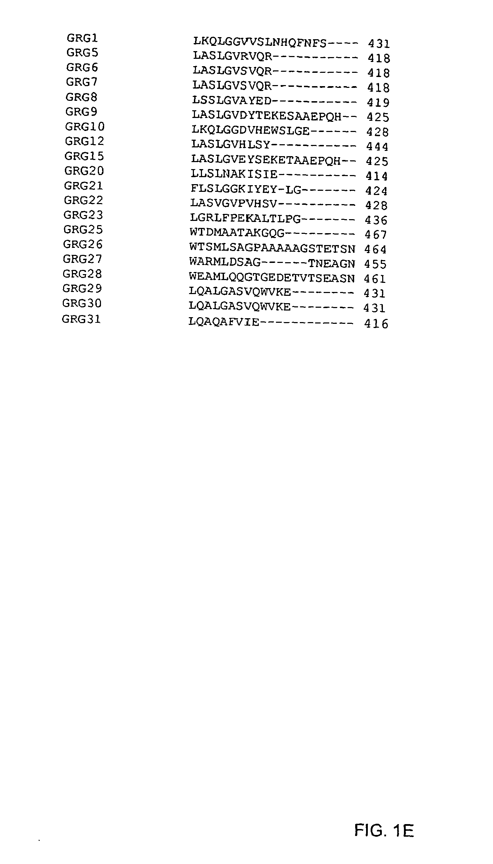

For example, conservative amino acid substitutions may be made at one or more predicted, preferably nonessential amino acid residues. A "nonessential" amino acid residue is a residue that can be altered from the wild-type sequence of an herbicide resistance protein without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Amino acid substitutions may be made in nonconserved regions that retain function. In general, such substitutions would not be made for conserved amino acid residues, or for amino acid residues residing within a conserved motif, where such residues are essential for protein activity. However, one of skill in the art would understand that functional variants may have minor conserved or nonconserved alterations in the conserved residues. Examples of residues that are conserved and that may be essential for protein activity include, for example, residues that are identical between all proteins contained in the alignment of Figure 1. Examples of residues that are conserved but that may allow conservative amino acid substitutions and still retain activity include, for example, residues that have only conservative substitutions between all proteins contained in the alignment of Figure 1.

Lys-22, Arg-124, Asp-313, Arg-344, Arg-386, and Lys-411, are conserved residues of the EPSP synthase from *E*. *coli* (Schönbrunn et al. (2001) Proc. Natl. Acad. Sci. USA 98:1376-1380). Conserved residues important for EPSP synthase activity also include Arg-100, Asp-242, and Asp-384 (Selvapandiyan et al. (1995) FEBS Letters 374:253-256). Arg-27 binds to S3P (Shuttleworth et al. (1999) Biochemistry 38:296-302).

Alternatively, variant nucleotide sequences can be made by introducing mutations randomly along all or part of the coding sequence, such as by saturation mutagenesis, and the resultant mutants can be screened for ability to confer herbicide resistance activity to identify mutants that retain activity. Following mutagenesis, the encoded protein can be expressed recombinantly, and the activity of the protein can be determined using standard assay techniques.

Using methods such as PCR, hybridization, and the like, corresponding herbicide resistance sequences can be identified, such sequences having substantial identity to the sequences of the invention. *See,* for example, Sambrook J., and Russell, D.W. (2001) Molecular Cloning: A Laboratory Manual. (Cold Spring Harbor Laboratory Press, Cold Spring Harbor, NY) and Innis, et al. (1990) PCR Protocols: A Guide to Methods and Applications (Academic Press, NY).

In a hybridization method, all or part of the herbicide resistance nucleotide sequence can be used to screen cDNA or genomic libraries. Methods for construction of such cDNA and genomic libraries are generally known in the art and are disclosed in Sambrook and Russell, 2001, *supra.* The so-called hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known herbicide resistance-encoding nucleotide sequences disclosed herein. Degenerate primers designed on the basis of conserved nucleotides or amino acid residues in the nucleotide sequences or encoded amino acid sequences can additionally be used. The probe typically comprises a region of nucleotide sequence that hybridizes under stringent conditions to at least about 12, preferably about 25, at least about 50, 75, 100, 125, 150, 175, 200, 250, 300, 350, 400, 500, 600, 700, 800, 900, 1000, 1200, 1300 consecutive nucleotides of an herbicide resistance-encoding nucleotide sequence of the invention or a fragment or variant thereof. Methods for the preparation of probes for hybridization are generally known in the art and are disclosed in Sambrook and Russell, 2001, *supra* and Sambrook et al. (1989) Molecular Cloning: A Laboratory Manual (2d ed., Cold Spring Harbor Laboratory Press, Cold Spring Harbor, New York), both of which are herein incorporated by reference.

For example, an entire herbicide resistance sequence disclosed herein, or one or more portions thereof, may be used as a probe capable of specifically hybridizing to corresponding herbicide resistance sequences and messenger RNAs. To achieve specific hybridization under a variety of conditions, such probes include sequences that are unique and are at least about 10 nucleotides in length, or at least about 20 nucleotides in length. Such probes may be used to amplify corresponding herbicide resistance sequences from a chosen organism by PCR. This technique may be used to isolate additional coding sequences from a desired organism or as a diagnostic assay to determine the presence of coding sequences in an organism. Hybridization techniques include hybridization screening of plated DNA libraries (either plaques or colonies; see, for example, Sambrook et al. (1989) Molecular Cloning: A Laboratory Manual (2d ed., Cold Spring Harbor Laboratory Press, Cold Spring Harbor, New York).

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances. By controlling the stringency of the hybridization and/or washing conditions, target sequences that are 100% complementary to the probe can be identified (homologous probing). Alternatively, stringency conditions can be adjusted to allow some mismatching in sequences so that lower degrees of similarity are detected (heterologous probing). Generally, a probe is less than about 1000 nucleotides in length, preferably less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCI, 1% SDS (sodium dodecyl sulfate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCI, 1% SDS at 37°C, and a wash in 0.5X to IX SSC at 55 to 60°C. Exemplary high stringency conditions include hybridization in 50% formamide, 1 M NaCI, 1% SDS at 37°C, and a wash in 0.1X SSC at 60 to 65°C. Optionally, wash buffers may comprise about 0.1% to about 1% SDS. Duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with ≥90% identity are sought, the Tₘ can be decreased 1O°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 1 S, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Cold Spring Harbor, New York).

### Isolated Proteins and Variants and Fragments Thereof

Herbicide resistance proteins are also encompassed within the present invention. By "herbicide resistance protein" is intended a protein having the amino acid sequence set forth in SEQ ID NOS:2, 4, 6, 8, 10, 12, or 14. Fragments, biologically active portions, and variants thereof are also provided, and may be used to practice the methods of the present invention.

"Fragments" or "biologically active portions" include polypeptide fragments comprising a portion of an amino acid sequence encoding an herbicide resistance protein as set forth in SEQ ID NOS:2, 4, 6, 8, 10, 12, or 14, and that retains herbicide resistance activity. A biologically active portion of an herbicide resistance protein can be a polypeptide that is, for example, 10, 25, 50, 100 or more amino acids in length. Such biologically active portions can be prepared by recombinant techniques and evaluated for herbicide resistance activity. Methods for measuring herbicide resistance activity are well known in the art. See, for example, U.S. Patent Nos. 4,535,060, and 5,188,642, each of which are herein incorporated by reference in their entirety. As used here, a fragment comprises at least 8 contiguous amino acids of SEQ ID NOS:2, 4, 6, 8, 10, 12, or 14. The invention encompasses other fragments, however, such as any fragment in the protein greater than about 10, 20, 30, 50, 100, 150, 200, 250, 300, 350, or 400 amino acids.

By "variants" is intended proteins or polypeptides having an amino acid sequence that is at least about 60%, 65%, about 70%, 75%, 80%, 85%, or 90%, 91%, 92%, 93%, 94%, 95%, 96%, 97%, 98% or 99% identical to the amino acid sequence of SEQ ID NOS:2, 4, 6, 8, 10, 12, or 14. Variants also include polypeptides encoded by a nucleic acid molecule that hybridizes to the nucleic acid molecule of SEQ ID NOS:1, 3, 5, 7,9,11,13, 28,29, 30, 31, 32, 33, or 34, or a complement thereof, under stringent conditions. Variants include polypeptides that differ in amino acid sequence due to mutagenesis. Variant proteins encompassed by the present invention are biologically active, that is they continue to possess the desired biological activity of the native protein, that is, retaining herbicide resistance activity. Methods for measuring herbicide resistance activity are well known in the art. See, for example, U.S. Patent Nos. 4,535,060, and 5,188,642, each of which are herein incorporated by reference in their entirety.

Bacterial genes, such as the *grg25, grg26, grg27, grg28, grg29, grg30, grg3l, syngrg25, syngrg26, syngrg27, syngrg28, syngrg29, syngrg30,* and *syngrg31* genes of this invention, quite often possess multiple methionine initiation codons in proximity to the start of the open reading frame. Often, translation initiation at one or more of these start codons will lead to generation of a functional protein. These start codons can include ATG codons. However, bacteria such as *Bacillus sp.* also recognize the codon GTG as a start codon, and proteins that initiate translation at GTG codons contain a methionine at the first amino acid. Furthermore, it is not often determined a *priori* which of these codons are used naturally in the bacterium. Thus, it is understood that use of one of the alternate methionine codons may lead to generation of variants *of grg25, grg26, grg27, grg28, grg29, grg30, grg3l, syngrg25, syngrg26, syngrg27, syngrg28, syngrg29, syngrg30, and syngrg31* that confer herbicide resistance. These herbicide resistance proteins are encompassed in the present invention and may be used in the methods of the present invention.

Antibodies to the polypeptides of the present invention, or to variants or fragments thereof, are also encompassed. Methods for producing antibodies are well known in the art (see, for example, Harlow and Lane (1988) Antibodies: A Laboratory Manual, Cold Spring Harbor Laboratory, Cold Spring Harbor, N.Y.; U.S. Patent No. 4,196,265).

### Altered or Improved Variants

It is recognized that DNA sequences *of grg25, grg26, grg27, grg28, grg29, grg30, grg31, syngrg25, syngrg26, syngrg27, syngrg28, syngrg29, syngrg30,* and *syngrg31* may be altered by various methods, and that these alterations may result in DNA sequences encoding proteins with amino acid sequences different than that encoded *by grg25, grg26, grg27, grg28, grg29, grg30,* or *grg31,* respectively. This protein may be altered in various ways including amino acid substitutions, deletions, truncations, and insertions of one or more amino acids of SEQ ID NO:1, 3, 5, 7, 9, 11, 13, 28, 29, 30, 31, 32, 33, or 34, including up to about 2, about 3, about 4, about 5, about 6, about 7, about 8, about 9, about 10, about 15, about 20, about 25, about 30, about 35, about 40, about 45, about 50, about 55, about 60, about 65, about 70, about 75, about 80, about 85, about 90, about 100 or more amino acid substitutions, deletions or insertions.

Methods for such manipulations are generally known in the art. For example, amino acid sequence variants of the GRG proteins disclosed herein can be prepared by mutations in the DNA. This may also be accomplished by one of several forms of mutagenesis and/or in directed evolution. In some aspects, the changes encoded in the amino acid sequence will not substantially affect function of the protein. Such variants will possess the desired herbicide resistance activity. However, it is understood that the ability of GRG25, GRG26, GRG27, GRG28, GRG29, GRG30, or GRG31 to confer herbicide resistance may be improved by one use of such techniques upon the compositions of this invention. For example, one may express GRG25, GRG26, GRG27, GRG28, GRG29, GRG30, or GRG31 in host cells that exhibit high rates of base misincorporation during DNA replication, such as XL-1 Red (Stratagene, La Jolla, CA). After propagation in such strains, one can isolate the DNA of the invention (for example by preparing plasmid DNA, or by amplifying by PCR and cloning the resulting PCR fragment into a vector), culture the *grg* mutations in a non-mutagenic strain, and identify mutated genes with improved resistance to an herbicide such as glyphosate, for example by growing cells in increasing concentrations of glyphosate and testing for clones that confer ability to tolerate increased concentrations of glyphosate.

Alternatively, alterations may be made to the protein sequence of many proteins at the amino or carboxy terminus without substantially affecting activity. This can include insertions, deletions, or alterations introduced by modem molecular methods, such as PCR, including PCR amplifications that alter or extend the protein coding sequence by virtue of inclusion of amino acid encoding sequences in the oligonucleotides utilized in the PCR amplification. Alternatively, the protein sequences added can include entire protein-coding sequences, such as those used commonly in the art to generate protein fusions. Such fusion proteins are often used to (1) increase expression of a protein of interest, (2) introduce a binding domain, enzymatic activity, or epitope to facilitate either protein purification, protein detection, or other experimental uses known in the art, or, (3) target secretion or translation of a protein to a subcellular organelle, such as the periplasmic space of gram-negative bacteria, or the endoplasmic reticulum of eukaryotic cells, the latter of which often results in glycosylation of the protein.

Variant nucleotide and amino acid sequences of the present invention also encompass sequences derived from mutagenic and recombinogenic procedures such as DNA shuffling. With such a procedure, one or more different herbicide resistance protein coding regions can be used to create a new herbicide resistance protein possessing the desired properties. In this manner, libraries of recombinant polynucleotides are generated from a population of related sequence polynucleotides comprising sequence regions that have substantial sequence identity and can be homologously recombined *in vitro or in vivo.* For example, using this approach, sequence motifs encoding a domain of interest may be shuffled between the herbicide resistance gene of the invention and other known herbicide resistance genes to obtain a new gene coding for a protein with an improved property of interest, such as an increased glyphosate resistance activity. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad. Sci. USA 94:4504-4509; Crameri et al. (1998) Nature 391:288-291; and U.S. Patent Nos. 5,605,793 and 5,837,458.

### Temperature spectrum

Several studies of glyphosate metabolism in plants have been carried out, and reveal that glyphosate is not metabolized by plants or is metabolized very slowly. Glyphosate penetrates the cuticle rapidly, and is translocated throughout plants over a considerable period of time (reviewed in Kearney and Kaufman, Eds (1988) Herbicides; Chemistry, Degradation & Mode of Action Marcel Dekker , Inc., New York, 3:1-70 and Grossbard and Atkinson, Eds. (1985) The Herbicide Glyphosate Butterworths, London, p. 25-34). Thus, it is likely that glyphosate tolerance is necessary over a sustained period of time following glyphosate exposure in agronomically-important plants. Where temperatures frequently exceed 30°C during the growing season, it would be advantageous to employ a glyphosate-tolerance EPSP synthase that maintains activity at elevated temperatures.

In one embodiment of the present invention, the EPSP synthase exhibits thermal stability at a temperature that is higher or lower than ambient environmental temperature. By "thermal stability" is intended that the enzyme is active at a higher or lower temperature than ambient environmental temperature for a longer period of time than an EPSP synthase that is not thermal stable at that temperature. For example, a thermal stable EPSP synthase has enzymatic activity for greater than about 1 hour, greater than about 2 hours, about 3, about 4, about 5, about 6, about 7, about 8, about 9, about 10, about 11, about 12, about 13, about 14, about 15, about 20, about 25 hours, or longer, at a temperature that is higher or lower than ambient environmental temperature. For the purposes of the present invention, "ambient" environmental temperature is about 30°C. In some embodiments, a higher than ambient temperature is a temperature at or above about 32°C, about 34°C, about 37°C, about 40°C, about 45°C, about 50°C, about 55°C, about 60°C, about 65°C, or higher. A lower than ambient temperature is a temperature at or below about 28°C, below about 27°C, about 26°C, about 25°C, about 23°C, about 20°C, about 18°C, about 15°C, about 10°C, at or below about 5°C, or around O°C. Methods to assay for EPSP synthase activity are discussed in further details elsewhere herein. For the purposes of the present invention, a thermal stable EPSP synthase is considered active when it functions at about 90% to 100%, about 80% to about 90%, about 70% to about 80%, about 60% to about 70% or about 50% to about 60% of the maximum activity level observed at the optimum temperature for that enzyme.

In another embodiment, the temperature optimum for an EPSP synthase enzyme of the invention is higher or lower than the temperature optimum of a wild-type glyphosate tolerance EPSP synthase enzyme. For the purposes of the present invention, a wild type glyphosate tolerance EPSP synthase enzyme is one that has maximal activity at ambient environmental temperature (e.g., the glyphosate tolerance EPSP synthase enzyme described at GRG23 in U.S. Patent Application No. 11/605,824, filed November 29, 2006, herein incorporated by reference in its entirety). By "optimum" is intended the maximal activity observed for an EPSP synthase enzyme, for example, when measured across multiple temperature ranges. In non-limiting examples, the EPSP synthase employed in the methods of the invention can have optimal activity from about 0°C to about 10°C, about 10°C to about 20°C, about 20°C to about 30°C, about 30°C to about 40°C, about 40°C to about 50°C, about 50°C to about 60°C, about 60°C to about 70°C, or about 70°C to about 80°C.

Thus, provided herein are methods and compositions for increasing glyphosate tolerance at temperatures higher or lower than ambient environmental temperatures. In one embodiment, the methods comprise introducing into a plant a nucleotide sequence encoding a glyphosate tolerance EPSP synthase enzyme that is thermal stable at a temperature that is higher or lower than ambient temperature, and growing the plant at a temperature that is higher or lower, respectively, than ambient environmental temperature. In specific embodiments, the growing temperature is higher or lower than ambient temperature for an average of at least about 2 hours per day, at least about 3 hours per day, at least about 4 hours per day, at least about 5, about 6, about 7, about 8, about 9, about 10, about 11, about 12, about 14, about 16, about 18, about 20, at least about 22 hours per day, or up to about 24 hours a day during the growing season of the plant.

In another embodiment, the method comprises introducing into a plant a nucleotide sequence encoding a glyphosate tolerant EPSP synthase enzyme other than SEQ ID NO:35, 36, or 37 that has a temperature optimum higher than ambient environmental temperature, contacting the plant with an herbicidally-effective concentration of glyphosate, and growing said plant at temperature that exceeds ambient environmental temperature for at least 1 hour, at least about 2 hours, at least about 3, at least about 4, or more hours per day for at least 2, 3, 4, 5, 6, 7, 8, 9, 10, 11, 12, 13, 14, 15, 16, 17, 18, 19, 20 or more days after glyphosate is applied to the plant, wherein the days in which the temperature exceeds ambient environmental temperature occur during the growing season of the plant. In another embodiment, the method comprises introducing into a plant a nucleotide sequence encoding a glyphosate tolerant EPSP synthase enzyme other than SEQ ID NO:38 that has a temperature optimum lower than ambient environmental temperature, contacting the plant with an herbicidally-effective concentration of glyphosate, and growing said plant at a temperature that is below ambient environmental temperature for at least about 1 hour, at least about 2 hours, at least about 3, at least about 4, or more hours per day for at least 2, 3, 4, 5, 6, 7, 8, 9, 10, 11, 12, 13, 14, 15, 16, 17, 18, 19, 20 or more days after glyphosate is applied to the plant, wherein the days in which the temperature exceeds ambient environmental temperature occur during the growing season of the plant.

In yet another embodiment, the method comprises introducing into a plant a nucleotide sequence encoding a glyphosate tolerant EPSP synthase that is thermal stable at temperatures higher than ambient environmental temperature, contacting the plant with an herbicidally-effective concentration of glyphosate, and growing the plant at a temperature that is higher than ambient environmental temperature, wherein the temperature is higher than ambient environmental temperature for at least about 1 hour, at least about 2 hours, at least about 3, at least about 4, or more hours per day for at least 2, 3, 4, 5, 6, 7, 8, 9, 10, 11, 12, 13, 14, 15, 16, 17, 18, 19, 20 or more days after glyphosate is applied to the plant, wherein the days in which the temperature exceeds ambient environmental temperature occur during the growing season of the plant. In one non-limiting example, the thermal stable EPSP synthase enzyme is set forth in SEQ ID NO:14. Alternatively, the method comprises introducing into a plant a nucleotide sequence encoding a glyphosate tolerant EPSP synthase enzyme that is thermal stable at temperatures lower than ambient environmental temperature, contacting the plant with an herbicidally-effective concentration of glyphosate, and growing the plant at a temperature that is lower than ambient environmental temperature for at least about 1 hour, at least about 2 hours, at least about 3, at least about 4, or more hours per day for at least 2, 3, 4, 5, 6, 7, 8, 9, 10, 11, 12, 13, 14, 15, 16, 17, 18, 19, 20 or more days after glyphosate is applied to the plant, wherein the days in which the temperature exceeds ambient environmental temperature occur during the growing season of the plant.

In various embodiments, the glyphosate tolerance EPSP synthase enzyme that is thermal stable at temperatures higher or lower than ambient environmental temperatures, or has a thermal optimum at a temperature lower or higher than ambient environmental temperature, is not a plant-derived EPSP synthase. By "plant-derived" is intended the native plant EPSP synthase sequence, or a sequence that is at least about 80%, at least about 85%, at least about 90%, about 91 %, about 92%, about 93%, about 94%, about 95%, about 96%, about 97%, about 98%, or at least about 99% identical to the native plant sequence. See, for example, the plant-derived glyphosate tolerance EPSP synthase sequences described in U.S. Patent Application Publication Nos. 20060143727, 20030049814, 20030079246, 20030200560, 2004148650, and 20050223436; U.S. Patent Nos. 5188642, 6040497, 7214535, 7169970, 6867293, 7183110, 5094945, 6225114, 141722, 7045684, 5312910, 6566587, and RE037287, each of which is herein incorporated by reference in its entirety.

### Transformation of Bacterial or Plant Cells

Provided herein are novel isolated genes that confer resistance to an herbicide. Also provided are amino acid sequences of the GRG proteins of the invention. The protein resulting from translation of this gene allows cells to function in the presence of concentrations of an herbicide that are otherwise toxic to cells including plant cells and bacterial cells. In one aspect of the invention, the *grg25, grg26, grg27, grg28, grg29, grg30, grg31, syngrg25, syngrg26, syngrg27, syngrg28, syngrg29, syngrg30,* and *syngrg31* genes are useful as markers to assess transformation of bacterial or plant cells. Methods for detecting the presence of a transgene in a plant, plant organ (e.g., leaves, stems, roots, etc.), seed, plant cell, propagule, embryo or progeny of the same are well known in the art.

By engineering the genes of the invention to be expressed from a promoter known to stimulate transcription in the organism to be tested and properly translated to generate an intact GRG peptide, and placing the cells in an otherwise toxic concentration of herbicide, one can identify cells that have been transformed with the DNA by virtue of their resistance to herbicide. By "promoter" is intended a nucleic acid sequence that functions to direct transcription of a downstream coding sequence. The promoter, together with other transcriptional and translational regulatory nucleic acid sequences, (also termed as "control sequences") are necessary for the expression of a DNA sequence of interest.

Transformation of bacterial cells is accomplished by one of several techniques known in the art, including but not limited to electroporation or chemical transformation (see, for example, Ausubel, ed. (1994) Current Protocols in Molecular Biology, John Wiley and Sons, Inc., Indianapolis, IN). Markers conferring resistance to toxic substances are useful in identifying transformed cells (having taken up and expressed the test DNA) from non-transformed cells (those not containing or not expressing the test DNA). In one aspect of the invention, the *grg25, grg26, grg27, grg28, grg29, grg30, grg31, syngrg25, syngrg26, syngrg27, syngrg28, syngrg29, syngrg30,* and *syngrg31* genes are useful as markers to assess transformation of bacterial or plant cells.

Transformation of plant cells can be accomplished in similar fashion. By "plant" is intended whole plants, plant organs (e.g., leaves, stems, roots, etc.), seeds, plant cells, propagules, embryos and progeny of the same. Plant cells can be differentiated or undifferentiated (e.g. callus, suspension culture cells, protoplasts, leaf cells, root cells, phloem cells, pollen). "Transgenic plants" or "transformed plants" or "stably transformed" plants or cells or tissues refer to plants that have incorporated or integrated exogenous nucleic acid sequences or DNA fragments into the plant cell. By "stable transformation" is intended that the nucleotide construct introduced into a plant integrates into the genome of the plant and is capable of being inherited by progeny thereof.

The *grg* genes of the invention may be modified to obtain or enhance expression in plant cells. The herbicide resistance sequences of the invention may be provided in expression cassettes for expression in the plant of interest. "Plant expression cassette" includes DNA constructs, including recombinant DNA constructs, that are capable of resulting in the expression of a protein from an open reading frame in a plant cell. The cassette will include in the 5'-3' direction of transcription, a transcriptional initiation region (i.e., promoter, particularly a heterologous promoter) operably-linked to a DNA sequence of the invention, and/or a transcriptional and translational termination region (i.e., termination region) functional in plants. The cassette may additionally contain at least one additional gene to be cotransformed into the organism, such as a selectable marker gene. Alternatively, the additional gene(s) can be provided on multiple expression cassettes. Such an expression cassette is provided with a plurality of restriction sites for insertion of the herbicide resistance sequence to be under the transcriptional regulation of the regulatory regions.

The promoter may be native or analogous, or foreign or heterologous, to the plant host and/or to the DNA sequence of the invention. Additionally, the promoter may be the natural sequence or alternatively a synthetic sequence. Where the promoter is "native" or "homologous" to the plant host, it is intended that the promoter is found in the native plant into which the promoter is introduced. Where the promoter is "foreign" or "heterologous" to the DNA sequence of the invention, it is intended that the promoter is not the native or naturally occurring promoter for the operably linked DNA sequence of the invention. "Heterologous" generally refers to the nucleic acid sequences that are not endogenous to the cell or part of the native genome in which they are present, and have been added to the cell by infection, transfection, microinjection, electroporation, microprojection, or the like. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame.

Often, such constructs will also contain 5' and 3' untranslated regions. Such constructs may contain a "signal sequence" or "leader sequence" to facilitate cotranslational or post-translational transport of the peptide of interest to certain intracellular structures such as the chloroplast (or other plastid), endoplasmic reticulum, or Golgi apparatus, or to be secreted. For example, the gene can be engineered to contain a signal peptide to facilitate transfer of the peptide to the endoplasmic reticulum. By "signal sequence" is intended a sequence that is known or suspected to result in cotranslational or post-translational peptide transport across the cell membrane. In eukaryotes, this typically involves secretion into the Golgi apparatus, with some resulting glycosylation. By "leader sequence" is intended any sequence that when translated, results in an amino acid sequence sufficient to trigger co-translational transport of the peptide chain to a sub-cellular organelle. Thus, this includes leader sequences targeting transport and/or glycosylation by passage into the endoplasmic reticulum, passage to vacuoles, plastids including chloroplasts, mitochondria, and the like. It may also be preferable to engineer the plant expression cassette to contain an intron, such that mRNA processing of the intron is required for expression.

By "3' untranslated region" is intended a nucleotide sequence located downstream of a coding sequence. Polyadenylation signal sequences and other sequences encoding regulatory signals capable of affecting the addition of polyadenylic acid tracts to the 3' end of the mRNA precursor are 3' untranslated regions. By "5' untranslated region" is intended a nucleotide sequence located upstream of a coding sequence.

Other upstream or downstream untranslated elements include enhancers. Enhancers are nucleotide sequences that act to increase the expression of a promoter region. Enhancers are well known in the art and include, but are not limited to, the SV40 enhancer region and the 35S enhancer element.

The termination region may be native with the transcriptional initiation region, may be native with the herbicide resistance sequence of the present invention, or may be derived from another source. Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

In one aspect of the invention, synthetic DNA sequences are designed for a given polypeptide, such as the polypeptides of the invention. Expression of the open reading frame of the synthetic DNA sequence in a cell results in production of the polypeptide of the invention. Synthetic DNA sequences can be useful to simply remove unwanted restriction endonuclease sites, to facilitate DNA cloning strategies, to alter or remove any potential codon bias, to alter or improve GC content, to remove or alter alternate reading frames, and/or to alter or remove intron/exon splice recognition sites, polyadenylation sites, Shine-Delgamo sequences, unwanted promoter elements and the like that may be present in a native DNA sequence. It is also possible that synthetic DNA sequences may be utilized to introduce other improvements to a DNA sequence, such as introduction of an intron sequence, creation of a DNA sequence that in expressed as a protein fusion to organelle targeting sequences, such as chloroplast transit peptides, apoplast/vacuolar targeting peptides, or peptide sequences that result in retention of the resulting peptide in the endoplasmic reticulum. Synthetic genes can also be synthesized using host cell-preferred codons for improved expression, or may be synthesized using codons at a host-preferred codon usage frequency. See, for example, Campbell and Gowri (1990) Plant Physiol. 92: 1-11; U.S. Patent Nos. 6,320,100; 6,075,185; 5,380,831; and 5,436,391, U.S. Published Application Nos. 20040005600 and 20010003849, and Murray et al. (1989) Nucleic Acids Res. 17:477-498, herein incorporated by reference.

In one embodiment, the nucleic acids of interest are targeted to the chloroplast for expression. In this manner, where the nucleic acid of interest is not directly inserted into the chloroplast, the expression cassette will additionally contain a nucleic acid encoding a transit peptide to direct the gene product of interest to the chloroplasts. Such transit peptides are known in the art. See, for example, Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

The nucleic acids of interest to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831, herein incorporated by reference.

Typically this "plant expression cassette" will be inserted into a "plant transformation vector." By "transformation vector" is intended a DNA molecule that is necessary for efficient transformation of a cell. Such a molecule may consist of one or more expression cassettes, and may be organized into more than one "vector" DNA molecule. For example, binary vectors are plant transformation vectors that utilize two non-contiguous DNA vectors to encode all requisite cis- and trans-acting functions for transformation of plant cells (Hellens and Mullineaux (2000) Trends in Plant Science 5:446-451). "Vector" refers to a nucleic acid construct designed for transfer between different host cells. "Expression vector" refers to a vector that has the ability to incorporate, integrate and express heterologous DNA sequences or fragments in a foreign cell.

This plant transformation vector may be comprised of one or more DNA vectors needed for achieving plant transformation. For example, it is a common practice in the art to utilize plant transformation vectors that are comprised of more than one contiguous DNA segment. These vectors are often referred to in the art as "binary vectors." Binary vectors as well as vectors with helper plasmids are most often used for *Agrobacteriurn-mediated* transformation, where the size and complexity of DNA segments needed to achieve efficient transformation is quite large, and it is advantageous to separate functions onto separate DNA molecules. Binary vectors typically contain a plasmid vector that contains the cis-acting sequences required for T-DNA transfer (such as left border and right border), a selectable marker that is engineered to be capable of expression in a plant cell, and a "gene of interest" (a gene engineered to be capable of expression in a plant cell for which generation of transgenic plants is desired). Also present on this plasmid vector are sequences required for bacterial replication. The cis-acting sequences are arranged in a fashion to allow,efficient transfer into plant cells and expression therein. For example, the selectable marker gene and the gene of interest are located between the left and right borders. Often a second plasmid vector contains the trans-acting factors that mediate T-DNA transfer from *Agrobacterium* to plant cells. This plasmid often contains the virulence functions (Vir genes) that allow infection of plant cells by *Agrobacterium,* and transfer of DNA by cleavage at border sequences and vir-mediated DNA transfer, as is understood in the art (Hellens and Mullineaux (2000) Trends in Plant Science, 5:446-451). Several types of *Agrobacterium* strains (e.g. LBA4404, GV3101, EHA101, EHA105, etc.) can be used for plant transformation. The second plasmid vector is not necessary for transforming the plants by other methods such as microprojection, microinjection, electroporation, polyethylene glycol, etc.

### Plant Transformation

Methods of the invention involve introducing a nucleotide construct into a plant. By "introducing" is intended to present to the plant the nucleotide construct in such a manner that the construct gains access to the interior of a cell of the plant. The methods of the invention do not require that a particular method for introducing a nucleotide construct to a plant is used, only that the nucleotide construct gains access to the interior of at least one cell of the plant. Methods for introducing nucleotide constructs into plants are known in the art including, but not limited to, stable transformation methods, transient transformation methods, and virus-mediated methods.

In general, plant transformation methods involve transferring heterologous DNA into target plant cells (e.g. immature or mature embryos, suspension cultures, undifferentiated callus, protoplasts, etc.), followed by applying a maximum threshold level of appropriate selection (depending on the selectable marker gene and in this case "glyphosate") to recover the transformed plant cells from a group of untransformed cell mass. Explants are typically transferred to a fresh supply of the same medium and cultured routinely. Subsequently, the transformed cells are differentiated into shoots after placing on regeneration medium supplemented with a maximum threshold level of selecting agent (e.g. "glyphosate"). The shoots are then transferred to a selective rooting medium for recovering rooted shoot or plantlet. The transgenic plantlet then grow into mature plant and produce fertile seeds (e.g. Hiei et al. (1994) The Plant Journal 6:271-282; Ishida et al. (1996) Nature Biotechnology 14:745-750). Explants are typically transferred to a fresh supply of the same medium and cultured routinely. A general description of the techniques and methods for generating transgenic plants are found in Ayres and Park (1994) Critical Reviews in Plant Science 13:219-239 and Bommineni and Jauhar (1997) Maydica 42:107-120. Since the transformed material contains many cells, both transformed and non-transformed cells are present in any piece of subjected target callus or tissue or group of cells. The ability to kill non-transformed cells and allow transformed cells to proliferate results in transformed plant cultures. Often, the ability to remove non-transformed cells is a limitation to rapid recovery of transformed plant cells and successful generation of transgenic plants. Molecular and biochemical methods can then be used to confirm the presence of the integrated heterologous gene of interest in the genome of transgenic plant.

Generation of transgenic plants may be performed by one of several methods, including but not limited to introduction of heterologous DNA by *Agrobacterium* into plant cells *(Agrobacterium-mediated* transformation), bombardment of plant cells with heterologous foreign DNA adhered to particles, and various other non-particle direct-mediated methods (e.g. Hiei et al. (1994) The Plant Journal 6:271-282; Ishida et al. (1996) Nature Biotechnology 14:745-750; Ayres and Park (1994) Critical Reviews in Plant Science 13:219-239; Bommineni and Jauhar (1997) Maydica 42:107-120) to transfer DNA.

Methods for transformation of chloroplasts are known in the art. See, for example, Svab et al. (1990) Proc. Natl. Acad. Sci. USA 87:8526-8530; Svab and Maliga (1993) Pt-oc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a nucleotide construct of the invention, for example, an expression cassette of the invention, stably incorporated into their genome.

### Plants

The present invention may be used for transformation of any plant species, including, but not limited to, monocots and dicots. Examples of plants of interest include, but are not limited to, corn (maize), sorghum, wheat, sunflower, tomato, crucifers, peppers, potato, cotton, rice, soybean, sugarbeet, sugarcane, tobacco, barley, and oilseed rape, *Brassica* sp., alfalfa, rye, millet, safflower, peanuts, sweet potato, cassava, coffee, coconut, pineapple, citrus trees, cocoa, tea, banana, avocado, fig, guava, mango, olive, papaya, cashew, macadamia, almond, oats, vegetables, ornamentals, and conifers.

Vegetables include, but are not limited to, tomatoes, lettuce, green beans, lima beans, peas, and members of the genus *Curcumis* such as cucumber, cantaloupe, and musk melon. Ornamentals include, but are not limited to, azalea, hydrangea, hibiscus, roses, tulips, daffodils, petunias, carnation, poinsettia, and chrysanthemum. Preferably, plants of the present invention are crop plants (for example, maize, sorghum, wheat, sunflower, tomato, crucifers, peppers, potato, cotton, rice, soybean, sugarbeet, sugarcane, tobacco, barley, oilseed rape, etc.).

This invention is particularly suitable for any member of the monocot plant family including, but not limited to, maize, rice, barley, oats, wheat, sorghum, rye, sugarcane, pineapple, yams, onion, banana, coconut, and dates.

### Evaluation of Plant Transformation

Following introduction of heterologous foreign DNA into plant cells, the transformation or integration of heterologous gene in the plant genome is confirmed by various methods such as analysis of nucleic acids, proteins and metabolites associated with the integrated gene.

PCR analysis is a rapid method to screen transformed cells, tissue or shoots for the presence of incorporated gene at the earlier stage before transplanting into the soil (Sambrook and Russell (2001) Molecular Cloning: A Laboratory Manual, Cold Spring Harbor Laboratory Press, Cold Spring Harbor, NY). PCR is carried out using oligonucleotide primers specific to the gene of interest or *Agrobacterium* vector background, etc.

Plant transformation may be confirmed by Southern blot analysis of genomic DNA (Sambrook and Russell, 2001, *supra).* In general, total DNA is extracted from the transformant, digested with appropriate restriction enzymes, fractionated in an agarose gel and transferred to a nitrocellulose or nylon membrane. The membrane or "blot" is then probed with, for example, radiolabeled ³²P target DNA fragments to confirm the integration of the introduced gene in the plant genome according to standard techniques (Sambrook and Russell, 2001, *supra).*

In Northern analysis, RNA is isolated from specific tissues of transformant, fractionated in a formaldehyde agarose gel, blotted onto a nylon filter according to standard procedures that are routinely used in the art (Sambrook and Russell, 2001, *supra).* Expression of RNA encoded by *grg25, grg26, grg27, grg28, grg29, grg30, grg31, syngrg25, .ryngrg26, syngrg27, syngrg28, syngrg29, syngrg30,* and *syngrg31* is then tested by hybridizing the filter to a radioactive probe derived from a polynucleotide of the invention, by methods known in the art (Sambrook and Russell, *2001, supra)*

Western blot and biochemical assays and the like may be carried out on the transgenic plants to determine the presence of protein encoded by the herbicide resistance gene by standard procedures (Sambrook and Russell, 2001, *supra)* using antibodies that bind to one or more epitopes present on the herbicide resistance protein.

### Methods for increasing plant yield

Methods for increasing plant yield are provided. The methods comprise introducing into a plant or plant cell a polynucleotide comprising a *grg* sequence disclosed herein. As defined herein, the "yield" of the plant refers to the quality and/or quantity of biomass produced by the plant. By "biomass" is intended any measured plant product. An increase in biomass production is any improvement in the yield of the measured plant product. Increasing plant yield has several commercial applications. For example, increasing plant leaf biomass may increase the yield of leafy vegetables for human or animal consumption. Additionally, increasing leaf biomass can be used to increase production of plant-derived pharmaceutical or industrial products. An increase in yield can comprise any statistically significant increase including, but not limited to, at least a 1% increase, at least a 3% increase, at least a 5% increase, at least a 10% increase, at least a 20% increase, at least a 30%, at least a 50%, at least a 70%, at least a 100% or a greater increase.

In specific methods, the plant is treated with an effective concentration of an herbicide, where the herbicide application results in enhanced plant yield. By "effective concentration" is intended the concentration which allows the increased yield in the plant. Such effective concentrations for herbicides of interest are generally known in the art. The herbicide may be applied either pre- or post emergence in accordance with usual techniques for herbicide application to fields comprising crops which have been rendered resistant to the herbicide by heterologous expression of a *grg* gene of the invention.

Methods for conferring herbicide resistance in a plant or plant part are also provided. In such methods, a *grg* polynucleotide disclosed herein is introduced into the plant, wherein expression of the polynucleotide results in glyphosate tolerance or resistance. Plants produced via this method can be treated with an effective concentration of an herbicide and display an increased tolerance to the herbicide. An "effective concentration" of an herbicide in this application is an amount sufficient to slow or stop the growth of plants or plant parts that are not naturally resistant or rendered resistant to the herbicide.

In another embodiment, methods for conferring herbicide resistance in a plant or plant part are provided, wherein the plant or plant part is grown under higher or lower than ambient environmental temperatures as described *supra.* Glyphosate tolerant EPSP synthase enzymes having thermal stability at higher or lower temperatures, or have temperature optima at higher or lower temperatures, are useful for conferring glyphosate tolerance in plants that are grown under such conditions.

### Methods of controlling weeds in a field

Methods for selectively controlling weeds in a field containing a plant are also provided. In one embodiment, the plant seeds or plants are glyphosate resistant as a result *of a grg* polynucleotide disclosed herein being inserted into the plant seed or plant. In specific methods, the plant is treated with an effective concentration of an herbicide, where the herbicide application results in a selective control of weeds or other untransformed plants. By "effective concentration" is intended the concentration which controls the growth or spread of weeds or other untransformed plants without significantly affecting the glyphosate-resistant plant or plant seed. Such effective concentrations for herbicides of interest are generally known in the art. The herbicide may be applied either pre- or post emergence in accordance with usual techniques for herbicide application to fields comprising plants or plant seeds which have been rendered resistant to the herbicide.

The following examples are offered by way of illustration and not by way of limitation.

### EXPERIMENTAL

### Example 1: Isolation of ATX8909

ATX8909 was isolated by plating samples of soil on HEPES Mineral Salts Medium (HMSM) containing glyphosate as the sole source of phosphorus. Since HMSM contains no aromatic amino acids, a strain must be resistant to glyphosate in order to grow on this media.

Two grams of soil were suspended in approximately 10 ml of water, vortexed for 15 seconds and permitted to settle for 15 minutes. A 10 µ1 loopful of this suspension was added to 3 ml of HMSM supplemented with 10 mM glyphosate (pH 7.0). HMSM contains (per liter): 10 g glucose, 2 g NH₄SO₄, 9.53 g HEPES, 1.0 ml 0.8 M MgS0₄,1.0 ml 0.1 M CaCl₂, 1.0 ml Trace Elements Solution (In 100 ml of 1000x solution: 0.1 g FeSO₄·7H₂O, 0.5 mg C_{U}SO₄·5H₂O, 1.0 mg H₃BO₃, 1.0 mg MnSO₄·5H₂O, 7.0 mg ZnSO₄·7H₂O, 1.0 mg Mo0₃, 4.0 g KCl). The culture was grown in a shaker incubator for four days at 28°C and then 20 µl was used to inoculate 2.5 ml of fresh HMSM containing 10 mM glyphosate as the only phosphorus source. After two days, 20 µl was used to inoculate another fresh 2.5 ml culture. After 5 days, 20 µl was used to inoculate a fresh 2.5 ml culture. After sufficient growth, the culture was plated onto solid media by streaking a 1 µl loop onto the surface of agar plate containing HMSM agar containing 100 mM glyphosate as the sole phosphorus source and stored at 28°C. The culture was then replated for isolation. One particular strain, designated ATX8909, was selected due to its ability to grow in the presence of high glyphosate concentrations.

Strains ATX1367, ATX1394, ATX21307, ATX21310, ATX21315 and ATX21318 were isolated as described above and found to grow in a liquid minimal medium containing up to 300 mM glyphosate. Each of the strains was tested by 16S rDNA sequencing or fatty acid content by methods known in the art to identify the strain: The strain identifications are shown in Table 1:

**Table 1.**

| Strain Name | Strain ID | EPSP synthase |
|---|---|---|
| | | Gene Name |
| ATX8909 | *Arthrobacter arilaiti* | *grg25* |
| ATX21307 | *Arthrobacter ramosus* | *grg26* |
| ATX21310 | Gram + (no match to MIDI database) | *grg27* |
| ATX21315 | *Arthrobacter ureafaciens* | *grg28* |
| ATX1367 | *Paenibacillus pabuli* | *grg29* |
| ATX1394 | *Paenibacillus pabuli* | *grg30* |
| ATX21318 | *Sphingobacterium multivorum*/*faecium* | *grg31* |

### Example 2. Cloning of glyphosate-resistant EPSP Synthases

Genomic DNA was extracted from the strains described in Table 1 and the resulting DNA was partially digested with restriction enzyme *Sau3A* 1 to yield DNA fragments approximately 5 kilobases in size. These DNA molecules were size selected on agarose gels, purified, and ligated into LAMBDA ZAP® vector arms predigested with *BamH* I. The ligated arms were then packaged into phage particles, and phage titers determined as known in the art. The resulting libraries were amplified by methods known in the art to generate a library titer of between 3 x 10⁷ and 3 x 10⁸ PFU/mL. For each independent library, *E. coli* (XL1 Blue MRF') was then co-transfected with phage from an amplified library as well as M13 helper phage into to allow mass excision of the library in the form of an infectious, circular ssDNA as known in the art *(*Short et al. (1988) Nucleic Acids Research 16:7583-7600). After centrifugation of the co-infected cells, the phage-containing supernatant was heated to 65-70°C for 15-20 minutes to incapacitate any residual lambda phage particles. Dilutions of the resulting ssDNA plasmid library were transfected into a fresh culture of competent *E. coli* XL-Blue *MRF'(aroA)* cells (XL1 Blue MRF'). The resulting transfected cells were plated onto M63 plates containing kanamycin, 0.1 mM IPTG and either 0 mM, 20 mM or 50 mM glyphosate.

The *E. coli* XL-Blue *MRF'(aroA)* used for the transfection expresses the F-pilus, and also contains a deletion of the *aroA* gene encoding the endogenous *E. coli* EPSP synthase enzyme. This strain is also referred to as herein as Δ*aroA.* This Δ*aroA* strain is unable to grow on minimal media lacking aromatic amino acids, unless complemented by a functional EPSP synthase. Since glyphosate is a potent inhibitor of typical, glyphosate-sensitive EPSP synthases, such as type I EPSP synthases, transfected clones expressing a non-glyphosate resistant EPSP synthase would be able to grown on M63 plates lacking glyphosate, but would be unable to grow on M63 containing either 20mM or 50mM glyphosate. In order to grow on M63 plates containing 20mM or 50mM glyphosate, the cells must contain a plasmid that expresses an EPSP synthase that is both (1) capable of complementing the Δ*aroA* mutation of these cells, and (2) resistant to glyphosate. Thus, this screening method allows identification of clones containing glyphosate-resistant EPSP synthases.

Colonies growing on 20mM or 50mM glyphosate were picked and their plasmids analyzed by restriction digest to identify plasmids with shared restriction patterns. Individual plasmids were sequenced by methods known in the art, with preference given to plasmids that conferred resistance to 50 mM glyphosate.

Using this approach, as sometimes modified for each library as known and appreciated in the art, library clones containing EPSP synthase genes were identified for each of the seven strains of Table 1.

### Example 3. DNA and protein sequences of EPSP synthases

The DNA sequences of the glyphosate-resistant EPSP synthases was determined for each of the clones in Table 2 by methods well known in the art.

*grg25.* The DNA sequence of *grg25* is provided herein as SEQ ID NO:1. The predicted translation product of *grg25* (GRG25) is provided herein as SEQ ID NO:2.

*grg26.* The DNA sequence of *grg26* is provided herein as SEQ ID NO:3. The predicted translation product *of grg26* (GRG26) is provided herein as SEQ ID NO:4.

*grg27.* The DNA sequence of *grg27* is provided herein as SEQ ID NO:5. The predicted translation product of *grg27* (GRG27) is provided herein as SEQ ID NO:6.

*grg28.* The DNA sequence of *grg28* is provided herein as SEQ ID NO: 7. The predicted translation product of *grg28* (GRG28) is provided herein as SEQ ID NO:8.

*grg29.* The DNA sequence of *grg29* is provided herein as SEQ ID NO:9. The predicted translation product *of grg29* (GRG29) is provided herein as SEQ ID NO:10.

*grg30.* The DNA sequence of *grg30* is provided herein as SEQ ID NO:11. The predicted translation product of *grg30* (GRG30) is provided herein as SEQ ID NO:12.

*grg31.* The DNA sequence of *grg31* is provided herein as SEQ ID NO:13. The predicted translation product of *grg31*(GRG31) is provided herein as SEQ ID NO:14.

*syngrg31.* The synthetic DNA sequence of *syngrg31* is provided herein as SEQ ID NO:28. The predicted translation product of syngrg1 is identical to that of *grg31,* and is provided herein as SEQ ID NO:14.

*syngrg25.* The synthetic DNA sequence of *syngrg25* is provided herein as SEQ ID NO:29. The predicted translation product of syngrg1 is identical to that of *grg25,* and is provided herein as SEQ ID NO:2.

*syngrg26.* The synthetic DNA sequence of *syngrg26* is provided herein as SEQ ID NO:30. The predicted translation product of syngrg1 is identical to that of *grg26,* and is provided herein as SEQ ID NO: 4.

*syngrg27.* The synthetic DNA sequence of *syngrg27* is provided herein as SEQ ID NO:31. The predicted translation product of syngrg1is identical to that of *grg27,* and is provided herein as SEQ ID NO:6.

*syngrg28.* The synthetic DNA sequence of *syngrg28* is provided herein as SEQ ID NO:32. The predicted translation product of syngrg1 is identical to that of *grg28,* and is provided herein as SEQ ID NO:8.

*syngrg29.* The synthetic DNA sequence of *syngrg29* is provided herein as SEQ ID NO:33. The predicted translation product of syngrg1 is identical to that of *grg29,* and is provided herein as SEQ ID NO:10.

*syngrg30.* The synthetic DNA sequence of *syngrg30* is provided herein as SEQ ID NO:34. The predicted translation product of syngrg1 is identical to that of *grg30,* and is provided herein as SEQ ID NO:12.

Clones containing each of the *grg25, grg26, grg27, grg28, grg29, grg30,* and *grg31* EPSP synthase genes were deposited at NRRL, and assigned deposit numbers as in Table 2.

**Table 2. Clones containing glyphosate-resistant EPSP synthases**

| EPSPS | Strain yielding EPSPS | Original Isolate in pBKCMV | NRRL Number |
|---|---|---|---|
| GRG25 | ATX8909 | pAX1932 | NRRL B-30911 |
| GRG26 | ATX21307 | pAX1933 | NRRL B-30912 |
| GRG27 | ATX21310 | pAX1934 | NRRL B-30913 |
| GRG28 | ATX21315 | pAX1935 | NRRL B-30914 N |
| GRG31 | ATX21318 | pAX1936 | NRRL B-30915 |
| GRG30 | ATX1394 | pAX1937 | NRRL B-30916 N |
| GRG29 | ATX1367 | pAX1938 | NRRL B-30917 N |

Each of the proteins GRG25 - GRG31 showed homology to EPSP synthase enzymes in the NCBI database by BLAST search. The EPSPS enzyme with the highest protein sequence identity to each GRG enzyme is listed in the following table.

**Table 3. Homology of GRG25-GRG31 to known EPSP synthases**

| | **Strain with homologous EPSPS** | |
|---|---|---|
| **Protein** | **enzyme** | **% Identity** |
| GRG25 | *Nocardia farcinica* | 50.5 |
| GRG26 | *Arthrobacter* sp. FB24 | 82.9 |
| GRG27 | *Arthrobacter* sp. FB24 | 78 |
| GRG28 | *Arthrobacter* sp. FB24 | 81.4 |
| GRG29 | *Symbiobacterium thermophilum* | 51.6 |
| GRG30 | *Symbiobacterium thermophilum* | 51.3 |
| GRG31 | *Bacteroides fragilis* | 43.3 |

### Example 4. Cloning of novel Glyphosate-resistant EPSP synthases into an E. coli Expression vector

The EPSP synthase genes contained in the clones of Table 2 were sub-cloned into the *E.coli* expression vector pRSF1b (Invitrogen). Resulting clones were confirmed by DNA sequencing, and used to induce expression of each EPSP synthase in *E. coli.* The expressed His-tagged protein was then purified as known in the art.

### Example 5. Glyphosate resistance of EPSP synthases

The pRSF1b clones were plated onto M63+ plates containing antibiotic and either 0mM or 50mM glyphosate. Growth was scored after two days growth at 37°C. All of the seven EPSP synthases were observed to confer resistance to 50mM glyphosate in *E. coli* cells (Table 4).

**Table 4. Glyphosate screen**

| EPSPS | Clone in pRSF1B | Growth on 50mM glyphosate |
|---|---|---|
| Vector | - | - |
| GRG25 | pAX1939 | +++ |
| GRG26 | pAX1940 | +++ |
| GRG27 | pAX1941 | +++ |
| GRG28 | pAX1942 | +++ |
| GRG30 | pAX1944 | +++ |
| GRG29 | pAX1943 | +++ |
| GRG31 | pAX1945 | +++ |

### Example 6. Temperature optimum of GRG31 enzymatic activity

A vector that places expression of GRG31 under the control of the viral T7 promoter was constructed (pAX3535) and used to transform an *E. coli* strain possessing the viral T7 gene immediately 3' to a lactose inducible promoter. Following IPTG induction, the GRG31 protein was purified to homogeneity by standard methods. To measure enzymatic activity, the purified GRG31 enzyme was diluted to an appropriate assay concentration in buffer containing HEPES (50 mM, pH 7) and 50 mM KCl, and then incubated for 15 minutes at either 10, 20, 30, 40, 50 or 60°C. Following incubation, the enzyme was heated to 90°C for 1 minute to denature the enzyme, and then cooled to 4°C. The phosphate generated by each reaction was then added to a second assay containing inosine, purine nucleoside phosphorylase, xanthine oxidase, horseradish peroxidase, and the fluorescent substrate AMPLEX® Red (U.S. Patent Application No. 60/741,166, filed December 1, 2005). Following incubation for 15 minutes at room temperature, fluorescent product was quantified using a Gemini XPS spectrofluorometer (Molecular Devices Corporation, Sunnyvale, CA). EPSP synthase product formation was measured, and is expressed in Table 5 as a percentage of maximal activity.

**Table 5. Temperature optimum for GRG31**

| Temperature, °C | Percentage of Maximal Activity |
|---|---|
| 10 | 42% |
| 20 | 62% |
| 30 | 69% |
| 40 | 80% |
| 50 | 100% |
| 60 | 96% |
| 70 | 67% |
| 80 | 36% |

### Example 7. Thermal stability of GRG31 enzymatic activity

A vector that places expression of GRG31 under the control of the viral T7 promoter was constructed (pAX3535) and used to transform an *E. coli* strain possessing the viral T7 gene immediately 3' to a lactose inducible promoter. Following IPTG induction, the GRG31 protein was purified to homogeneity by standard methods. To measure enzymatic activity, the purified GRG31 enzyme was diluted to an appropriate assay concentration in buffer containing HEPES (50 mM, pH 7) and 50 mM KCl, and then incubated at 37°C for 0, 2, 6 or 22 hours. A control sample was incubated alongside at 4°C. At each timepoint, an aliquot was removed from each sample and enzymatic assays were carried out in a final volume of 50 µl containing 0.5 mM shikimate-3-phosphate, 200 uM phosphoenolpyruvate (PEP), 1 U/ml xanthine oxidase, 2 U/ml nucleoside phosphorylase, 2.25 mM inosine, 1 U/ml horseradish peroxidase, 2 mM glyphosate, 50 mM HEPES/KOH pH 7.0, 100 mM KCl, and AMPLEX® Red (Invitrogen) according to the manufacturer's instructions. Assays were started by adding shikimate-3-phosphate. EPSP synthase activity was measured using a Spectramax Gemini XPS fluorescence spectrometer (Molecular Dynamics, excitation: 555 nm; emission: 590 nm). EPSP synthase thermal stability was calculated as the percentage of enzymatic activity at each timepoint at 37°C relative to enzymatic activity in the control sample incubated alongside at 4°C (Table 6).

**Table 6. Thermal stability of GRG31**

| Time, hours | Percentage of control |
|---|---|
| 0 | 100% |
| 2 | 86% |
| 6 | 68% |
| 22 | 29% |

### Example 8. Engineering of grg25, grg26, grg27, grg28, grg29, grg30, grg31 and syngrg25, syngrg26, syngrg27, syngrg28, syngrg29, syngrg30, and syngrg31 for Plant Transformation

The open reading frame (ORF) for each of the *grg* genes is amplified by PCR from a full-length cDNA template. Hind III restriction sites are added to each end of the ORF during PCR. Additionally, the nucleotide sequence ACC is added immediately 5' to the start codon of the gene to increase translational efficiency (Kozak (1987) Nucleic Acids Research 15:8125-8148; Joshi (1987) Nucleic Acids Research 15:6643-6653). The PCR product is cloned and sequenced, using techniques well known in the art, to ensure that no mutations are introduced during PCR. The plasmid containing the *grg* PCR product is digested with, for example, *Hind* III and the fragment containing the intact ORF is isolated.

One may generate similar constructs that contain a chloroplast targeting sequence linked to the polynucleotide of the invention by methods known in the art.

A DNA fragment containing the EPSP synthase (and either containing or not containing a chloroplast targeting sequence) is cloned into a plasmid, for example at the Hind III site of pAX200 pAX200 is a plant expression vector containing the rice actin promoter (McElroy et al. (1991) Molec. Gen. Genet. 231:150-160), and the PinII terminator *(*An et al. (1989) The Plant Cell 1:115-122). The promoter- gene-terminator fragment (or the promoter-leader-gene-terminator fragment) from this intermediate plasmid is subcloned into a plasmid such as pSB11 (Japan Tobacco, Inc.) to form a final plasmid, referred to herein as, for example, pSB11GRG25. pSB11GRG25 is organized such that the DNA fragment containing, for example, the promoter-*grg25 -* terminator construct (or the promoter-leader-grg25 - terminator construct) may be excised by appropriate restriction enzymes and also used for transformation into plants, for example, by aerosol beam injection. The structure of pSB11GRG25 is verified by restriction digest and gel electrophoresis, as well as by sequencing across the various cloning junctions. The same methods can be used to generate a final plasmid for each of the *grg* genes described herein. The plasmid is mobilized into *Agrobacterium tumefaciens* strain LBA4404 which also harbors the plasmid pSB1 (Japan Tobacco, Inc.), using triparental mating procedures well known in the art, and plating on media containing antibiotic. Plasmid pSB11GRG25 carries spectinomycin resistance but is a narrow host range plasmid and cannot replicate in *Agrobacterium.* Antibiotic resistant colonies arise when pSB11GRG25 integrates into the broad host range plasmid pSB1 through homologous recombination. The resulting cointegrate product is verified by Southern hybridization. The *Agrobacterium* strain harboring the cointegrate can be used to transform maize, for example, by the PureIntro method (Japan Tobacco).

### Example 9. Transformation of grg25,grg26, grg27, grg28, grg29, grg30, grg31, syngrg25, syngrg26, syngrg27, syngrg28, syngrg29, syngrg30, and syngrg31 into Plant Cells

Maize ears are best collected 8-12 days after pollination. Embryos are isolated from the ears, and those embryos 0.8-1.5 mm in size are preferred for use in transformation. Embryos are plated scutellum side-up on a suitable incubation media, such as DN62A5S media (3.98 g/L N6 Salts; 1 mL/L (of 1000x Stock) N6 Vitamins; 800 mg/L L-Asparagine; 100 mg/L Myo-inositol; 1.4 g/L L-Proline; 100 mg/L Casamino acids; 50 g/L sucrose; 1 mL/L (of 1 mg/mL Stock) 2,4-D). However, media and salts other than DN62A5S are suitable and are known in the art. Embryos are incubated overnight at 25°C in the dark. However, it is not *necessary per se* to incubate the embryos overnight.

The resulting explants are transferred to mesh squares (30-40 per plate), transferred onto osmotic media for about 30-45 minutes, then transferred to a beaming plate (see, for example, PCT Publication No. WO/0138514 and U.S. Patent No. 5,240,842).

DNA constructs designed to express the GRG proteins of the present invention in plant cells are accelerated into plant tissue using an aerosol beam accelerator, using conditions essentially as described in PCT Publication No. WO/0138514. After beaming, embryos are incubated for about 30 min on osmotic media, and placed onto incubation media overnight at 25°C in the dark. To avoid unduly damaging beamed explants, they are incubated for at least 24 hours prior to transfer to recovery media. Embryos are then spread onto recovery period media, for about 5 days, 25°C in the dark, then transferred to a selection media. Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated by methods known in the art. The resulting shoots are allowed to root on rooting media, and the resulting plants are transferred to nursery pots and propagated as transgenic plants.

### Materials

**Table 7. DN62A5S Media**

| **Components** | **Per Liter** | **Source** |
|---|---|---|
| Chu's N6 Basal Salt Mixture | 3.98 g/L | Phytotechnology Labs |
| (Prod. No. C 416) | | |
| Chu's N6 Vitamin Solution | 1 mL/L (of 1000x Stock) | Phytotechnology Labs |
| (Prod. No. C 149) | | |
| L-Asparagine | 800 mg/L | Phytotechnology Labs |
| Myo-inositol | 100 mg/L | Sigma |
| L-Proline | 1.4 g/L | Phytotechnology Labs |
| Casamino acids | 100 mg/L | Fisher Scientific |
| Sucrose | 50 g/L | Phytotechnology Labs |
| 2,4-D (Prod. No. D-7299) | 1 mL/L (of 1 mg/mL Stock) | Sigma |

The pH of the solution is adjusted to pH 5.8 with 1N KOH/1N KCl, Gelrite (Sigma) is added at a concentration up to 3g/L, and the media is autoclaved. After cooling to 50°C, 2 ml/L of a 5 mg/ml stock solution of silver nitrate (Phytotechnology Labs) is added.

### Example 10. Transformation of grg25, grg26, grg27, grg28, grg29, grg30, grg31, syngrg25, syngrg26, syngrg27, syngrg28, syngrg29, or syngrg31 into Maize Plant Cells by Agrobacterium-Mediated Transformation

Ears are best collected 8-12 days after pollination. Embryos are isolated from the ears, and those embryos 0.8-1.5 mm in size are preferred for use in transformation. Embryos are plated scutellum side-up on a suitable incubation media, and incubated overnight at 25°C in the dark. However, it is not necessary *per se* to incubate the embryos overnight. Embryos are contacted with an *Agrobacterium* strain containing the appropriate vectors for Ti plasmid mediated transfer for about 5-10 min, and then plated onto co-cultivation media for about 3 days (25°C in the dark). After co-cultivation, explants are transferred to recovery period media for about five days (at 25°C in the dark). Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated as known in the art. The resulting shoots are allowed to root on rooting media, and the resulting plants are transferred to nursery pots and propagated as transgenic plants.

All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

### NUMBERED FURTHER ASPECTS OF THE INVENTION

(1) An isolated nucleic acid molecule comprising a nucleotide sequence selected from the group consisting of:
   (a) the nucleotide sequence of SEQ ID Nos: 13, 1, 3, 5, 7, 9, 11, 28, 29, 30, 31, 32, 33, or 34, or a complement thereof;
   (b) a nucleotide sequence having at least 90% sequence identity to the nucleotide sequence of SEQ ID NOs: 13, 1, 3, 5, 7, 9, 11, 28, 29, 30, 31, 32, 33, or 34, or a complement thereof;
   (c) the herbicide resistance nucleotide sequence of the DNA insert of the plasmid deposited as Accession Nos. NRRL B-30915, NRRL B-30911, NRRL B-30912, NRRL B-30913, NRRL B-30914 N, NRRL B-30916 N, or NRRL B-30917 N, or a complement thereof;
   (d) a nucleotide sequence that encodes a polypeptide comprising the amino acid sequence of SEQ ID Nos: 14, 2, 4, 6, 8, 10, or 12; and,
   (e) a nucleotide sequence encoding a polypeptide having at least 90% amino acid sequence identity to the amino acid sequence of SEQ ID Nos: 14, 2, 4, 6, 8, 10, or 12.
(2) The isolated nucleic acid molecule of (1), wherein said nucleotide sequence is a synthetic sequence that has been designed for expression in a plant.
(3) A vector comprising the nucleic acid molecule of (1).
(4) The vector of (3), further comprising a nucleic acid molecule encoding a heterologous polypeptide.
(5) A host cell that contains the vector of (3).
(6) The host cell of (5) that is a bacterial host cell.
(7) The host cell of (5) that is a plant cell.
(8) A transgenic plant comprising the host cell of (7).
(9) A plant of (8), wherein said plant is selected from the group consisting of maize, sorghum, wheat, sunflower, tomato, crucifers, peppers, potato, cotton, rice, soybean, sugarbeet, sugarcane, tobacco, barley, and oilseed rape.
(10) A transformed seed of the plant of (8).
(11) An isolated polypeptide selected from the group consisting of:
   (a) a polypeptide comprising the amino acid sequence of SEQ ID NOs: 14, 2, 4, 6, 8, 10, or 12;
   (b) a polypeptide encoded by the nucleotide sequence of SEQ ID NOs: 13, 1, 3, 5, 7, 9, 11, 28, 29, 30, 31, 32, 33, or 34;
   (c) a polypeptide comprising an amino acid sequence having at least 90% sequence identity to the amino acid sequence of SEQ ID Nos: 14, 2, 4, 6, 8, 10, or 12, wherein said polypeptide has herbicide resistance activity;
   (d) a polypeptide that is encoded by a nucleotide sequence that is at least 90% identical to the nucleotide sequence of SEQ ID Nos: 13, 1, 3, 5, 7, 9, 11, 28, 29, 30, 31, 32, 33, or 34, wherein said polypeptide has herbicide resistance activity; and,
   (e) a polypeptide that is encoded by the herbicide resistance nucleotide sequence of the DNA insert of the plasmid deposited as Accession Nos. NRRL B-30915, NRRL B-30911, NRRL B-30912, NRRL B-30913, NRRL B-30914 N, NRRL B-30916 N, or NRRL B-30917 N.
(12) The polypeptide of (11) further comprising a heterologous amino acid sequence.
(13) A method for producing a polypeptide with herbicide resistance activity, comprising culturing the host cell of (5) under conditions in which a nucleic acid molecule encoding the polypeptide is expressed, said polypeptide being selected from the group consisting of:
   (a) a polypeptide comprising the amino acid sequence of SEQ ID NOs: 14, 2, 4, 6, 8, 10, or 12;
   (b) a polypeptide encoded by the nucleic acid sequence of SEQ ID NOs: 13, 1, 3, 5, 7, 9, 11, 28, 29, 30, 31, 32, 33, or 34;
   (c) a polypeptide comprising an amino acid sequence having at least 90% sequence identity to a polypeptide with the amino acid sequence of SEQ ID Nos: 14, 2, 4, 6, 8, 10, or 12, wherein said polypeptide has herbicide resistance activity;
   (d) a polypeptide encoded by a nucleic acid molecule comprising a nucleotide sequence having at least 90% sequence identity to the nucleic acid sequence of SEQ ID Nos: 13, 1, 3, 5, 7, 9, 11, 28, 29, 30, 31, 32, 33, or 34, wherein said polypeptide has herbicide resistance activity; and,
   (e) a polypeptide that is encoded by the herbicide resistance nucleotide sequence of the DNA insert of the plasmid deposited as Accession Nos. NRRL B-30915, NRRL B-30911, NRRL B-30912, NRRL B-30913, NRRL B-30914 N, NRRL B-30916 N, or NRRL B-30917 N.
(14) A method for conferring resistance to an herbicide in a plant, said method comprising transforming said plant with a DNA construct, said construct comprising a promoter that drives expression in a plant cell operably linked with a nucleotide sequence, and regenerating a transformed plant, wherein said nucleotide sequence is selected from the group consisting of:
   (a) the nucleotide sequence of SEQ ID Nos: 13, 1, 3, 5, 7, 9, 11, 28, 29, 30, 31, 32, 33, or 34, or a complement thereof;
   (b) a nucleotide sequence having at least 90% sequence identity to the nucleotide sequence of SEQ ID NOs: 13, 1, 3, 5, 7, 9, 11, 28, 29, 30, 31, 32, 33, or 34, or a complement thereof, wherein said nucleotide sequence encodes a polypeptide having herbicide resistance activity;
   (c) the herbicide resistance nucleotide sequence of the DNA insert of the plasmid deposited as Accession Nos. NRRL B-30915, NRRL B-30911, NRRL B-30912, NRRL B-30913, NRRL B-30914 N, NRRL B-30916 N, or NRRL B-30917 N, or a complement thereof;
   (d) a nucleotide sequence that encodes a polypeptide comprising the amino acid sequence of SEQ ID Nos: 14, 2, 4, 6, 8, 10, or 12; and,
   (e) a nucleotide sequence encoding a polypeptide having at least 90% amino acid sequence identity to the amino acid sequence of SEQ ID Nos: 14, 2, 4, 6, 8, 10, or 12, wherein said polypeptide has herbicide resistance activity.
(15) The method of (14), wherein said plant produces a polypeptide having herbicide resistance activity.
(16) The method of (14), wherein said herbicide is a glyphosate.
(17) A plant having stably incorporated into its genome a DNA construct comprising a nucleotide sequence that encodes a protein having herbicide resistance activity, wherein said nucleotide sequence is selected from the group consisting of:
   (a) a nucleotide sequence of SEQ ID Nos: 13, 1, 3, 5, 7, 9, 11, 28, 29, 30, 31, 32, 33, or 34;
   (b) a nucleotide sequence having at least 90% sequence identity to a nucleotide sequence of SEQ ID Nos: 13, 1, 3, 5, 7, 9, 11, 28, 29, 30, 31, 32, 33, or 34, wherein said nucleotide sequence encodes a polypeptide having herbicide resistance activity;
   (c) a nucleotide sequence encoding a polypeptide comprising an amino acid sequence of SEQ ID Nos: 14, 2, 4, 6, 8, 10, or 12;
   (d) a nucleotide sequence encoding a polypeptide having at least 90% amino acid sequence identity to the amino acid sequence of SEQ ID Nos: 14, 2, 4, 6, 8, 10, or 12, wherein said polypeptide has herbicide resistance activity; and,
   (e) the herbicide resistance nucleotide sequence of the DNA insert of the plasmid deposited as Accession Nos. NRRL B-30915, NRRL B-30911, NRRL B-30912, NRRL B-30913, NRRL B-30914 N, NRRL B-30916 N, or NRRL B-30917 N; wherein said nucleotide sequence is operably linked to a promoter that drives expression of a coding sequence in a plant cell.
(18) The plant of (17), wherein said plant is a plant cell.
(19) A method for generating a polynucleotide variant of a parent *grg25, grg26, grg27, grg28, grg29, grg30, grg31, syngrg25, syngrg26, syngrg27, syngrg28, syngrg29, syngrg30, or syngrg31* polynucleotide, comprising using the nucleotide sequence of SEQ ID Nos: 13, 1, 3, 5, 7, 9, 11, 28, 29, 30, 31, 32, 33, or 34, or a fragment thereof, in a recombinogenic procedure, and testing a polypeptide encoded by the resulting polynucleotide sequence for an activity of interest.
(20) The method of (19), wherein the activity of interest is glyphosate resistance activity.
(21) The method of (20), wherein said polypeptide encoded by the resulting polynucleotide sequence has greater glyphosate resistance activity than a polypeptide encoded by said parent polynucleotide.
(22) The method of (19), wherein said recombinogenic procedure is DNA shuffling.
(23) A method for increasing vigor or yield in a plant comprising:
   (a) introducing into said plant a nucleotide sequence encoding a glyphosate tolerance EPSP synthase enzyme other than SEQ ID NO: 35, 36, or 37 that has a temperature optimum higher than ambient environmental temperature;
   (b) contacting said plant with an effective concentration of glyphosate; and,
   (c) growing said plant under conditions wherein the temperature is higher than ambient environmental temperature for at least two consecutive hours per day for at least four days following contact with said glyphosate, wherein said days following contact is within the growing season of the plant,
      wherein the vigor or yield of said plant is higher than the vigor or yield of a plant expressing a glyphosate tolerance EPSP synthase that does not have a temperature optimum higher than ambient environmental temperature.
(24) The method of (23), wherein said EPSP synthase is not a plant- derived EPSP synthase.
(25) The method of (23), wherein said EPSP synthase enzyme has a temperature optimum from about 35°C to about 55°C and the temperature in step (c) is about 35°C to about 55°C.
(26) The method of (23), wherein said EPSP synthase enzyme is set forth in SEQ ID No: 14 and the temperature in step (b) is about 45 °C to about 55 °C.
(27) A method for conferring resistance to glyphosate in a plant comprising:
   (a) introducing into said plant a nucleotide sequence encoding a glyphosate tolerance EPSP synthase enzyme other than SEQ ID NO:35, 36, or 37 that is thermal stable at temperatures higher than ambient environmental temperature;
   (b) contacting said plant with an effective concentration of glyphosate; and,
   (c) growing said plant under conditions wherein the temperature is higher than ambient environmental temperature for at least two consecutive hours per day for at least four days following contact with said glyphosate, wherein said days following contact is within the growing season of the plant.
(28) The method of (27), wherein said EPSP synthase is not a plant- derived EPSP synthase.
(29) The method of (27), wherein said EPSP synthase enzyme has thermal stability at a temperature from about 32°C to about 60°C and the temperature in step (b) is about 32°C to about 60°C.
(30) The method of (29), wherein said EPSP synthase enzyme has thermal stability at a temperature from about 35°C to about 45°C and the temperature in step (b) is about 35°C to about 45°C.
(31) The method of (27), wherein said EPSP synthase enzyme is set forth in SEQ ID No: 14 and the temperature in step (b) is about 37°C.

## Claims

1. An nucleic acid molecule comprising a nucleotide sequence selected from the group consisting of:
a) the nucleotide sequence of SEQ ID NOs: 11, 13, 1, 3, 5, 7, 9, 28, 29, 30, 31, 32, 33, or 34, or a complement thereof;
b) a nucleotide sequence having at least 90% sequence identity to the nucleotide sequence of SEQ ID NOs: 11, 13, 1, 3, 5, 7, 9, 28, 29, 30, 31, 32, 33, or 34, or a complement thereof;
c) the herbicide resistance nucleotide sequence of the DNA insert of the plasmid deposited as Accession Nos. NRRL B-30916 N, NRRL B-30915, NRRL B-30911, NRRL B-30912, NRRL B-30913, NRRL B-30914 N, or NRRL B-30917 N, or a complement thereof;
d) a nucleotide sequence that encodes a polypeptide comprising the amino acid sequence of SEQ ID NOs: 12,14, 2, 4, 6, 8, or 10; and,
e) a nucleotide sequence encoding a polypeptide having at least 90% amino acid sequence identity to the amino acid sequence of SEQ ID NOs: 12, 14, 2, 4, 6, 8, or 10.

2. The nucleic acid molecule of claim 1, wherein said nucleic acid molecule is operably linked to a promoter that drives expression of said nucleic acid molecule in a plant cell.

3. The nucleic acid molecule of claim 1 or 2, wherein said nucleotide sequence is a synthetic sequence that has been designed for expression in a plant.

4. A vector comprising the nucleic acid molecule of claim 1, 2 or 3, preferably further comprising a nucleic acid molecule encoding a heterologous polypeptide.

5. A host cell that contains the vector of claim 4, preferably where the host cell is:
(a) a bacterial host cell; or
(b) a plant cell.

6. A transgenic plant comprising the plant host cell of claim 5, preferably wherein said plant is selected from the group consisting of maize, sorghum, wheat, sunflower, tomato, crucifers, peppers, potato, cotton, rice, soybean, sugarbeet, sugarcane, tobacco, barley, and oilseed rape.

7. A seed comprising a nucleic acid of any one of claims 1 to 3, a vector according to claim 4 or a host cell according to claim 5.

8. An polypeptide selected from the group consisting of:
(a) a polypeptide comprising the amino acid sequence of SEQ ID NOs: 12, 14, 2, 4, 6, 8, or 10;
(b) a polypeptide encoded by the nucleotide sequence of SEQ ID NOs: 11, 13,1,3,5,7,9,28,29,30,31,32,33,or 34;
(c) a polypeptide comprising an amino acid sequence having at least 90% sequence identity to the amino acid sequence of SEQ ID NOs: 12, 14, 2, 4, 6, 8, or 10, wherein said polypeptide has herbicide resistance activity;
(d) a polypeptide that is encoded by a nucleotide sequence that is at least 90% identical to the nucleotide sequence of SEQ ID NOs: 11, 13, 1, 3, 5, 7, 9, 28, 29, 30, 31, 32, 33, or 34, wherein said polypeptide has herbicide resistance activity; and,
(e) a polypeptide that is encoded by the herbicide resistance nucleotide sequence of the DNA insert of the plasmid deposited as Accession Nos. NRRL B-30916 N, NRRL B-30915, NRRL B-30911, NRRL B-30912, NRRL B-30913, NRRL B-30914 N, or NRRL B-30917 N,
preferably where the polypeptide further comprising a heterologous amino acid sequence.

9. A method for producing a polypeptide with herbicide resistance activity, comprising culturing the host cell of claim 4 under conditions in which a nucleic acid molecule encoding the polypeptide is expressed, said polypeptide being selected from the group consisting of:
(a) a polypeptide comprising the amino acid sequence of SEQ ID NOs: 12, 14, 2, 4, 6, 8, or 10;
(b) a polypeptide encoded by the nucleic acid sequence of SEQ ID NOs: 11, 13, 1, 3, 5, 7, 9, 28, 29, 30, 31, 32, 33, or 34;
(c) a polypeptide comprising an amino acid sequence having at least 90% sequence identity to a polypeptide with the amino acid sequence of SEQ ID NOs: 12, 14, 2, 4, 6, 8, or 10, wherein said polypeptide has herbicide resistance activity;
(d) a polypeptide encoded by a nucleic acid molecule comprising a nucleotide sequence having at least 90% sequence identity to the nucleic acid sequence of SEQ ID NOs: 11, 13, 1, 3, 5, 7, 9, 28, 29, 30, 31, 32, 33, or 34, wherein said polypeptide has herbicide resistance activity; and,
(e) a polypeptide that is encoded by the herbicide resistance nucleotide sequence of the DNA insert of the plasmid deposited as Accession Nos. NRRL B-30916 N, NRRL B-30915, NRRL B-30911, NRRL B-30912, NRRL B-30913, NRRL B-30914 N, or NRRL B-30917 N.

10. A method for conferring resistance to an herbicide in a plant, said method comprising transforming said plant with a DNA construct, said construct comprising a promoter that drives expression in a plant cell operably linked with a nucleotide sequence, and regenerating a transformed plant, wherein said nucleotide sequence is selected from the group consisting of:
(a) the nucleotide sequence of SEQ ID NOs: 11, 13, 1, 3, 5, 7, 9, 28, 29, 30, 31, 32, 33, or 34, or a complement thereof;
(b) a nucleotide sequence having at least 90% sequence identity to the nucleotide sequence of SEQ ID NOs: 11, 13, 1, 3, 5, 7, 9, 28, 29, 30, 31, 32, 33, or 34, or a complement thereof, wherein said nucleotide sequence encodes a polypeptide having herbicide resistance activity;
(c) the herbicide resistance nucleotide sequence of the DNA insert of the plasmid deposited as Accession Nos. NRRL B-30916 N, NRRL B-30915, NRRL B-30911, NRRL B-30912, NRRL B-30913, NRRL B-30914 N, or NRRL B-30917 N, or a complement thereof;
(d) a nucleotide sequence that encodes a polypeptide comprising the amino acid sequence of SEQ ID NOs: 12, 14, 2, 4, 6, 8, or 10; and,
(e) a nucleotide sequence encoding a polypeptide having at least 90% amino acid sequence identity to the amino acid sequence of SEQ ID NOs: 12, 14, 2, 4, 6, 8, or 10, wherein said polypeptide has herbicide resistance activity,
preferably where:
(i) said plant produces a polypeptide having herbicide resistance activity; and/or
(ii) said herbicide is a glyphosate.

11. A plant having stably incorporated into its genome a DNA construct comprising a nucleotide sequence that encodes a protein having herbicide resistance activity, wherein said nucleotide sequence is selected from the group consisting of:
(a) a nucleotide sequence of SEQ ID NOs: 11, 13, 1, 3, 5, 7, 9, 28, 29, 30, 31, 32, 33, or 34;
(b) a nucleotide sequence having at least 90% sequence identity to a nucleotide sequence of SEQ ID NOs: 11, 13, 1, 3, 5, 7, 9, 28, 29, 30, 31, 32, 33, or 34, wherein said nucleotide sequence encodes a polypeptide having herbicide resistance activity;
(c) a nucleotide sequence encoding a polypeptide comprising an amino acid sequence of SEQ ID NOs: 12, 14, 2, 4, 6, 8, or 10;
(d) a nucleotide sequence encoding a polypeptide having at least 90% amino acid sequence identity to the amino acid sequence of SEQ ID NOs: 12, 14, 2, 4, 6, 8, or 10, wherein said polypeptide has herbicide resistance activity; and,
(e) the herbicide resistance nucleotide sequence of the DNA insert of the plasmid deposited as Accession Nos. NRRL B-30916 N, NRRL B-3091 S, NRRL B-30911, NRRL B-30912, NRRL B-30913, NRRL B-30914 N, or NRRL B-30917 N;
wherein said nucleotide sequence is operably linked to a promoter that drives expression of a coding sequence in a plant cell,
preferably where said plant is a plant cell.

12. A method for generating a polynucleotide variant of a parent *grg30, grg25, grg26, grg27, grg28, grg29, grg31, syngrg25, syngrg26, syngrg27, syngrg28, syngrg29, syngrg30,* or *syngrg31* polynucleotide, comprising using the nucleotide sequence of SEQ ID NOs: 11, 13, 1, 3, 5, 7, 9, 28, 29, 30, 31, 32, 33, or 34, or a fragment thereof, in a recombinogenic procedure, and testing a polypeptide encoded by the resulting polynucleotide sequence for an activity of interest, wherein the activity of interest is glyphosate resistance activity, preferably wherein said polypeptide encoded by the resulting polynucleotide sequence has greater glyphosate resistance activity than a polypeptide encoded by said parent polynucleotide, and wherein said recombinogenic procedure is DNA shuffling.

13. A method for selectively controlling weeks in a field containing a plant having planted seeds or plants comprising the steps of:
(a) planting the seeds or plants which are glyphosphate tolerant as a result of the composition of any of claims 1 to 4 being inserted into the seeds or the plant; and
(b) applying to the plants and weeds in an effective concentration of glyphosphate herbicide to control weeds without significantly affecting the plants.

14. A method for increasing yield in a plant comprising growing in a field the plant or plant cell of any of claims 5, 6 or 11 or the seed of claim 7 and applying to the plant an effective concentration of glyphosphate resistance.
